# EUROPEAN PATENT SPECIFICATION

(11) **EP 3 934 634 B1**
(45) Date of publication and mention of the grant of the patent: **24.07.2024**
(21) Application number: 20767324.5
(22) Date of filing: 06.03.2020
(51) Int. Cl.: C07D 215/38, C07D 241/46, C07D 401/12, C07D 417/12, C07D 471/04, C07C 229/10, A61K 31/4709, A61P 35/00, A61P 35/02

(54) **SUBSTITUTED BICYCLIC AND TETRACYCLIC QUINONES AND RELATED METHODS OF USE**
SUBSTITUIERTE BICYCLISCHE UND TETRACYCLISCHE CHINONE UND VERWANDTE VERFAHREN ZU DEREN VERWENDUNG
QUINONES BICYCLIQUES ET TÉTRACYCLIQUES SUBSTITUÉES ET PROCÉDÉS D'UTILISATION ASSOCIÉS

(30) Priority: 06.03.2019 US 201962814470 P
(43) Date of publication of application: 12.01.2022
(73) Proprietor: The Regents Of The University Of Michigan, Ann Arbor, Michigan 48109 (US)
(72) Inventor: NEAMATI, Nouri, Ann Arbor, Michigan 48109-2509 (US); MAO, Shuai, Ann Arbor, Michigan 48109-2590 (US)
(74) Representative: Algemeen Octrooi- en Merkenbureau B.V.
(86) International application number: PCT/US2020/021444
(87) International publication number: WO 2020/181207

(56) References cited:
- WO-A1-2016/040896
- WO-A1-2017/155991
- WO-A1-2017/155991
- WO-A1-97/21432
- US-A1- 2017 283 374
- US-A1- 2018 116 977
- SANTOSO KRISTIANA T ET AL: "The synthesis and evaluation of quinolinequinones as anti-mycobacterial agents", BIOORGANIC, ELSEVIER, AMSTERDAM, NL, vol. 27, no. 16, 1 June 2019 (2019-06-01), pages 3532 - 3545, XP085746099, ISSN: 0968-0896, [retrieved on 20190601], DOI: 10.1016/J.BMC.2019.06.002
- PRATT YOLANDA T.: "Quinolinequinones. VI. Reactions with Aromatic Amines 1", THE JOURNAL OF ORGANIC CHEMISTRY, vol. 27, no. 11, 1 November 1962 (1962-11-01), pages 3905 - 3910, XP055943000, ISSN: 0022-3263, DOI: 10.1021/jo01058a036
- ABDELWAHAB AHMED BAKR ET AL: "Novel Cytotoxic Drugs from Extremophilic Actinomycetes View project Anti-inflammatory compounds View project", 1 August 2014 (2014-08-01), pages 1098 - 1109, XP055943093, Retrieved from the Internet <URL:https://www.researchgate.net/profile/Ahmed-Abdelwahab-3/publication/286308300_Synthesis_and_cytotoxicity_studies_of_some_new_amino_isoquinolin-58-dione_and_quinolin-58-dione_derivatives/links/5721c92708ae82260fab4ad4/Synthesis-and-cytotoxicity-studies-of-some-new-amino-isoquinolin-5-8-dione-and-quin> [retrieved on 20220715]
- HUSSAIN HIDAYAT ET AL: "New quinoline-5,8-dione and hydroxynaphthoquinone derivatives inhibit a chloroquine resistantPlasmodium falciparumstrain", EUROPEAN JOURNAL OF MEDICINAL CHEMISTRY, vol. 54, 3 July 2012 (2012-07-03), pages 936 - 942, XP028932540, ISSN: 0223-5234, DOI: 10.1016/J.EJMECH.2012.06.046
- CHOI S Y ET AL: "The development of 3D-QSAR study and recursive partitioning of heterocyclic quinone derivatives with antifungal activity", BIOORGANIC, ELSEVIER, AMSTERDAM, NL, vol. 14, no. 5, 1 March 2006 (2006-03-01), pages 1608 - 1617, XP027992416, ISSN: 0968-0896, [retrieved on 20060301]
- LEE HYUN-JUNG ET AL: "Synthesis and cytotoxicity evaluation of 6,11-dihydro-pyridazo- and 6,11-dihydro-pyrido[2,3-b]phenazine-6,11-diones", BIOORGANIC, vol. 12, no. 7, 1 April 2004 (2004-04-01), AMSTERDAM, NL, pages 1623 - 1628, XP055943102, ISSN: 0968-0896, DOI: 10.1016/j.bmc.2004.01.029
- KIM ET AL: "Synthesis of 6-chloroisoquinoline-5,8-diones and pyrido[3,4-b]phenazine-5,12-diones and evaluation of their cytotoxicity and DNA topoisomerase II inhibitory activity", BIOORGANIC & MEDICINAL CHEMISTRY, ELSEVIER, AMSTERDAM, NL, vol. 15, no. 1, 15 November 2006 (2006-11-15), pages 451 - 457, XP005764628, ISSN: 0968-0896, DOI: 10.1016/J.BMC.2006.09.040
- KIM YOUNG-SHIN ET AL: "Synthesis and cytotoxicity of 6,11-Dihydro-pyrido- and 6,11-Dihydro-benzo[2,3-b]phenazine-6,11-dione derivatives", BIOORGANIC & MEDICINAL CHEMISTRY, vol. 11, no. 8, 1 April 2003 (2003-04-01), AMSTERDAM, NL, pages 1709 - 1714, XP055969705, ISSN: 0968-0896, DOI: 10.1016/S0968-0896(03)00028-2
- KIM ET AL: "Synthesis of 6-chloroisoquinoline-5,8-diones and pyrido[3,4-b]phenazine-5,12-diones and evaluation of their cytotoxicity and DNA topoisomerase II inhibitory activity", BIOORGANIC & MEDICINAL CHEMISTRY, ELSEVIER, AMSTERDAM, NL, vol. 15, no. 1, 15 November 2006 (2006-11-15), pages 451 - 457, XP005764628, ISSN: 0968-0896, DOI: 10.1016/J.BMC.2006.09.040
- KIM YOUNG-SHIN ET AL: "Synthesis and cytotoxicity of 6,11-Dihydro-pyrido- and 6,11-Dihydro-benzo[2,3-b]phenazine-6,11-dione derivatives", BIOORGANIC & MEDICINAL CHEMISTRY, vol. 11, no. 8, 1 April 2003 (2003-04-01), AMSTERDAM, NL, pages 1709 - 1714, XP055969705, ISSN: 0968-0896, DOI: 10.1016/S0968-0896(03)00028-2
- GOPINATH PUSHPARATHINAM, MAHAMMED ATIF, EILON-SHAFFER TAL, NAWATHA MICKAL, OHAYON SHIMRIT, SHABAT DORON, GROSS ZEEV, BRIK ASHRAF: "Switching Futile para -Quinone to Efficient Reactive Oxygen Species Generator: Ubiquitin-Specific Protease-2 Inhibition, Electrocatalysis, and Quantification", CHEMBIOCHEM ., vol. 18, no. 17, 22 June 2017 (2017-06-22), pages 1683 - 1687, XP055735568, DOI: 10.1002/cbic.201700330

## Description

### STATEMENT REGARDING FEDERALLY SPONSORED RESEARCH OR DEVELOPMENT

This invention was made with government support under CA188252 awarded by the National Institutes of Health. The government has certain rights in the invention.

### FIELD OF THE INVENTION

This invention is in the field of medicinal chemistry. In particular, the invention relates to small molecule compounds having bicyclic and tetracyclic quinone scaffolds which have antiproliferative activities through, for example, induction of reactive oxygen species. The invention further relates to processes for preparing these compounds, and to these compounds for use in treating cancer (e.g., pancreatic cancer, leukemia, non-small cell lung cancer, colon cancer, CNS cancer, melanoma, ovarian cancer, breast cancer, renal cancer, and prostate cancer).

### INTRODUCTION

Cancer is the second leading cause of death in Europe and North America. Surgery, radiation, chemo- and immunotherapies are the major approaches in treating various cancers. Although significant advances have been made during the past decade, the cure rate is still very low for most cancers. Therefore, novel therapeutics is urgently needed to enhance the survival of patients with these devastating diseases. Cancer cells accumulate numerous mutations during the course of their evolution altering multiple signaling pathways and networks. As such, combination of multiple drugs or single compounds having multiple targets tend to show superior activity as compared to single agent drugs. Among various classes of small-molecule drugs tested, quinone containing compounds showed great promise due to their significant increase in oxygen consumption rate in treated cells. Quinones can inhibit various pathways due to their redox, metal-chelation, and in some cases, reactivity toward nucleophiles through Michael addition. Over two dozen drugs containing a quinone moiety have been approved by the FDA or are under clinical investigations not only in oncology but also for other diseases. For example, doxorubicin and dozens of its analogues, mitoxantrone, and mitomycin C are some of the most commonly used FDA approved chemotherapeutic agents for numerous cancers that contain a quinone group.

Other compounds for use in the treatment of cancer can be found in the references listed as follows.

The international patent application with publication number WO 2017/155991A1 discloses small molecule inducers of reactive oxygen species (ROS) and inhibitors of mitochondrial activity. The compounds have quinazolinedione structure and they are proposed for use in the treatment of cancer due to its function as ROS inducers.

Anticancer drugs with the quinone moiety are disclosed also by LEE HYUN-JUNG ET AL: "Synthesis and cytotoxicity evaluation of 6,11-dihydro-pyridazo- and 6,11-dihydro-pyrido[2,3-b]phenazine-6,11-diones", BIOORGANIC, vol. 12, no. 7, 1 April 2004 (2004-04-01), pages 1623-1628, DOI: 10.1016/j.bmc.2004.01.029. Derivatives of 6,11-dihydro-pyridazo[2,3-b]phenazine-6,11-dione and 6,11-dihydro-pyrido[2,3-b]phenazine-6,11-dione were synthesized and their cytotoxicity activities against the tumor cell lines A459 (human lung), SK-OV-3 (human ovarian), SK-MEL-2 (human melanoma), XF498 (human CNS), and HCT 15 (human colon) was evaluated.

Finally, in the international patent application with publication number WO 2016/040896A1, compositions and methods for use in the treatment of prostate carcinoma are disclosed, in which 1,4-naphthoquinone analogs are synthesized and tested in animal models to be proposed as good candidates for the treatment.

The synthesis of some of particular quinones is disclosed in documents such as the one of PRATT YOLANDA T.: "Quinolinequinones. VL Reactions with Aromatic Amines 1", THE JOURNAL OF ORGANIC CHEMISTRY, vol. 27, no. 11, 1 November 1962 (1962-11-01), pages 3905-3910, DOI: 10.1021/jo01058a036.

The synthesis of 7-amino derivatives of isoquinoline-5,8-dione and 7-amino derivatives of quinoline-5,8-dione has been efficiently reported by Abdelwahab Ahmed Bakr ET AL: "Novel Cytotoxic Drugs from Extremophilic Actinomycetes View project Anti-inflammatory compounds View project", 1 August 2014 (2014-08-01), pages 1098-1109. The therein disclosed derivatives shown cytotoxic activity in brine shrimps but were not active for example against bacteria.

Other derivatives of quinoline-5,8-dione and of hydroxynaphthoquinone are reported by HUSSAIN HIDAYAT ET AL: "New quinoline-5,8-dione and hydroxynaphthoquinone derivatives inhibit a chloroquine resistant Plasmodium falciparumstrain", EUROPEAN JOURNAL OF MEDICINAL CHEMISTRY, vol. 54 , pages 936-942, DOI: 10.1016/J.EJMECH.2012.06.046. The compounds are proposed, however, as active against a resistant Plasmodium falciparum strain, causing malaria.

Also with antimicrobial activity, but now as potent antifungals are disclosed 1,4-quinone derivatives in the document of CHOI S Y ET AL: "The development of 3D-QSAR study and recursive partitioning of heterocyclic quinone derivatives with antifungal activity", BIOORGANIC, ELSEVIER, AMSTERDAM, NL, vol. 14, no. 5, 1 March 2006 (2006-03-01), pages 1608-1617. The authors of this document performed three-dimensional quantitative structure-activity relationship studies for a series of compounds using comparative molecular field analysis (ComFA) with the aim to study and guide in the design of more potent active agents.

Improved small molecule compounds having bicyclic and tetracyclic quinone scaffolds for treating cancer are needed.

The present invention addresses this need.

### SUMMARY OF THE INVENTION

Altered redox homeostasis in cancer cells provides a new opportunity for tumor intervention. Reactive oxygen species (ROS), a natural by product from mitochondrial respiration, play an important role as second messengers in cell signaling. In tumor cells, antioxidant enzymes are often active as a result of elevated levels of intrinsic ROS. Altered redox homeostasis in tumors make them more susceptible to induced oxidative stress that overwhelms their adaptive antioxidant capacity and triggers ROS-mediated cell death.

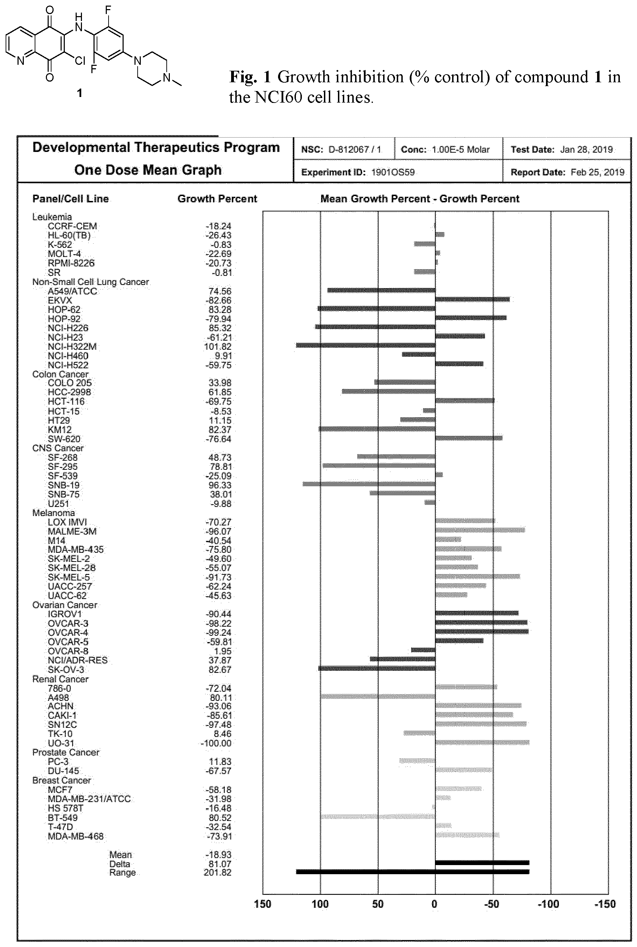

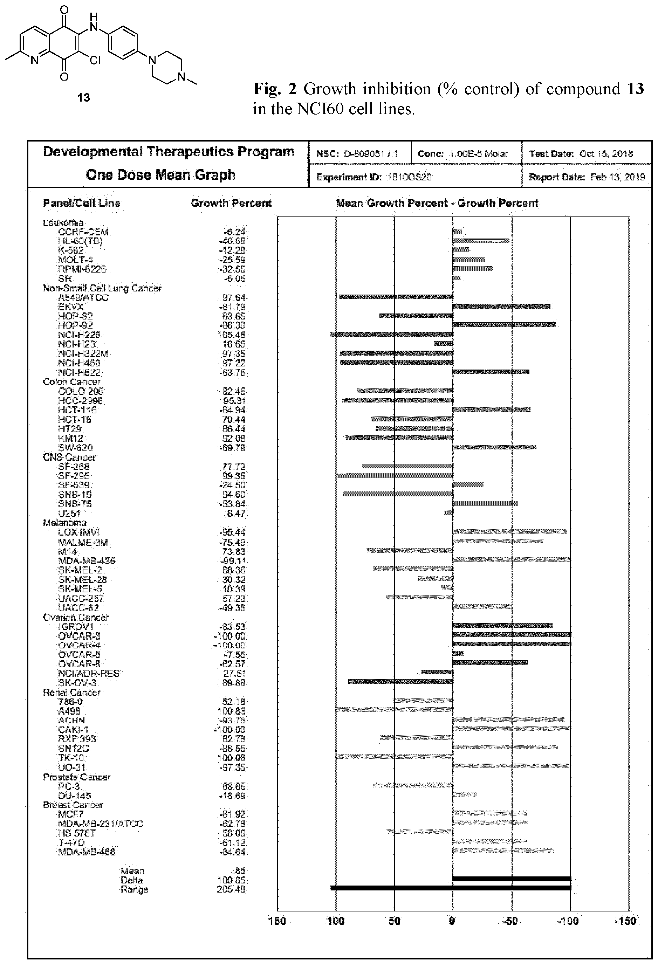

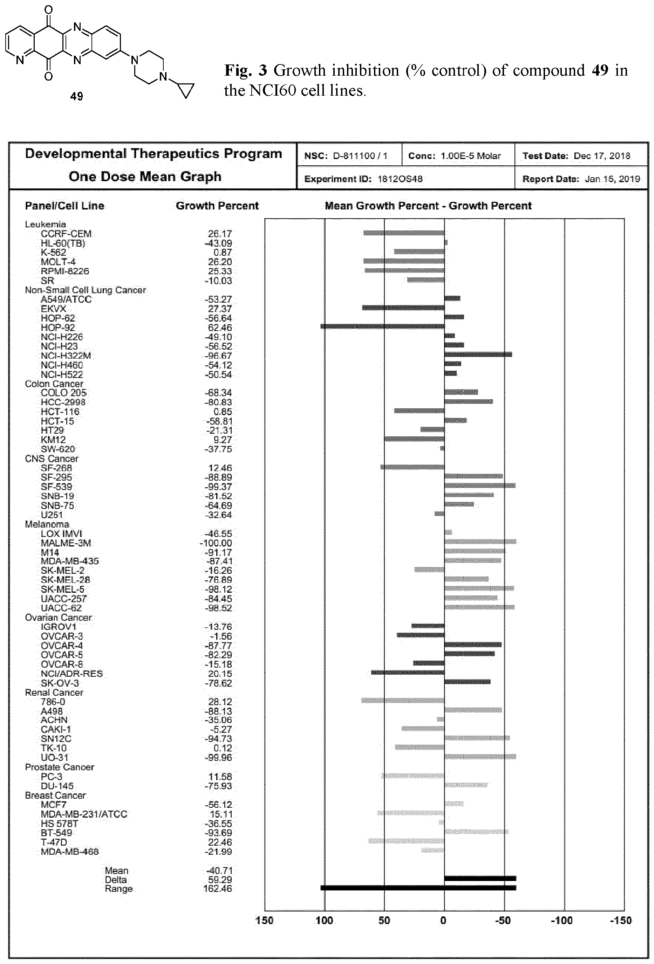

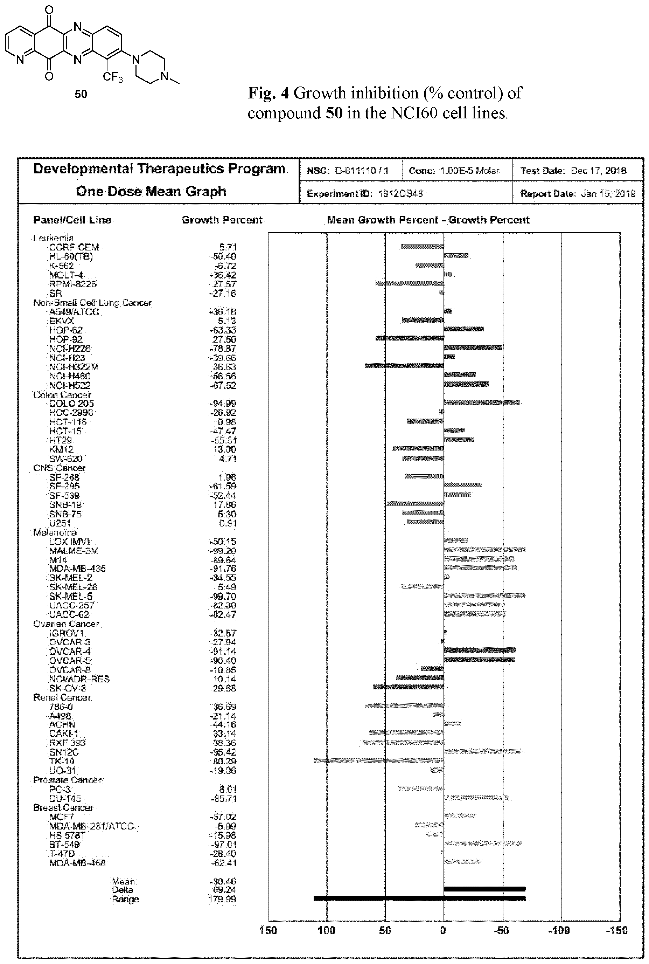

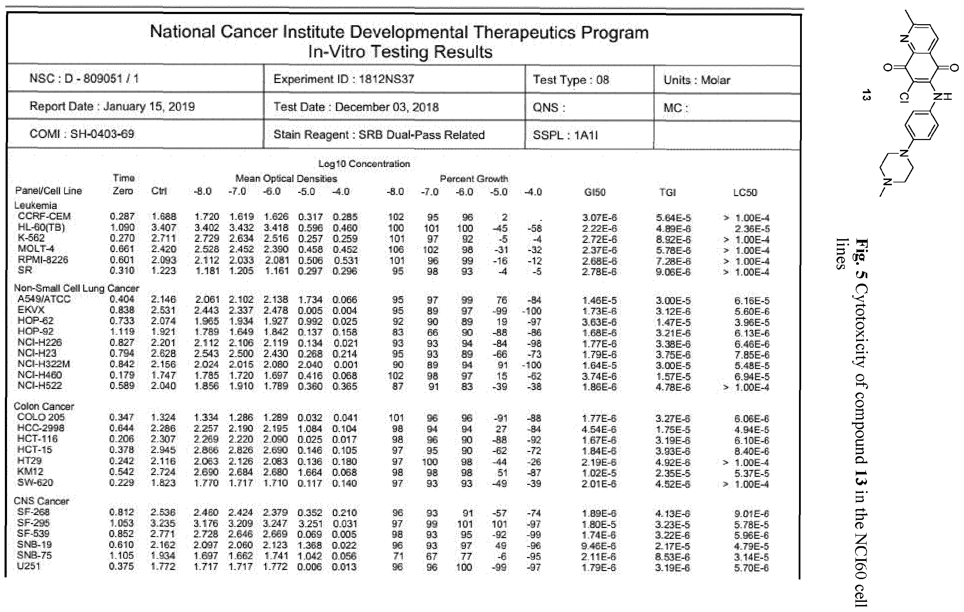

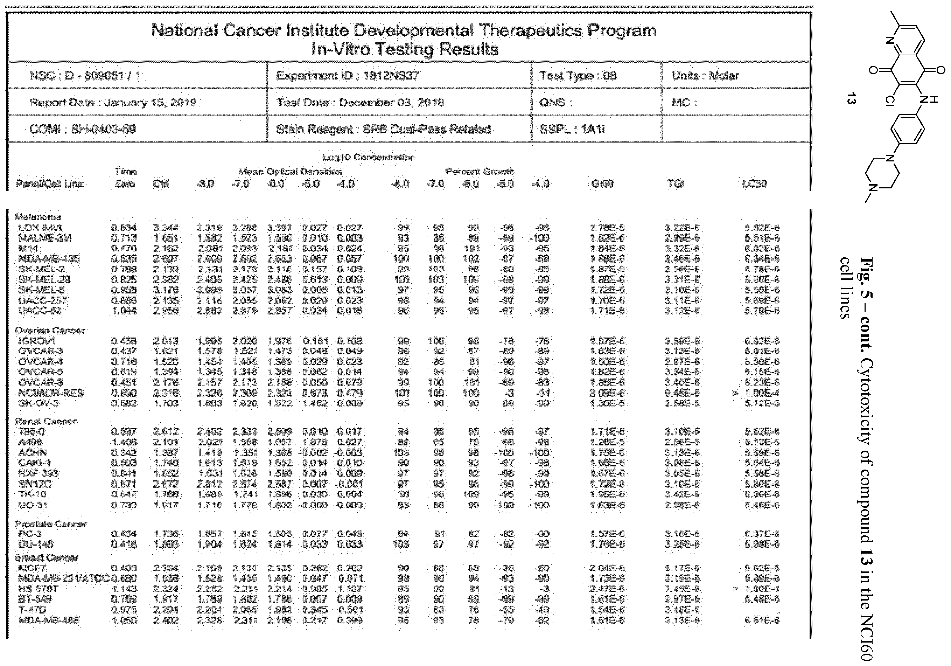

Experiments conducted during the course of developing synthesized various new small molecules having bicyclic and tetracyclic quinone scaffolds with improved metabolic stability, solubility, and pharmacokinetic properties. Indeed, as described in Example III, various compounds of the present invention were shown to inhibit the growth of various cancer cell lines (see, Tables 2, 3 4 and 5; Figures 1, 2, 3, 4, and 5).

As such, the present invention provides a new class of small-molecules having a bicyclic and tetracyclic quinone structure which have improved metabolic stability, solubility, and pharmacokinetic properties. The invention further provides uses for such small-molecules as therapeutics for the treatment of cancer.

Accordingly, the present invention contemplates that exposure of animals (e.g., humans) suffering from cancer (e.g., and/or cancer related disorders) to therapeutically effective amounts of drug(s) having a bicyclic and tetracyclic quinone structure will inhibit the growth of cancer cells or supporting cells outright and/or render such cells as a population more susceptible to the cell death-inducing activity of cancer therapeutic drugs or radiation therapies.

The present invention contemplates that the compounds of the present invention (e.g., compounds having a bicyclic and tetracyclic quinone structure) satisfy an unmet need for the treatment of multiple cancer types, either when they are for use administered as monotherapy to induce cell growth inhibition, apoptosis and/or cell cycle arrest in cancer cells, or when they are for use administered in a temporal relationship with additional agent(s), such as other cell death-inducing or cell cycle disrupting cancer therapeutic drugs or radiation therapies (combination therapies), so as to render a greater proportion of the cancer cells or supportive cells susceptible to executing the apoptosis program compared to the corresponding proportion of cells in an animal treated only with the cancer therapeutic drug or radiation therapy alone.

In certain embodiments of the invention, a therapeutically effective amount of a compound of the present invention and a course of an anticancer agent for use in combination treatment of animals produces a greater tumor response and clinical benefit in such animals compared to those treated with the compound or anticancer drugs/radiation alone. Since the doses for all approved anticancer drugs and radiation treatments are known, the present invention contemplates the various combinations of them with the present compounds.

Certain compounds having an indole scaffold of the present invention may exist as stereoisomers including optical isomers. The invention includes all stereoisomers, both as pure individual stereoisomer preparations and enriched preparations of each, and both the racemic mixtures of such stereoisomers as well as the individual diastereomers and enantiomers that may be separated according to methods that are well known to those of skill in the art.

Compounds having a bicyclic and tetracyclic quinone structure of Formulas XII, XIII, XIV, and XV are provided: 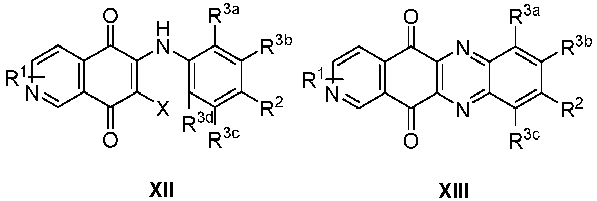 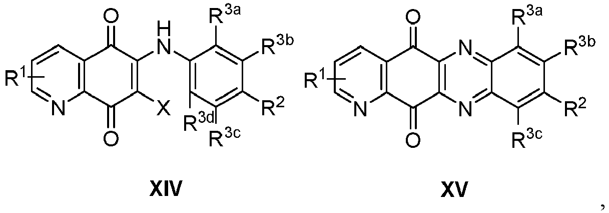 including pharmaceutically acceptable salts, and/or solvates thereof; wherein
R¹ is selected from the group consisting of hydrogen, or optionally substituted alkyl, or halogen;
R² is substituted selected from the group consisting of: 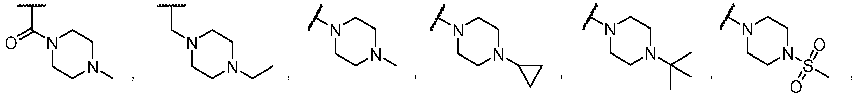 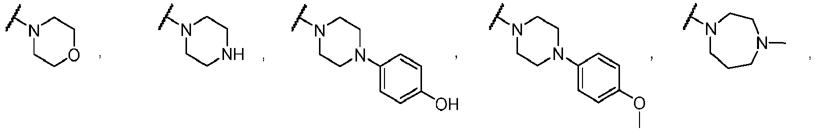 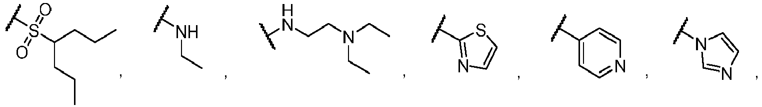 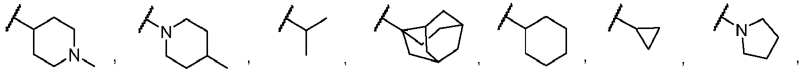 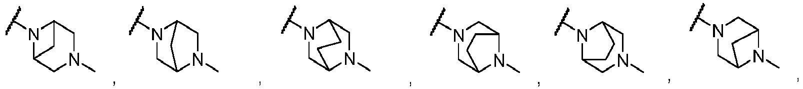 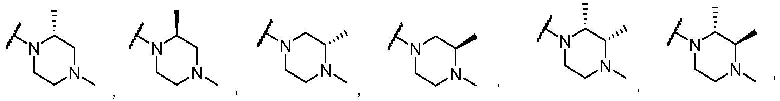 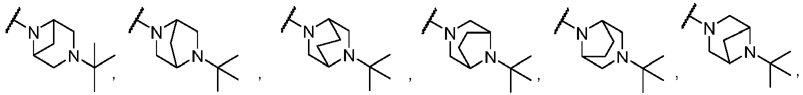 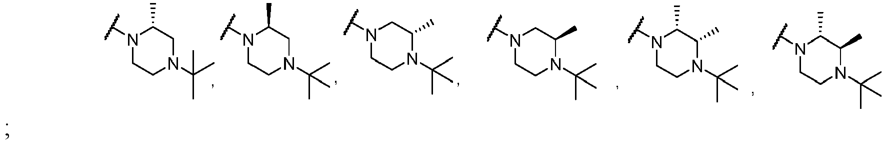
R^{3a}, R^{3b}, R^{3c}, or R^{3d} is selected from the group consisting of hydrogen, halogen, methyl, methoxy, trifluoromethyl, hydroxyl or cyano;
X is selected from the group consisting of hydrogen, halogen, amino, heterocycloalkyl, or hydroxyl.

In some embodiments, R¹ is selected from the group consisting of hydrogen, halo, alkyl, heteroalkyl, optionally substituted C₆-C₁₄ aryl, and optionally substituted 5 to14 membered heteroaryl.

In some embodiments, R¹ is selected from the group consisting of:

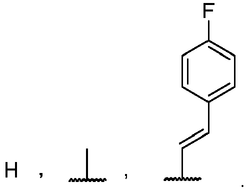

In some embodiments, R² is optionally selected from the group consisting of:

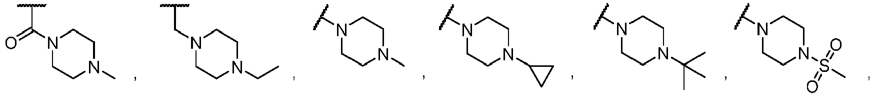

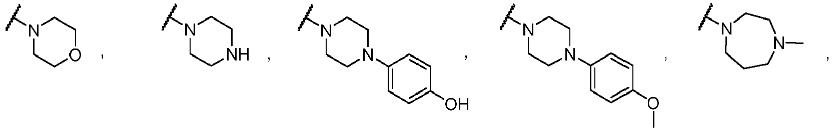

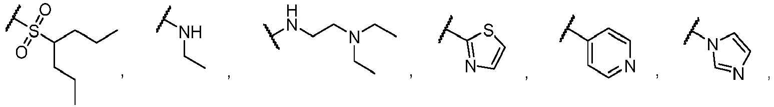

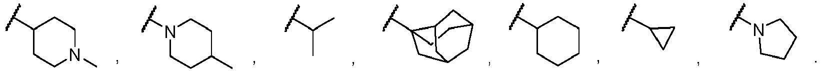

In some embodiments, R^{3a}, R^{3b}, R^{3c}, or R^{3d} is optionally monosubstituted or polysubstituted selected from the group consisting of:

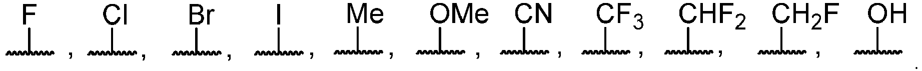

In some embodiments, X is selected from the group consisting of hydrogen, halogen, amino, heterocycloalkyl, or hydroxyl.

In some embodiments, X is optionally selected from the group consisting of:

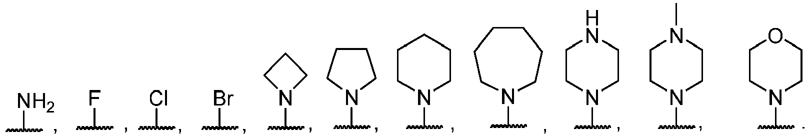

In some embodiments, compounds shown in Table 1 are contemplated for Formulas XII-XV.

**Table 1. Structures of representative compounds contemplated for Formulas XII-XV.**

| **Cpd. No.** | **Structure** | **Name** |
|---|---|---|
| **1** | 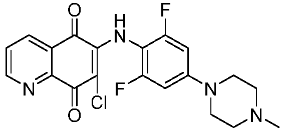 | 7-chloro-6-((2,6-difluoro-4-(4-methylpiperazin-1-yl)phenyl)amino)quinoline-5,8-dione |
| **2** | 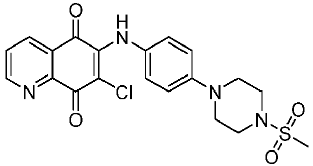 | 7-chloro-6-((4-(4-(methylsulfonyl)piperazin-1 - yl)phenyl)amino)quinoline-5,8-dione |
| **3** | 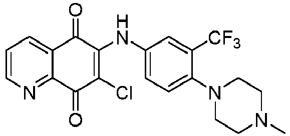 | 7-chloro-6-((4-(4-methylpiperazin-1-yl)-3-(trifluoromethyl)phenyl)amino)quinoline-5,8-dione |
| **4** | 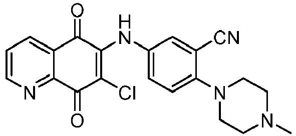 | 5-((7-chloro-5,8-dioxo-5,8-dihydroquinolin-6-yl)amino)-2-(4-methylpiperazin-1-yl)benzonitrile |
| **5** | 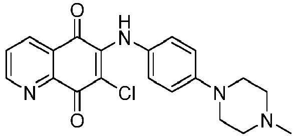 | 7-chloro-6-((4-(4-methylpiperazin-1-yl)phenyl)amino)quinoline-5,8-dione |
| **6** | 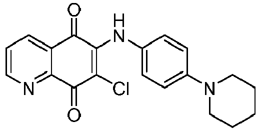 | 7-chloro-6-((4-(piperidin-1-yl)phenyl)amino)quinoline-5,8-dione |
| **7** | 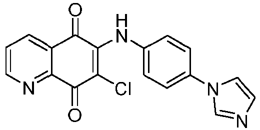 | 6-((4-(1H-imidazol-1-yl)phenyl)amino)-7-chloroquinoline-5,8-dione |
| **8** | 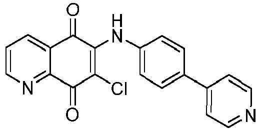 | 7-chloro-6-((4-(pyridin-4-yl)phenyl)amino)quinoline-5,8-dione |
| **9** | 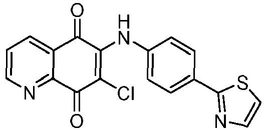 | 7-chloro-6-((4-(thiazol-2-yl)phenyl)amino)quinoline-5,8-dione |
| **10** | 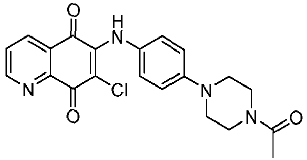 | 6-((4-(4-acetylpiperazin-1- yl)phenyl)amino)-7-chloroquinoline-5,8-dione |
| **11** | 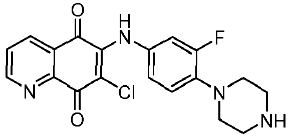 | 7-chloro-6-((3-fluoro-4-(piperazin-1-yl)phenyl)amino)quinoline-5,8-dione |
| **12** | 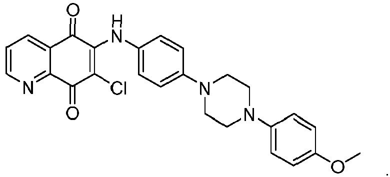 | 7-chloro-6-((4-(4-(4-methoxyphenyl)piperazin-1- yl)phenyl)amino)quinoline-5,8-dione |
| **13** | 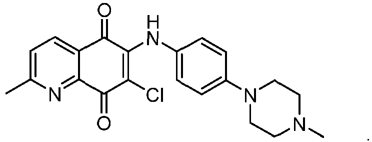 | 7-chloro-2-methyl-6-((4-(4-methylpiperazin-1-yl)phenyl)amino)quinoline-5,8-dione |
| **14** | 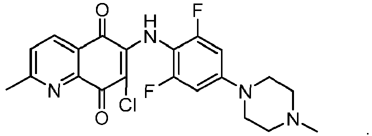 | 7-chloro-6-((2,6-difluoro-4-(4-methylpiperazin-1-yl)phenyl)amino)-2-methylquinoline-5,8-dione |
| **15** | 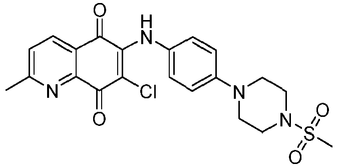 | 7-chloro-2-methyl-6-((4-(4-(methylsulfonyl)piperazin-1- yl)phenyl)amino)quinoline-5,8-dione |
| **16** | 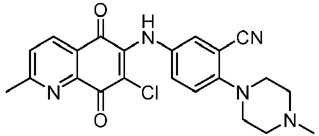 | 5-((7-chloro-2-methyl-5,8-dioxo-5,8-dihydroquinolin-6-yl)amino)-2-(4-methylpiperazin-1-yl)benzonitrile |
| **17** | 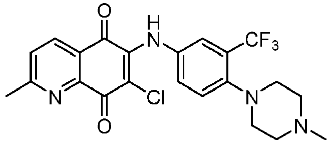 | 7-chloro-2-methyl-6-((4-(4-methylpiperazin-1-yl)-3-(trifluoromethyl)phenyl)amino)quinoline-5,8-dione |
| **18** | 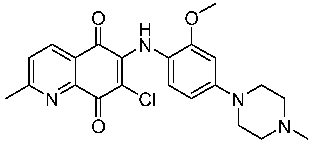 | 7-chloro-6-((2-methoxy-4-(4-methylpiperazin-1-yl)phenyl)amino)-2-methylquinoline-5,8-dione |
| **19** | 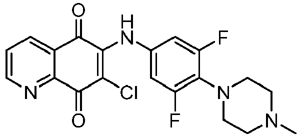 | 7-chloro-6-((3,5-difluoro-4-(4-methylpiperazin-1-yl)phenyl)amino)quinoline-5,8-dione |
| **20** | 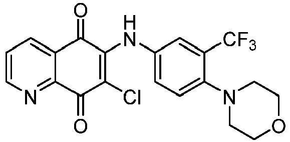 | 7-chloro-6-((4-morpholino-3-(trifluoromethyl)phenyl)amino)quinoline-5,8-dione |
| **21** | 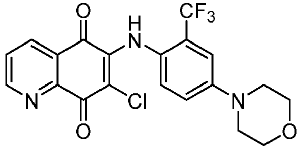 | 7-chloro-6-((4-morpholino-2-(trifluoromethyl)phenyl)amino)quinoline-5,8-dione |
| **22** | 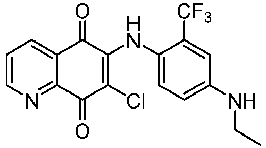 | 7-chloro-6-((4-(ethylamino)-2-(trifluoromethyl)phenyl)amino)quinoline-5,8-dione |
| **23** | 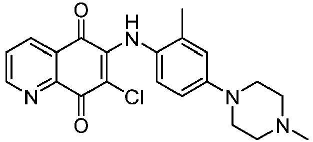 | 7-chloro-6-((2-methyl-4-(4-methylpiperazin-1-yl)phenyl)amino)quinoline-5,8-dione |
| **24** | 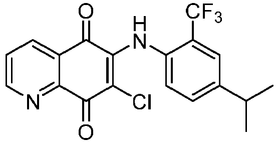 | 7-chloro-6-((4-isopropyl-2-(trifluoromethyl)phenyl)amino)quinoline-5,8-dione |
| **25** | 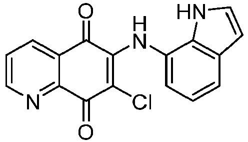 | 6-((1H-indol-7-yl)amino)-7-chloroquinoline-5,8-dione |
| **26** | 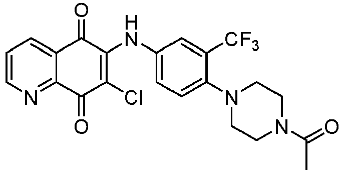 | 6-((4-(4-acetylpiperazin-1-yl)-3-(trifluoromethyl)phenyl)amino)-7-chloroquinoline-5,8-dione |
| **27** | 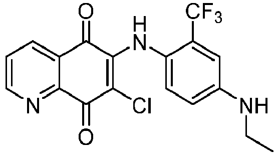 | 7-chloro-6-((4-(ethylamino)-2-(trifluoromethyl)phenyl)amino)quinoline-5,8-dione |
| **28** | 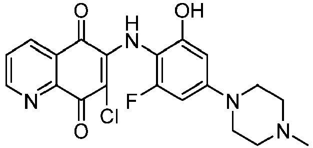 | 7-chloro-6-((2-fluoro-6-hydroxy-4-(4-methylpiperazin-1-yl)phenyl)amino)quinoline-5,8-dione |
| **29** | 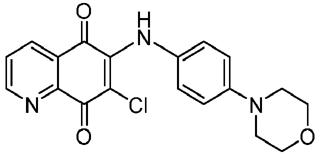 | 7-chloro-6-((4-morpholinophenyl)amino)quinoline-5, 8-dione |
| **30** | 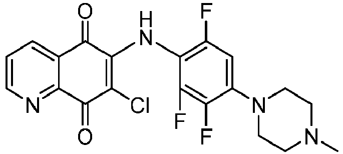 | 7-chloro-6-((2,3,6-trifluoro-4-(4-methylpiperazin-1-yl)phenyl)amino)quinoline-5,8-dione |
| **42** | 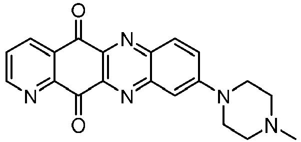 | 9-(4-methylpiperazin-1-yl)pyrido[2,3-b]phenazine-5,12-dione |
| **43** | 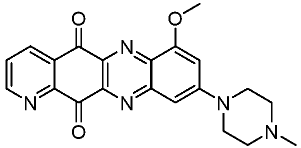 | 7-methoxy-9-(4-methylpiperazin-1-yl)pyrido[2,3-b]phenazine-5,12-dione |
| **44** | 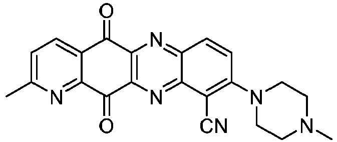 | 2-methyl-9-(4-methylpiperazin-1-yl)-5,12-dioxo-5,12-dihydropyrido[2,3-b]phenazine-10-carbonitrile |
| **45** | 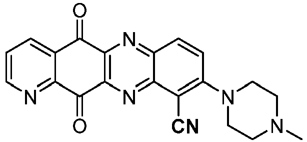 | 9-(4-methylpiperazin-1-yl)-5,12-dioxo-5,12-dihydropyrido[2,3-b]phenazine-10-carbonitrile |
| **46** | 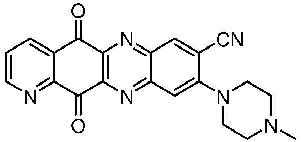 | 9-(4-methylpiperazin-1-yl)-5,12-dioxo-5,12-dihydropyrido[2,3-b]phenazine-8-carbonitrile |
| **47** | 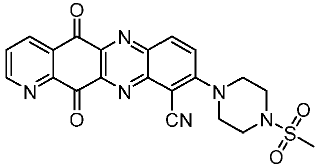 | 9-(4-(methylsulfonyl)piperazin-1-yl)-5,12-dioxo-5,12-dihydropyrido[2,3-b]phenazine-10-carbonitrile |
| **48** | 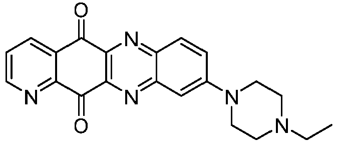 | 9-(4-ethylpiperazin-1-yl)pyrido[2,3-b]phenazine-5,12-dione |
| **49** | 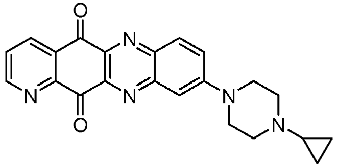 | 9-(4-cyclopropylpiperazin-1-yl)pyrido[2,3-b]phenazine-5,12-dione |
| **50** | 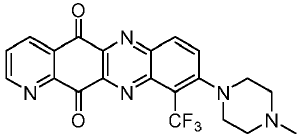 | 9-(4-methylpiperazin-1 -yl)-10-(trifluoromethyl)pyrido[2,3-b]phenazine-5,12-dione |
| **51** | 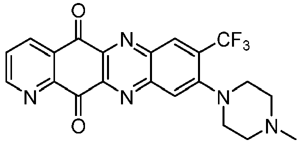 | 9-(4-methylpiperazin-1-yl)-8-(trifluoromethyl)pyrido[2,3-b]phenazine-5,12-dione |
| **52** | 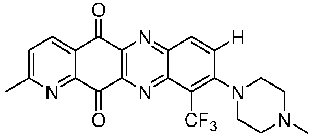 | 2-methyl-9-(4-methylpiperazin-1-yl)-10-(trifluoromethyl)pyrido[2,3 -b]phenazine-5,12-dione |
| **53** | 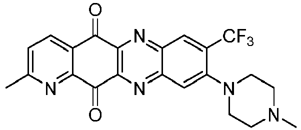 | 2-methyl-9-(4-methylpiperazin-1-yl)-8-(trifluoromethyl)pyrido[2,3-b]phenazine-5,12-dione |
| **54** | 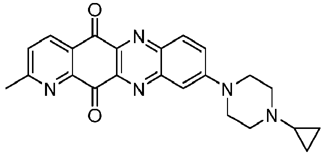 | 9-(4-cyclopropylpiperazin-1-yl)-2-methylpyrido[2,3-b]phenazine-5,12-dione |
| **55** | 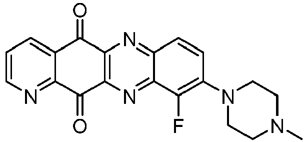 | 10-fluoro-9-(4-methylpiperazin-1-yl)pyrido[2,3-b]phenazine-5,12-dione |
| **56** | 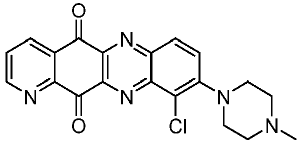 | 10-chloro-9-(4-methylpiperazin-1- yl)pyrido[2,3-b]phenazine-5,12-dione |
| **57** | 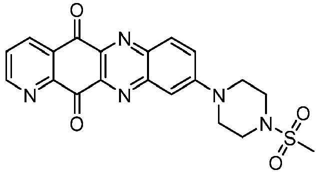 | 9-(4-(methylsulfonyl)piperazin-1-yl)pyrido[2,3-b]phenazine-5,12-dione |
| **58** | 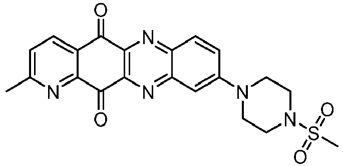 | 2-methyl-9-(4-(methylsulfonyl)piperazin-1-yl)pyrido[2,3-b]phenazine-5,12-dione |
| **59** | 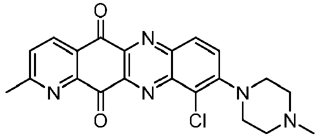 | 10-chloro-2-methyl-9-(4-methylpiperazin-1-yl)pyrido[2,3-b]phenazine-5,12-dione |
| **60** | 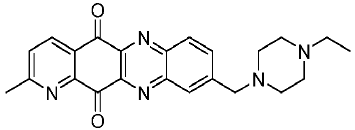 | 9-((4-ethylpiperazin-1-yl)methyl)-2-methylpyrido[2,3-b]phenazine-5,12-dione |
| **61** | 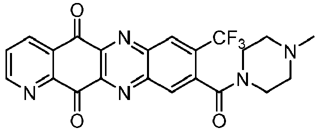 | 9-(4-methylpiperazine-1-carbonyl)-8-(trifluoromethyl)pyrido[2,3-b]phenazine-5,12-dione |
| **62** | 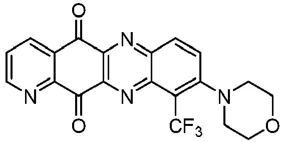 | 9-morpholino-10-(trifluoromethyl)pyrido[2,3 -b]phenazine-5,12-dione |
| **63** | 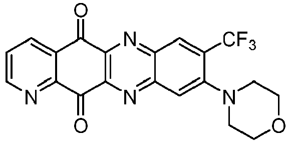 | 9-morpholino-8-(trifluoromethyl)pyrido[2,3 -b]phenazine-5,12-dione |
| **64** | 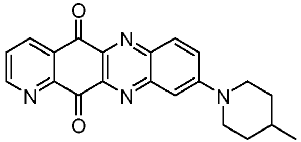 | 9-(4-methylpiperidin-1-yl)pyrido[2,3-b]phenazine-5,12-dione |
| **65** | 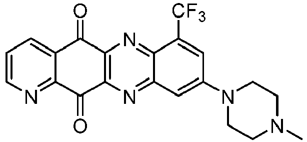 | 9-(4-methylpiperazin-1-yl)-7-(trifluoromethyl)pyrido[2,3-b]phenazine-5,12-dione |
| **66** | 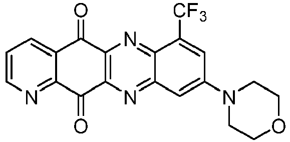 | 9-morpholino-7-(trifluoromethyl)pyrido[2,3-b]phenazine-5,12-dione |
| **67** | 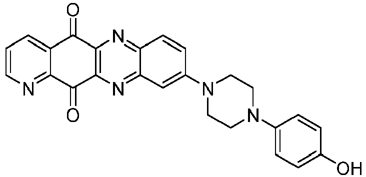 | 9-(4-(4-hydroxyphenyl)piperazin-1-yl)pyrido[2,3-b]phenazine-5,12-dione |
| **68** | 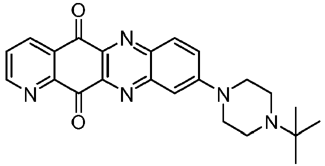 | 9-(4-(tert-butyl)piperazin-1- yl)pyrido[2,3-b]phenazine-5,12-dione |
| **69** | 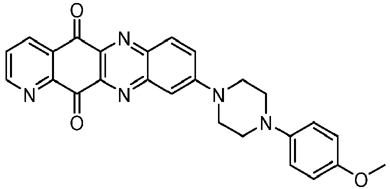 | 9-(4-(4-methoxyphenyl)piperazin-1-yl)pyrido[2,3-b]phenazine-5,12-dione |
| **70** | 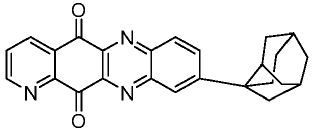 | 9-((3 aS, 5 S,7aS)-octahydro-7aH-2, 5-methanoinden-7a-yl)pyrido[2,3-b]phenazine-5,12-dione |
| **71** | 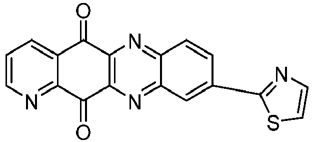 | 9-(thiazol-2-yl)pyrido[2,3-b]phenazine-5,12-dione |
| **72** | 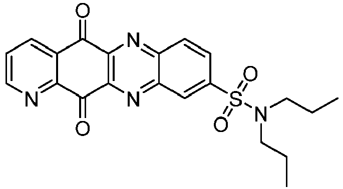 | 5,12-dioxo-N,N-dipropyl-5,12-dihydropyrido[2,3-b]phenazine-9-sulfonamide |
| **73** | 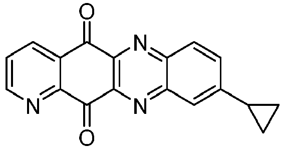 | 9-cyclopropylpyrido[2,3-b]phenazine-5,12-dione |
| **74** | 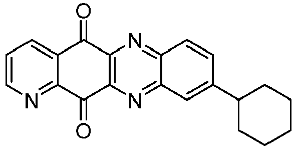 | 9-cyclohexylpyrido[2,3-b]phenazine-5,12-dione |
| **75** | 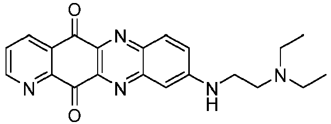 | 9-((2-(diethylamino)ethyl)amino)pyrido[2,3-b]phenazine-5,12-dione |
| **78** | 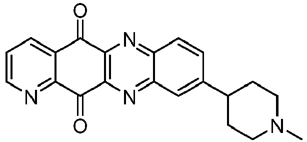 | 9-(1-methylpiperidin-4-yl)pyrido[2,3-b]phenazine-5,12-dione |
| **79** | 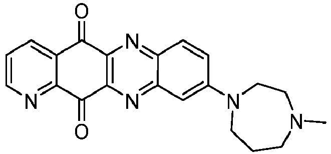 | 9-(4-methyl-1,4-diazepan-1-yl)pyrido[2,3-b]phenazine-5,12-dione |
| **80** | 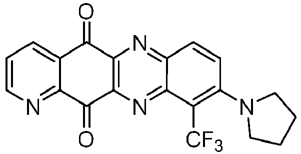 | 9-(pyrrolidin-1-yl)-10-(trifluoromethyl)pyrido[2,3 -b]phenazine-5,12-dione |
| **81** | 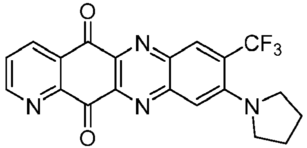 | 9-(pyrrolidin-1-yl)-8-(trifluoromethyl)pyrido[2,3-b]phenazine-5,12-dione |
| **82** | 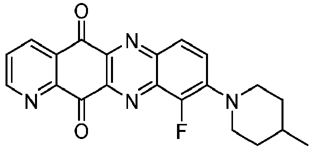 | 10-fluoro-9-(4-methylpiperidin-1-yl)pyrido[2,3-b]phenazine-5,12-dione |
| **83** | 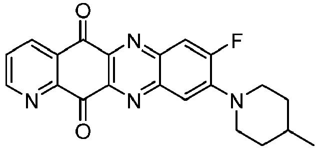 | 8-fluoro-9-(4-methylpiperidin-1-yl)pyrido[2,3-b]phenazine-5,12-dione |
| **84** | 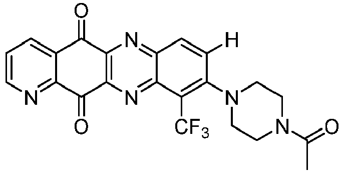 | 9-(4-acetylpiperazin-1-yl)-10-(trifluoromethyl) pyrido[2,3-b]phenazine-5,12-dione |
| **85** | 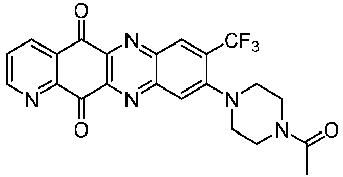 | 9-(4-acetylpiperazin-1-yl)-8-(trifluoromethyl)pyrido[2,3-b]phenazine-5,12-dione |
| **86** | 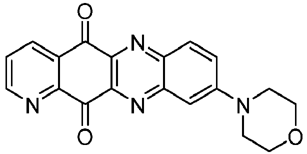 | 9-morpholinopyrido[2,3-b]phenazine-5,12-dione |
| **87** | 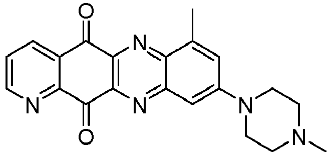 | 7-methyl-9-(4-methylpiperazin-1-yl)pyrido[2,3-b]phenazine-5,12-dione |
| **88** | 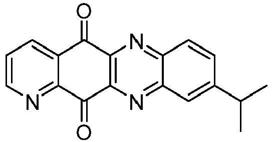 | 9-isopropylpyrido[2,3-b]phenazine-5,12-dione |

In some embodiments, any of the compounds described herein are 2,2,2-trifluoroacetate (TFA) salts.

The invention further provides processes for preparing any of the compounds of the present invention through following at least a portion of the techniques recited in the Experimental Section.

The invention also provides the use of the compounds to induce cell cycle arrest and/or apoptosis in cancer cells. The invention also relates to the use of compounds for sensitizing cells to additional agent(s), such as inducers of apoptosis and/or cell cycle arrest, and chemoprotection of normal cells through the induction of cell cycle arrest prior to treatment with chemotherapeutic agents.

The compounds of the invention are for use in the treatment, amelioration, or prevention of disorders, such as those responsive to induction of apoptotic cell death, e.g., disorders characterized by dysregulation of apoptosis, including hyperproliferative diseases such as cancer. In certain embodiments, the compounds can be used to treat, ameliorate, or prevent cancer that is characterized by resistance to cancer therapies (e.g., those cancer cells which are chemoresistant, radiation resistant, hormone resistant, and the like). In certain embodiments, the cancer is multiple myeloma, acute myeloid leukemia, melanoma, breast cancer, head or neck cancers, colon cancer, lung cancer, ovarian cancer, prostate cancer, and/or pancreatic cancer.

The invention also provides pharmaceutical compositions comprising the compounds of the invention in a pharmaceutically acceptable carrier.

The invention also provides kits comprising a compound of the invention and instructions for administering the compound to an animal. The kits may optionally contain other therapeutic agents, e.g., anticancer agents or apoptosis-modulating agents.

### BRIEF DESCRIPTION OF DRAWINGS

- FIG. 1:: Growth inhibition (% control) of compound **1** in the NCI60 cell lines.
- FIG. 2:: Growth inhibition (% control) of compound **13** in the NCI60 cell lines.
- FIG. 3:: Growth inhibition (% control) of compound **49** in the NCI60 cell lines.
- FIG. 4:: Growth inhibition (% control) of compound **50** in the NCI60 cell lines.
- FIG. 5:: Cytotoxicity of compound **13** in the NCI60 cell lines.

### DEFINITIONS

The term "anticancer agent" as used herein, refer to any therapeutic agents *(e.g.,* chemotherapeutic compounds and/or molecular therapeutic compounds), antisense therapies, radiation therapies, or surgical interventions, used in the treatment of hyperproliferative diseases such as cancer *(e.g.,* in mammals, *e.g..,* in humans).

The term "pharmaceutically acceptable salt" as used herein, refers to any salt *(e.g.,* obtained by reaction with an acid or a base) of a compound of the present invention that is physiologically tolerated in the target animal *(e.g.,* a mammal). Salts of the compounds of the present invention may be derived from inorganic or organic acids and bases. Examples of acids include, but are not limited to, hydrochloric, hydrobromic, sulfuric, nitric, perchloric, fumaric, maleic, phosphoric, glycolic, lactic, salicylic, succinic, toluene-p-sulfonic, tartaric, acetic, citric, methanesulfonic, ethanesulfonic, formic, benzoic, malonic, sulfonic, naphthalene-2-sulfonic, benzenesulfonic acid, and the like. Other acids, such as oxalic, while not in themselves pharmaceutically acceptable, may be employed in the preparation of salts useful as intermediates in obtaining the compounds of the invention and their pharmaceutically acceptable acid addition salts.

Examples of bases include, but are not limited to, alkali metal (*e.g*., sodium) hydroxides, alkaline earth metal (*e.g*., magnesium) hydroxides, ammonia, and compounds of formula NW₄⁺, wherein W is C₁₋₄ alkyl, and the like.

Examples of salts include, but are not limited to: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, flucoheptanoate, glycerophosphate, hemisulfate, heptanoate, hexanoate, chloride, bromide, iodide, 2-hydroxyethanesulfonate, lactate, maleate, mesylate, methanesulfonate, 2-naphthalenesulfonate, nicotinate, oxalate, palmoate, pectinate, persulfate, phenylpropionate, picrate, pivalate, propionate, succinate, tartrate, thiocyanate, tosylate, undecanoate, and the like. Other examples of salts include anions of the compounds of the present invention compounded with a suitable cation such as Na⁺, NH₄⁺, and NW₄⁺ (wherein W is a C₁₋₄ alkyl group), and the like. For therapeutic use, salts of the compounds of the present invention are contemplated as being pharmaceutically acceptable. However, salts of acids and bases that are non-pharmaceutically acceptable may also find use, for example, in the preparation or purification of a pharmaceutically acceptable compound.

The term "solvate" as used herein, refers to the physical association of a compound of the invention with one or more solvent molecules, whether organic or inorganic. This physical association often includes hydrogen bonding. In certain instances, the solvate is capable of isolation, for example, when one or more solvate molecules are incorporated in the crystal lattice of the crystalline solid. "Solvate" encompasses both solution-phase and isolable solvates. Exemplary solvates include hydrates, ethanolates, and methanolates.

The term "therapeutically effective amount," as used herein, refers to that amount of the therapeutic agent sufficient to result in amelioration of one or more symptoms of a disorder, or prevent advancement of a disorder, or cause regression of the disorder. For example, with respect to the treatment of cancer, in one embodiment, a therapeutically effective amount will refer to the amount of a therapeutic agent that decreases the rate of tumor growth, decreases tumor mass, decreases the number of metastases, increases time to tumor progression, or increases survival time by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100%.

The terms "sensitize" and "sensitizing," as used herein, refer to making, through the administration of a first agent (*e.g*., a benzoic acid compound of the invention), an animal or a cell within an animal more susceptible, or more responsive, to the biological effects (*e.g*., promotion or retardation of an aspect of cellular function including, but not limited to, cell division, cell growth, proliferation, invasion, angiogenesis, necrosis, or apoptosis) of a second agent. The sensitizing effect of a first agent on a target cell can be measured as the difference in the intended biological effect (*e.g*., promotion or retardation of an aspect of cellular function including, but not limited to, cell growth, proliferation, invasion, angiogenesis, or apoptosis) observed upon the administration of a second agent with and without administration of the first agent. The response of the sensitized cell can be increased by at least about 10%, at least about 20%, at least about 30%, at least about 40%, at least about 50%, at least about 60%, at least about 70%, at least about 80%, at least about 90%, at least about 100%, at least about 150%, at least about 200%, at least about 250%, at least 300%, at least about 350%, at least about 400%, at least about 450%, or at least about 500% over the response in the absence of the first agent.

The term "dysregulation of apoptosis," as used herein, refers to any aberration in the ability of (*e.g*., predisposition) a cell to undergo cell death via apoptosis. Dysregulation of apoptosis is associated with or induced by a variety of conditions, non-limiting examples of which include, autoimmune disorders (*e.g*., systemic lupus erythematosus, rheumatoid arthritis, graft-versus-host disease, myasthenia gravis, or Sjögren's syndrome), chronic inflammatory conditions (*e.g*., psoriasis, asthma or Crohn's disease), hyperproliferative disorders (*e.g.,* tumors, B cell lymphomas, or T cell lymphomas), viral infections (*e.g.,* herpes, papilloma, or HIV), and other conditions such as osteoarthritis and atherosclerosis.

The term "hyperproliferative disease," as used herein, refers to any condition in which a localized population of proliferating cells in an animal is not governed by the usual limitations of normal growth. Examples of hyperproliferative disorders include tumors, neoplasms, lymphomas and the like. A neoplasm is said to be benign if it does not undergo invasion or metastasis and malignant if it does either of these. A "metastatic" cell means that the cell can invade and destroy neighboring body structures. Hyperplasia is a form of cell proliferation involving an increase in cell number in a tissue or organ without significant alteration in structure or function. Metaplasia is a form of controlled cell growth in which one type of fully differentiated cell substitutes for another type of differentiated cell.

The pathological growth of activated lymphoid cells often results in an autoimmune disorder or a chronic inflammatory condition. As used herein, the term "autoimmune disorder" refers to any condition in which an organism produces antibodies or immune cells which recognize the organism's own molecules, cells or tissues. Non-limiting examples of autoimmune disorders include autoimmune hemolytic anemia, autoimmune hepatitis, Berger's disease or IgA nephropathy, celiac sprue, chronic fatigue syndrome, Crohn's disease, dermatomyositis, fibromyalgia, graft versus host disease, Grave's disease, Hashimoto's thyroiditis, idiopathic thrombocytopenia purpura, lichen planus, multiple sclerosis, myasthenia gravis, psoriasis, rheumatic fever, rheumatic arthritis, scleroderma, Sjögren's syndrome, systemic lupus erythematosus, type 1 diabetes, ulcerative colitis, vitiligo, and the like.

The term "neoplastic disease," as used herein, refers to any abnormal growth of cells being either benign (non-cancerous) or malignant (cancerous).

The term "normal cell," as used herein, refers to a cell that is not undergoing abnormal growth or division. Normal cells are non-cancerous and are not part of any hyperproliferative disease or disorder.

The term "anti-neoplastic agent," as used herein, refers to any compound that retards the proliferation, growth, or spread of a targeted (*e.g*., malignant) neoplasm.

The terms "prevent," "preventing," and "prevention," as used herein, refer to a decrease in the occurrence of pathological cells (*e.g*., hyperproliferative or neoplastic cells) in an animal. The prevention may be complete, *e.g*., the total absence of pathological cells in a subject. The prevention may also be partial, such that the occurrence of pathological cells in a subject is less than that which would have occurred without the present invention.

The term "pharmaceutically acceptable carrier" or "pharmaceutically acceptable vehicle" encompasses any of the standard pharmaceutical carriers, solvents, surfactants, or vehicles. Suitable pharmaceutically acceptable vehicles include aqueous vehicles and nonaqueous vehicles. Standard pharmaceutical carriers and their formulations are described in Remington's Pharmaceutical Sciences, Mack Publishing Co., Easton, PA, 19th ed. 1995.

### DETAILED DESCRIPTION OF THE INVENTION

Experiments conducted during the course of developing synthesized various new small molecules having bicyclic and tetracyclic quinone scaffolds with improved metabolic stability, solubility, and pharmacokinetic properties. Indeed, as described in Example III, various compounds of the present invention were shown to inhibit the growth of various cancer cell lines (see, Tables 2, 3, 4 and 5; Figures 1, 2, 3, 4, and 5).

As such, the present invention provides a new class of small-molecules having a bicyclic and tetracyclic quinone structure which have improved metabolic stability, solubility, and pharmacokinetic properties. The invention further provides for such small-molecules for use as therapeutics in the treatment of cancer.

Compounds having a bicyclic and tetracyclic quinone structure of Formulas XII, XIII, XIV, and XV are provided: 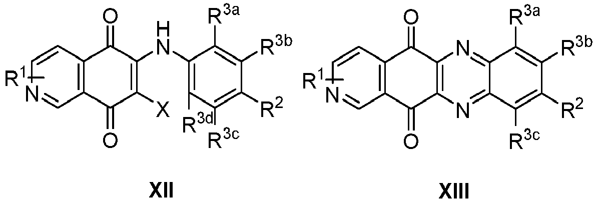 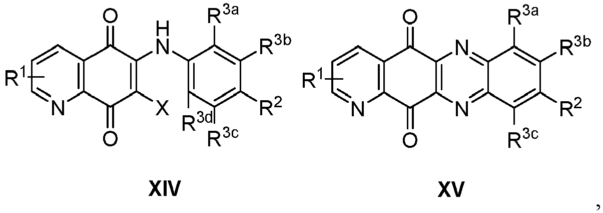 including pharmaceutically acceptable salts, and/or solvates thereof; wherein
R¹ is selected from the group consisting of hydrogen, or optionally substituted alkyl, or halogen;
R² is substituted selected from the group consisting of: 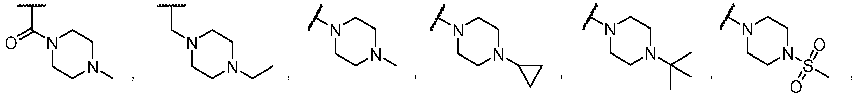 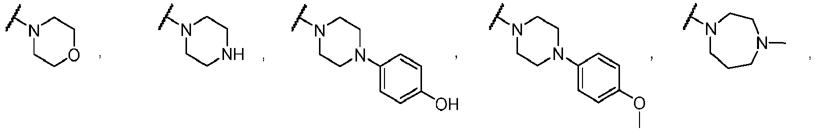 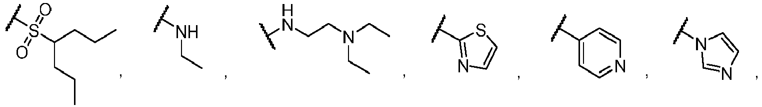 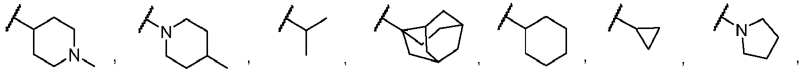 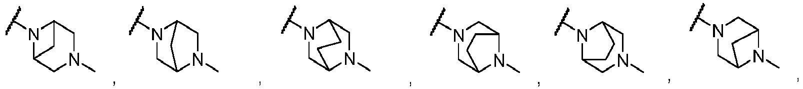 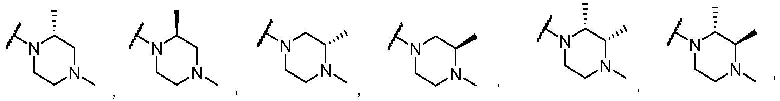 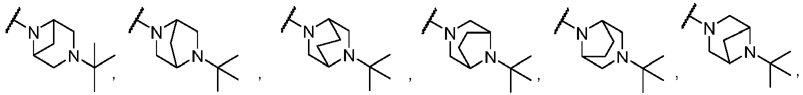 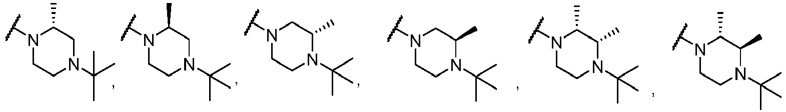
R^{3a}, R^{3b}, R^{3c}, or R^{3d} is selected from the group consisting of hydrogen, halogen, methyl, methoxy, trifluoromethyl, hydroxyl or cyano;
X is selected from the group consisting of hydrogen, halogen, amino, heterocycloalkyl, or hydroxyl.

In some embodiments, R¹ is selected from the group consisting of hydrogen, halo, alkyl, heteroalkyl, optionally substituted C₆-C₁₄ aryl, and optionally substituted 5 to14 membered heteroaryl.

In some embodiments, R¹ is selected from the group consisting of:

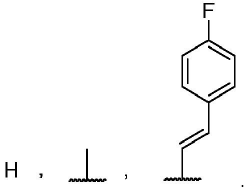

In some embodiments, R² is optionally selected from the group consisting of:

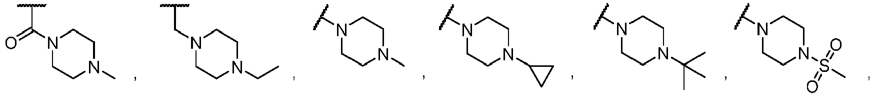

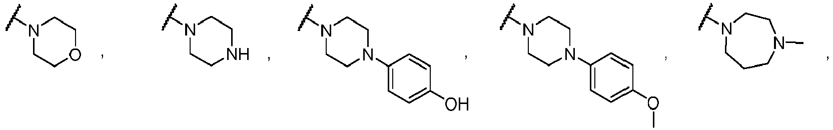

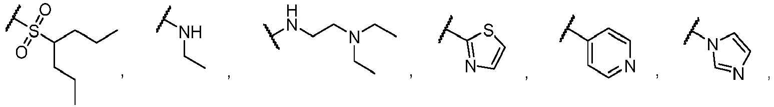

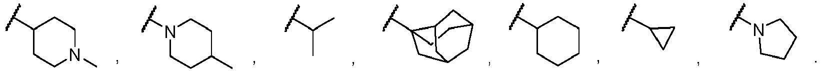

In some embodiments, R^{3a}, R^{3b}, R^{3c}, or R^{3d} is optionally monosubstituted or polysubstituted selected from the group consisting of:

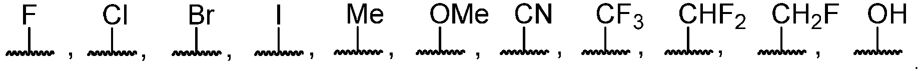

In some embodiments, X is selected from the group consisting of hydrogen, halogen, amino, heterocycloalkyl, or hydroxyl.

In some embodiments, X is optionally selected from the group consisting of:

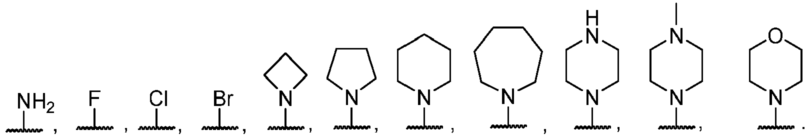

In some embodiments, compounds shown in Table 1 are contemplated for Formulas XII-XV.

An important aspect of the present invention is that compounds of the invention induce cell cycle arrest and/or apoptosis and also potentiate the induction of cell cycle arrest and/or apoptosis either alone or in response to additional apoptosis induction signals. Therefore, it is contemplated that these compounds sensitize cells to induction of cell cycle arrest and/or apoptosis, including cells that are resistant to such inducing stimuli. The compounds of the present invention (e.g., indole (or similar) compounds) can be used to induce apoptosis in any disorder that can be treated, ameliorated, or prevented by the induction of apoptosis. In one embodiment, the modulators (e.g., inhibitors) can be used to induce apoptosis in cells through induction of reactive oxygen species in the relevant cell(s).

In some embodiments, the compositions of the present invention are for use in treating diseased cells, tissues, organs, or pathological conditions and/or disease states in an animal *(e.g.,* a mammalian patient including, but not limited to, humans and veterinary animals). In this regard, various diseases and pathologies are amenable to treatment or prophylaxis using the present methods and compositions. A non-limiting exemplary list of these diseases and conditions includes, but is not limited to, pancreatic cancer, breast cancer, prostate cancer, lymphoma, skin cancer, colon cancer, melanoma, malignant melanoma, ovarian cancer, brain cancer, primary brain carcinoma, head and neck cancer, glioma, glioblastoma, liver cancer, bladder cancer, non-small cell lung cancer, head or neck carcinoma, breast carcinoma, ovarian carcinoma, lung carcinoma, small-cell lung carcinoma, Wilms' tumor, cervical carcinoma, testicular carcinoma, bladder carcinoma, pancreatic carcinoma, stomach carcinoma, colon carcinoma, prostatic carcinoma, genitourinary carcinoma, thyroid carcinoma, esophageal carcinoma, myeloma, multiple myeloma, adrenal carcinoma, renal cell carcinoma, endometrial carcinoma, adrenal cortex carcinoma, malignant pancreatic insulinoma, malignant carcinoid carcinoma, choriocarcinoma, mycosis fungoides, malignant hypercalcemia, cervical hyperplasia, leukemia, acute lymphocytic leukemia, chronic lymphocytic leukemia, acute myelogenous leukemia, chronic myelogenous leukemia, chronic granulocytic leukemia, acute granulocytic leukemia, hairy cell leukemia, neuroblastoma, rhabdomyosarcoma, Kaposi's sarcoma, polycythemia vera, essential thrombocytosis, Hodgkin's disease, non-Hodgkin's lymphoma, soft-tissue sarcoma, osteogenic sarcoma, primary macroglobulinemia, and retinoblastoma, and the like, T and B cell mediated autoimmune diseases; inflammatory diseases; infections; hyperproliferative diseases; AIDS; degenerative conditions, vascular diseases, and the like. In some embodiments, the cancer cells being treated are metastatic. In other embodiments, the cancer cells being treated are resistant to anticancer agents.

Some embodiments of the present invention provide compositions or use in the treatment, amelioration, or prevention of a hyperproliferative disease in a patient, wherein an effective amount of a compound of the invention and at least one additional therapeutic agent (including, but not limited to, chemotherapeutic antineoplastics, apoptosis-modulating agents, antimicrobials, antivirals, antifungals, and anti-inflammatory agents) and/or therapeutic technique (*e.g*., surgical intervention, and/or radiotherapies) is administered . In a particular embodiment, the additional therapeutic agent(s) is an anticancer agent.

A number of suitable anticancer agents are contemplated for use in the methods of the present invention. Indeed, the present invention contemplates, but is not limited to, administration of numerous anticancer agents such as: agents that induce apoptosis; polynucleotides (*e.g*., anti-sense, ribozymes, siRNA); polypeptides (*e.g*., enzymes and antibodies); biological mimetics; alkaloids; alkylating agents; antitumor antibiotics; antimetabolites; hormones; platinum compounds; monoclonal or polyclonal antibodies (*e.g*., antibodies conjugated with anticancer drugs, toxins, defensins), toxins; radionuclides; biological response modifiers (*e.g*., interferons (*e.g*., IFN-α) and interleukins (*e.g*., IL-2)); adoptive immunotherapy agents; hematopoietic growth factors; agents that induce tumor cell differentiation (*e.g*., all-trans-retinoic acid); gene therapy reagents (*e.g*., antisense therapy reagents and nucleotides); tumor vaccines; angiogenesis inhibitors; proteosome inhibitors: NF-KB modulators; anti-CDK compounds; HDAC inhibitors; and the like. Numerous other examples of chemotherapeutic compounds and anticancer therapies suitable for co-administration with the disclosed compounds are known to those skilled in the art.

In certain embodiments, anticancer agents comprise agents that induce or stimulate apoptosis. Agents that induce apoptosis include, but are not limited to, radiation (*e.g*., X-rays, gamma rays, UV); tumor necrosis factor (TNF)-related factors (*e.g*., TNF family receptor proteins, TNF family ligands, TRAIL, antibodies to TRAIL-R1 or TRAIL-R2); kinase inhibitors (*e.g*., epidermal growth factor receptor (EGFR) kinase inhibitor, vascular growth factor receptor (VGFR) kinase inhibitor, fibroblast growth factor receptor (FGFR) kinase inhibitor, platelet-derived growth factor receptor (PDGFR) kinase inhibitor, and Bcr-Abl kinase inhibitors (such as GLEEVEC)); antisense molecules; antibodies (*e.g*., HERCEPTIN, RITUXAN, ZEVALIN, and AVASTIN); anti-estrogens (*e.g*., raloxifene and tamoxifen); anti-androgens (*e.g*., flutamide, bicalutamide, finasteride, aminoglutethamide, ketoconazole, and corticosteroids); cyclooxygenase 2 (COX-2) inhibitors (*e.g*., celecoxib, meloxicam, NS-398, and non-steroidal anti-inflammatory drugs (NSAIDs)); anti-inflammatory drugs (*e.g*., butazolidin, DECADRON, DELTASONE, dexamethasone, dexamethasone intensol, DEXONE, HEXADROL, hydroxychloroquine, METICORTEN, ORADEXON, ORASONE, oxyphenbutazone, PEDIAPRED, phenylbutazone, PLAQUENIL, prednisolone, prednisone, PRELONE, and TANDEARIL); and cancer chemotherapeutic drugs (*e.g*., irinotecan (CAMPTOSAR), CPT-11, fludarabine (FLUDARA), dacarbazine (DTIC), dexamethasone, mitoxantrone, MYLOTARG, VP-16, cisplatin, carboplatin, oxaliplatin, 5-FU, doxorubicin, gemcitabine, bortezomib, gefitinib, bevacizumab, TAXOTERE or TAXOL); cellular signaling molecules; ceramides and cytokines; staurosporine, and the like.

In still other embodiments, the compositions of the present invention provide a compound of the invention and at least one anti-hyperproliferative or antineoplastic agent selected from alkylating agents, antimetabolites, and natural products (*e.g*., herbs and other plant and/or animal derived compounds).

Alkylating agents suitable for use in the present compositions and methods include, but are not limited to: 1) nitrogen mustards (*e.g*., mechlorethamine, cyclophosphamide, ifosfamide, melphalan (L-sarcolysin); and chlorambucil); 2) ethylenimines and methylmelamines (*e.g*., hexamethylmelamine and thiotepa); 3) alkyl sulfonates (*e.g*., busulfan); 4) nitrosoureas (*e.g*., carmustine (BCNU); lomustine (CCNU); semustine (methyl-CCNU); and streptozocin (streptozotocin)); and 5) triazenes (*e.g*., dacarbazine (DTIC; dimethyltriazenoimid-azolecarboxamide).

In some embodiments, antimetabolites suitable for use in the present compositions and methods include, but are not limited to: 1) folic acid analogs (*e.g*., methotrexate (amethopterin)); 2) pyrimidine analogs (*e.g*., fluorouracil (5-fluorouracil; 5-FU), floxuridine (fluorode-oxyuridine; FudR), and cytarabine (cytosine arabinoside)); and 3) purine analogs (*e.g*., mercaptopurine (6-mercaptopurine; 6-MP), thioguanine (6-thioguanine; TG), and pentostatin (2'-deoxycoformycin)).

In still further embodiments, chemotherapeutic agents suitable for use in the compositions and methods of the present invention include, but are not limited to: 1) vinca alkaloids (*e.g*., vinblastine (VLB), vincristine); 2) epipodophyllotoxins (*e.g*., etoposide and teniposide); 3) antibiotics (*e.g*., dactinomycin (actinomycin D), daunorubicin (daunomycin; rubidomycin), doxorubicin, bleomycin, plicamycin (mithramycin), and mitomycin (mitomycin C)); 4) enzymes (*e.g*., L-asparaginase); 5) biological response modifiers (*e.g*., interferon-alfa); 6) platinum coordinating complexes (*e.g*., cisplatin (cis-DDP) and carboplatin); 7) anthracenediones (*e.g*., mitoxantrone); 8) substituted ureas (*e.g.,* hydroxyurea); 9) methylhydrazine derivatives (*e.g*., procarbazine (N-methylhydrazine; MIH)); 10) adrenocortical suppressants (*e.g*., mitotane (o,p'-DDD) and aminoglutethimide); 11) adrenocorticosteroids (*e.g*., prednisone); 12) progestins (*e.g*., hydroxyprogesterone caproate, medroxyprogesterone acetate, and megestrol acetate); 13) estrogens (*e.g.,* diethylstilbestrol and ethinyl estradiol); 14) antiestrogens (*e.g*., tamoxifen); 15) androgens (*e.g*., testosterone propionate and fluoxymesterone); 16) antiandrogens (*e.g*., flutamide): and 17) gonadotropin-releasing hormone analogs (*e.g*., leuprolide).

Any oncolytic agent that is routinely used in a cancer therapy context finds use in the compositions and methods of the present invention. For example, the U.S. Food and Drug Administration maintains a formulary of oncolytic agents approved for use in the United States. International counterpart agencies to the U.S.F.D.A. maintain similar formularies. Table 6 provides a list of exemplary antineoplastic agents approved for use in the U.S. Those skilled in the art will appreciate that the "product labels" required on all U.S. approved chemotherapeutics describe approved indications, dosing information, toxicity data, and the like, for the exemplary agents.

**Table 6**

| | | |
|---|---|---|
| Aldesleukin (des-alanyl-1, serine-125 human interleukin-2) | Proleukin | Chiron Corp., Emeryville, CA |
| Alemtuzumab (IgG1κ anti CD52 antibody) | Campath | Millennium and ILEX Partners, LP, Cambridge, MA |
| Alitretinoin (9-cis-retinoic acid) | Panretin | Ligand Pharmaceuticals, Inc., San Diego CA |
| Allopurinol (1,5-dihydro-4 H -pyrazolo[3,4-d]pyrimidin-4-one monosodium salt) | Zyloprim | GlaxoSmithKline, Research Triangle Park, NC |
| Altretamine (N,N,N',N',N",N",- hexamethyl-1,3,5-triazine-2, 4, 6-triamine) | Hexalen | US Bioscience, West Conshohocken, PA |
| Amifostine (ethanethiol, 2-[(3-aminopropyl)amino]-, dihydrogen phosphate (ester)) | Ethyol | US Bioscience |
| Anastrozole (1,3-Benzenediacetonitrile, a, a, a', a'-tetramethyl-5-(1H-1,2,4-triazol-1-ylmethyl)) | Arimidex | AstraZeneca Pharmaceuticals, LP, Wilmington, DE |
| Arsenic trioxide | Trisenox | Cell Therapeutic, Inc., Seattle, WA |
| Asparaginase (L-asparagine amidohydrolase, type EC-2) | Elspar | Merck & Co., Inc., Whitehouse Station, NJ |
| BCG Live (lyophilized preparation of an attenuated strain of *Mycobacterium bovis* (*Bacillus Calmette-Gukin* [BCG], substrain Montreal) | TICE BCG | Organon Teknika, Corp., Durham, NC |
| bexarotene capsules (4-[1-(5,6,7,8-tetrahydro-3,5,5,8,8-pentamethyl-2-napthalenyl) ethenyl] benzoic acid) | Targretin | Ligand Pharmaceuticals |
| bexarotene gel | Targretin | Ligand Pharmaceuticals |
| Bleomycin (cytotoxic glycopeptide antibiotics produced by *Streptomyces verticillus*; bleomycin A₂ and bleomycin B₂) | Blenoxane | Bristol-Myers Squibb Co., NY, NY |
| Capecitabine (5'-deoxy-5-fluoro-N-[(pentyloxy)carbonyl]-cytidine) | Xeloda | Roche |
| Carboplatin (platinum, diammine [1,1-cyclobutanedicarboxylato(2-)-0, 0']-,(SP-4-2)) | Paraplatin | Bristol-Myers Squibb |
| Carmustine | BCNU, BiCNU | Bristol-Myers Squibb |
| (1,3 -bis(2-chloroethyl)-1 -nitrosourea) | | |
| Carmustine with Polifeprosan 20 Implant | Gliadel Wafer | Guilford Pharmaceuticals, Inc., Baltimore, MD |
| Celecoxib (as 4-[5-(4-methylphenyl)-3- (trifluoromethyl)-1H-pyrazol-1-yl] benzenesulfonamide) | Celebrex | Searle Pharmaceuticals, England |
| Chlorambucil (4-[bis(2chlorethyl)amino]benzenebutanoic acid) | Leukeran | GlaxoSmithKline |
| Cisplatin (PtCl₂H₆N₂) | Platinol | Bristol-Myers Squibb |
| Cladribine (2-chloro-2'-deoxy-b-D-adenosine) | Leustatin, 2-CdA | R.W. Johnson Pharmaceutical Research Institute, Raritan, NJ |
| Cyclophosphamide (2-[bis(2-chloroethyl)amino] tetrahydro-2H-13,2-oxazaphosphorine 2-oxide monohydrate) | Cytoxan, Neosar | Bristol-Myers Squibb |
| Cytarabine (1-b-D-Arabinofuranosylcytosine, C₉H₁₃N₃O₅) | Cytosar-U | Pharmacia & Upjohn Company |
| cytarabine liposomal | DepoCyt | Skye Pharmaceuticals, Inc., San Diego, CA |
| Dacarbazine (5-(3,3-dimethyl-1-triazeno)-imidazole-4-carboxamide (DTIC)) | DTIC-Dome | Bayer AG, Leverkusen, Germany |
| Dactinomycin, actinomycin D (actinomycin produced by *Streptomyces parvullus,* C₆₂H₈₆N₁₂O₁₆) | Cosmegen | Merck |
| Darbepoetin alfa (recombinant peptide) | Aranesp | Amgen, Inc., Thousand Oaks, CA |
| daunorubicin liposomal ((8S-cis)-8-acetyl-10-[(3-amino-2,3,6-trideoxy-a-L-lyxo-hexopyranosyl)oxy]-7,8,9,10-tetrahydro-6,8,11-trihydroxy-1-methoxy-5,12-naphthacenedione hydrochloride) | DanuoXome | Nexstar Pharmaceuticals, Inc., Boulder, CO |
| Daunorubicin HCl, daunomycin ((1 *S*,3 *S*)-3-Acetyl-1,2,3,4,6,11-hexahydro-3,5,12-trihydroxy-10-methoxy-6,11-dioxo-1-naphthacenyl 3-amino-2,3,6-trideoxy-(alpha)-L- *lyxo*-hexopyranoside hydrochloride) | Cerubidine | Wyeth Ayerst, Madison, NJ |
| Denileukin diftitox (recombinant peptide) | Ontak | Seragen, Inc., Hopkinton, MA |
| Dexrazoxane ((S)-4,4'-(1-methyl-1,2-ethanediyl)bis-2,6-piperazinedione) | Zinecard | Pharmacia & Upjohn Company |
| Docetaxel ((2R,3S)-N-carboxy-3-phenylisoserine, N-tert-butyl ester, 13-ester with 5b-20-epoxy-12a,4,7b,10b,13a-hexahydroxytax-11-en-9-one 4-acetate 2-benzoate, trihydrate) | Taxotere | Aventis Pharmaceuticals, Inc., Bridgewater, NJ |
| Doxorubicin HCl (8S,10S)-10-[(3-amino-2,3,6-trideoxy-a-L-lyxo-hexopyranosyl)oxy] -8-glycolyl-7,8,9,10-tetrahydro-6,8,11- trihydroxy-1-methoxy-5,12-naphthacenedione hydrochloride) | Adriamycin, Rubex | Pharmacia & Upjohn Company |
| doxorubicin | Adriamycin PFS Intravenous injection | Pharmacia & Upjohn Company |
| doxorubicin liposomal | Doxil | Sequus Pharmaceuticals, Inc., Menlo park, CA |
| dromostanolone propionate (17b-Hydroxy-2a-methyl-5a-androstan-3-one propionate) | Dromostanolone | Eli Lilly & Company, Indianapolis, IN |
| dromostanolone propionate | Masterone injection | Syntex, Corp., Palo Alto, CA |
| Elliott's B Solution | Elliott's B Solution | Orphan Medical, Inc |
| Epirubicin ((8S-cis)-10-[(3-amino-2,3,6-trideoxy-a-L-arabino- hexopyranosyl)oxy]-7,8,9,10-tetrahydro-6,8,11-trihydroxy-8-(hydroxyacetyl)-1-methoxy-5,12-naphthacenedione hydrochloride) | Ellence | Pharmacia & Upjohn Company |
| Epoetin alfa (recombinant peptide) | Epogen | Amgen, Inc |
| Estramustine (estra-1,3,5(10)-triene-3,17-diol(17(beta))-, 3-[bis(2-chloroethyl)carbamate] 17-(dihydrogen phosphate), disodium salt, monohydrate, or estradiol 3-[bis(2-chloroethyl)carbamate] 17-(dihydrogen phosphate), disodium salt, monohydrate) | Emcyt | Pharmacia & Upjohn Company |
| Etoposide phosphate (4'-Demethylepipodophyllotoxin 9-[4,6-O-(R)-ethylidene-(beta)-D-glucopyranoside], 4'-(dihydrogen phosphate)) | Etopophos | Bristol-Myers Squibb |
| etoposide, VP-16 | Vepesid | Bristol-Myers Squibb |
| (4'-demethylepipodophyllotoxin 9-[4,6-0-(R)-ethylidene-(beta)-D-glucopyranoside]) | | |
| Exemestane (6-methylenandrosta-1,4-diene-3, 17-dione) | Aromasin | Pharmacia & Upjohn Company |
| Filgrastim (r-metHuG-CSF) | Neupogen | Amgen, Inc |
| floxuridine (intraarterial) (2'-deoxy-5-fluorouridine) | FUDR | Roche |
| Fludarabine (fluorinated nucleotide analog of the antiviral agent vidarabine, 9-b -D-arabinofuranosyladenine (ara-A)) | Fludara | Berlex Laboratories, Inc., Cedar Knolls, NJ |
| Fluorouracil, 5-FU (5-fluoro-2,4(1H,3H)-pyrimidinedione) | Adrucil | ICN Pharmaceuticals, Inc., Humacao, Puerto Rico |
| Fulvestrant (7-alpha-[9-(4,4,5,5,5-penta fluoropentylsulphinyl) nonyl]estra-1,3,5-(10)-triene-3,17-beta-diol) | Faslodex | IPR Pharmaceuticals, Guayama, Puerto Rico |
| Gemcitabine (2'-deoxy-2', 2'-difluorocytidine monohydrochloride (b-isomer)) | Gemzar | Eli Lilly |
| Gemtuzumab Ozogamicin (anti-CD33 hP67.6) | Mylotarg | Wyeth Ayerst |
| Goserelin acetate | Zoladex Implant | AstraZeneca Pharmaceuticals |
| Hydroxyurea | Hydrea | Bristol-Myers Squibb |
| Ibritumomab Tiuxetan (immunoconjugate resulting from a thiourea covalent bond between the monoclonal antibody Ibritumomab and the linker-chelator tiuxetan [N-[2-bis(carboxymethyl)amino]-3-(p-isothiocyanatophenyl)- propyl]-[N-[2-bis(carboxymethyl)amino]-2-(methyl) - ethyl]glycine) | Zevalin | Biogen IDEC, Inc., Cambridge MA |
| Idarubicin (5, 12-Naphthacenedione, 9-acetyl-7-[(3-amino-2,3,6-trideoxy-(alpha)-L- lyxo - hexopyranosyl)oxy]-7,8,9,10-tetrahydro-6,9,11-trihydroxyhydrochloride, (7S- cis )) | Idamycin | Pharmacia & Upjohn Company |
| Ifosfamide (3-(2-chloroethyl)-2-[(2-chloroethyl)amino]tetrahydro-2H-1,3,2-oxazaphosphorine 2-oxide) | IFEX | Bristol-Myers Squibb |
| Imatinib Mesilate | Gleevec | Novartis AG, Basel, |
| (4-[(4-Methyl-1-piperazinyl)methyl]-N-[4-methyl-3-[[4-(3-pyridinyl)-2-pyrimidinyl]amino]-phenyl]benzamide methanesulfonate) | | Switzerland |
| Interferon alfa-2a (recombinant peptide) | Roferon-A | Hoffmann-La Roche, Inc., Nutley, NJ |
| Interferon alfa-2b (recombinant peptide) | Intron A (Lyophilized Betaseron) | Schering AG, Berlin, Germany |
| Irinotecan HCl ((4S)-4,1 1-diethyl-4-hydroxy-9-[(4- piperidinopiperidino)carbonyloxy]-1H-pyrano[3', 4': 6,7] indolizino[1,2-b] quinoline-3,14(4H, 12H) dione hydrochloride trihydrate) | Camptosar | Pharmacia & Upjohn Company |
| Letrozole (4,4'-(1H-1,2,4 -Triazol-1-ylmethylene) dibenzonitrile) | Femara | Novartis |
| Leucovorin (L-Glutamic acid, N[4[[(2amino-5-formyl-1,4,5,6,7,8 hexahydro4oxo6-pteridinyl)methyl]amino]benzoyl], calcium salt (1:1)) | Wellcovorin, Leucovorin | Immunex, Corp., Seattle, WA |
| Levamisole HCl ((-)-(S)-2,3,5, 6-tetrahydro-6-phenylimidazo [2,1-b]thiazole monohydrochloride C₁₁H₁₂N₂S·HCl) | Ergamisol | Janssen Research Foundation, Titusville, NJ |
| Lomustine (1 -(2-chloro-ethyl)-3 -cyclohexyl -1 -nitrosourea) | CeeNU | Bristol-Myers Squibb |
| Meclorethamine, nitrogen mustard (2-chloro-N-(2-chloroethyl)-N-methylethanamine hydrochloride) | Mustargen | Merck |
| Megestrol acetate 17α(acetyloxy)- 6- methylpregna- 4,6- diene-3,20- dione | Megace | Bristol-Myers Squibb |
| Melphalan, L-PAM (4-[bis(2-chloroethyl) amino]-L-phenylalanine) | Alkeran | GlaxoSmithKline |
| Mercaptopurine, 6-MP (1,7-dihydro-6 H -purine-6-thione monohydrate) | Purinethol | GlaxoSmithKline |
| Mesna (sodium 2-mercaptoethane sulfonate) | Mesnex | Asta Medica |
| Methotrexate (N-[4-[[(2,4-diamino-6-pteridinyl)methyl]methylamino]benzoyl]-L- | Methotrexate | Lederle Laboratories |
| glutamic acid) | | |
| Methoxsalen (9-methoxy-7H-furo[3,2-g][1]-benzopyran-7-one) | Uvadex | Therakos, Inc., Way Exton, Pa |
| Mitomycin C | Mutamycin | Bristol-Myers Squibb |
| mitomycin C | Mitozytrex | SuperGen, Inc., Dublin, CA |
| Mitotane (1,1-dichloro-2-(o-chlorophenyl)-2-(p-chlorophenyl) ethane) | Lysodren | Bristol-Myers Squibb |
| Mitoxantrone (1,4-dihydroxy-5,8-bis[[2- [(2-hydroxyethyl)amino]ethyl]amino]-9,10-anthracenedione dihydrochloride) | Novantrone | Immunex Corporation |
| Nandrolone phenpropionate | Durabolin-50 | Organon, Inc., West Orange, NJ |
| Nofetumomab | Verluma | Boehringer Ingelheim Pharma KG, Germany |
| Oprelvekin (IL-11) | Neumega | Genetics Institute, Inc., Alexandria, VA |
| Oxaliplatin (cis-[(1R,2R)-1,2-cyclohexanediamine-N,N'] [oxalato(2-)-O,O'] platinum) | Eloxatin | Sanofi Synthelabo, Inc., NY, NY |
| Paclitaxel (5β, 20-Epoxy-1,2a, 4,7β, 10β, 13a-hexahydroxytax-11-en-9-one 4,10-diacetate 2-benzoate 13-ester with (2R, 3 S)- N-benzoyl-3-phenylisoserine) | TAXOL | Bristol-Myers Squibb |
| Pamidronate (phosphonic acid (3-amino-1-hydroxypropylidene) bis-, disodium salt, pentahydrate, (APD)) | Aredia | Novartis |
| Pegademase ((monomethoxypolyethylene glycol succinimidyl) 11 - 17 -adenosine deaminase) | Adagen (Pegademase Bovine) | Enzon Pharmaceuticals, Inc., Bridgewater, NJ |
| Pegaspargase (monomethoxypolyethylene glycol succinimidyl L-asparaginase) | Oncaspar | Enzon |
| Pegfilgrastim (covalent conjugate of recombinant methionyl human G-CSF (Filgrastim) and monomethoxypolyethylene glycol) | Neulasta | Amgen, Inc |
| Pentostatin | Nipent | Parke-Davis Pharmaceutical Co., |
| | | Rockville, MD |
| Pipobroman | Vercyte | Abbott Laboratories, Abbott Park, IL |
| Plicamycin, Mithramycin (antibiotic produced by *Streptomyces plicatus*) | Mithracin | Pfizer, Inc., NY, NY |
| Porfimer sodium | Photofrin | QLT Phototherapeutics, Inc., Vancouver, Canada |
| Procarbazine (N-isopropyl-µ-(2-methylhydrazino)-p-toluamide monohydrochloride) | Matulane | Sigma Tau Pharmaceuticals, Inc., Gaithersburg, MD |
| Quinacrine (6-chloro-9-(1 -methyl-4-diethyl-amine) butylamino-2-methoxyacridine) | Atabrine | Abbott Labs |
| Rasburicase (recombinant peptide) | Elitek | Sanofi-Synthelabo, Inc., |
| Rituximab (recombinant anti-CD20 antibody) | Rituxan | Genentech, Inc., South San Francisco, CA |
| Sargramostim (recombinant peptide) | Prokine | Immunex Corp |
| Streptozocin (streptozocin 2 -deoxy - 2 - [[(methylnitrosoamino)carbonyl]amino] - a(and b) - D - glucopyranose and 220 mg citric acid anhydrous) | Zanosar | Pharmacia & Upjohn Company |
| Talc (Mg₃Si₄O₁₀ (OH)₂) | Sclerosol | Bryan, Corp., Woburn, MA |
| Tamoxifen ((Z)2-[4-(1,2-diphenyl-1-butenyl) phenoxy]-N, N-dimethylethanamine 2-hydroxy-1,2,3-propanetricarboxylate (1:1)) | Nolvadex | AstraZeneca Pharmaceuticals |
| Temozolomide (3,4-dihydro-3-methyl-4-oxoimidazo[5,1-d]-as-tetrazine-8-carboxamide) | Temodar | Schering |
| teniposide, VM-26 (4'-demethylepipodophyllotoxin 9-[4,6-0-(R)-2- thenylidene-(beta)-D-glucopyranoside]) | Vumon | Bristol-Myers Squibb |
| Testolactone (13 -hydroxy-3 -oxo-13,17-secoandrosta-1,4-dien-17-oic acid [dgr ]-lactone) | Teslac | Bristol-Myers Squibb |
| Thioguanine, 6-TG (2-amino-1,7-dihydro-6 H - purine-6-thione) | Thioguanine | GlaxoSmithKline |
| Thiotepa (Aziridine, 1,1',1"-phosphinothioylidynetris-, or Tris (1-aziridinyl) phosphine sulfide) | Thioplex | Immunex Corporation |
| Topotecan HCl ((S)-10-[(dimethylamino) methyl]-4-ethyl-4,9-dihydroxy-1H-pyrano[3', 4': 6,7] indolizino [1,2-b] quinoline-3, 14-(4H, 12H)-dione monohydrochloride) | Hycamtin | GlaxoSmithKline |
| Toremifene (2-(p-[(Z)-4-chloro-1,2-diphenyl-1 -butenyl]-phenoxy)-N,N-dimethylethylamine citrate (1:1)) | Fareston | Roberts Pharmaceutical Corp., Eatontown, NJ |
| Tositumomab, 1131 Tositumomab (recombinant murine immunotherapeutic monoclonal IgG₂ₐ lambda anti-CD20 antibody (I 131 is a radioimmunotherapeutic antibody)) | Bexxar | Corixa Corp., Seattle, WA |
| Trastuzumab (recombinant monoclonal IgG₁ kappa anti-HER2 antibody) | Herceptin | Genentech, Inc |
| Tretinoin, ATRA (all-trans retinoic acid) | Vesanoid | Roche |
| Uracil Mustard | Uracil Mustard Capsules | Roberts Labs |
| Valrubicin, N-trifluoroacetyladriamycin-14-valerate ((2S-cis)-2- [1,2,3,4,6,11-hexahydro-2,5,12-trihydroxy-7 methoxy-6,11-dioxo-[[4 2,3,6-trideoxy-3- [(trifluoroacetyl)-amino-α-L-lyxo-hexopyranosyl]oxyl]-2-naphthacenyl]-2-oxoethyl pentanoate) | Valstar | Anthra --> Medeva |
| Vinblastine, Leurocristine (C₄₆H₅₆N₄O₁₀•H₂SO₄) | Velban | Eli Lilly |
| Vincristine (C₄₆H₅₆N₄O₁₀•H₂SO₄) | Oncovin | Eli Lilly |
| Vinorelbine (3' ,4'-didehydro-4'-deoxy-C'-norvincaleukoblastine [R-(R*,R*)-2,3-dihydroxybutanedioate (1:2)(salt)]) | Navelbine | GlaxoSmithKline |
| Zoledronate, Zoledronic acid ((1 -Hydroxy-2-imidazol-1-yl-phosphonoethyl) phosphonic acid monohydrate) | Zometa | Novartis |

Anticancer agents further include compounds which have been identified to have anticancer activity. Examples include, but are not limited to, 3-AP, 12-0-tetradecanoylphorbol-13-acetate, 17AAG, 852A, ABI-007, ABR-217620, ABT-751, ADI-PEG 20, AE-941, AG-013736, AGRO100, alanosine, AMG 706, antibody G250, antineoplastons, AP23573, apaziquone, APC8015, atiprimod, ATN-161, atrasenten, azacitidine, BB-10901, BCX-1777, bevacizumab, BG00001, bicalutamide, BMS 247550, bortezomib, bryostatin-1, buserelin, calcitriol, CCI-779, CDB-2914, cefixime, cetuximab, CG0070, cilengitide, clofarabine, combretastatin A4 phosphate, CP-675,206, CP-724,714, CpG 7909, curcumin, decitabine, DENSPM, doxercalciferol, E7070, E7389, ecteinascidin 743, efaproxiral, eflomithine, EKB-569, enzastaurin, erlotinib, exisulind, fenretinide, flavopiridol, fludarabine, flutamide, fotemustine, FR901228, G17DT, galiximab, gefitinib, genistein, glufosfamide, GTI-2040, histrelin, HKI-272, homoharringtonine, HSPPC-96, hu14.18-interleukin-2 fusion protein, HuMax-CD4, iloprost, imiquimod, infliximab, interleukin-12, IPI-504, irofulven, ixabepilone, lapatinib, lenalidomide, lestaurtinib, leuprolide, LMB-9 immunotoxin, lonafarnib, luniliximab, mafosfamide, MB07133, MDX-010, MLN2704, monoclonal antibody 3F8, monoclonal antibody J591, motexafin, MS-275, MVA-MUC1-IL2, nilutamide, nitrocamptothecin, nolatrexed dihydrochloride, nolvadex, NS-9, O6-benzylguanine, oblimersen sodium, ONYX-015, oregovomab, OSI-774, panitumumab, paraplatin, PD-0325901, pemetrexed, PHY906, pioglitazone, pirfenidone, pixantrone, PS-341, PSC 833, PXD101, pyrazoloacridine, R115777, RAD001, ranpirnase, rebeccamycin analogue, rhuAngiostatin protein, rhuMab 2C4, rosiglitazone, rubitecan, S-1, S-8184, satraplatin, SB-, 15992, SGN-0010, SGN-40, sorafenib, SR31747A, ST1571, SU011248, suberoylanilide hydroxamic acid, suramin, talabostat, talampanel, tariquidar, temsirolimus, TGFa-PE38 immunotoxin, thalidomide, thymalfasin, tipifarnib, tirapazamine, TLK286, trabectedin, trimetrexate glucuronate, TroVax, UCN-1, valproic acid, vinflunine, VNP40101M, volociximab, vorinostat, VX-680, ZD1839, ZD6474, zileuton, and zosuquidar trihydrochloride.

For a more detailed description of anticancer agents and other therapeutic agents, those skilled in the art are referred to any number of instructive manuals including, but not limited to, the Physician's Desk Reference and to Goodman and Gilman's "Pharmaceutical Basis of Therapeutics" tenth edition, Eds. Hardman et al., 2002.

The present invention provides a compound of the invention for use with radiation therapy. The invention is not limited by the types, amounts, or delivery and administration systems used to deliver the therapeutic dose of radiation to an animal. For example, the animal may receive photon radiotherapy, particle beam radiation therapy, other types of radiotherapies, and combinations thereof. In some embodiments, the radiation is delivered to the animal using a linear accelerator. In still other embodiments, the radiation is delivered using a gamma knife.

The source of radiation can be external or internal to the animal. External radiation therapy is most common and involves directing a beam of high-energy radiation to a tumor site through the skin using, for instance, a linear accelerator. While the beam of radiation is localized to the tumor site, it is nearly impossible to avoid exposure of normal, healthy tissue. However, external radiation is usually well tolerated by animals. Internal radiation therapy involves implanting a radiation-emitting source, such as beads, wires, pellets, capsules, particles, and the like, inside the body at or near the tumor site including the use of delivery systems that specifically target cancer cells (*e.g*., using particles attached to cancer cell binding ligands). Such implants can be removed following treatment, or left in the body inactive. Types of internal radiation therapy include, but are not limited to, brachytherapy, interstitial irradiation, intracavity irradiation, radioimmunotherapy, and the like.

The animal may optionally receive radiosensitizers (*e.g*., metronidazole, misonidazole, intra-arterial Budr, intravenous iododeoxyuridine (IudR), nitroimidazole, 5-substituted-4-nitroimidazoles, 2H-isoindolediones, [[(2-bromoethyl)-amino]methyl]-nitro-1H-imidazole-1-ethanol, nitroaniline derivatives, DNA-affinic hypoxia selective cytotoxins, halogenated DNA ligand, 1,2,4 benzotriazine oxides, 2-nitroimidazole derivatives, fluorine-containing nitroazole derivatives, benzamide, nicotinamide, acridineintercalator, 5-thiotretrazole derivative, 3-nitro-1,2,4-triazole, 4,5-dinitroimidazole derivative, hydroxylated texaphrins, cisplatin, mitomycin, tiripazamine, nitrosourea, mercaptopurine, methotrexate, fluorouracil, bleomycin, vincristine, carboplatin, epirubicin, doxorubicin, cyclophosphamide, vindesine, etoposide, paclitaxel, heat (hyperthermia), and the like), radioprotectors (*e.g*., cysteamine, aminoalkyl dihydrogen phosphorothioates, amifostine (WR 2721), IL-1, IL-6, and the like). Radiosensitizers enhance the killing of tumor cells. Radioprotectors protect healthy tissue from the harmful effects of radiation.

Any type of radiation can be administered to an animal, so long as the dose of radiation is tolerated by the animal without unacceptable negative side-effects. Suitable types of radiotherapy include, for example, ionizing (electromagnetic) radiotherapy (*e.g*., X-rays or gamma rays) or particle beam radiation therapy (*e.g*., high linear energy radiation). Ionizing radiation is defined as radiation comprising particles or photons that have sufficient energy to produce ionization, *i.e*., gain or loss of electrons (as described in, for example, U.S. 5,770,581). The effects of radiation can be at least partially controlled by the clinician. In one embodiment, the dose of radiation is fractionated for maximal target cell exposure and reduced toxicity.

In one embodiment, the total dose of radiation administered to an animal is about .01 Gray (Gy) to about 100 Gy. In another embodiment, about 10 Gy to about 65 Gy (*e.g*., about 15 Gy, 20 Gy, 25 Gy, 30 Gy, 35 Gy, 40 Gy, 45 Gy, 50 Gy, 55 Gy, or 60 Gy) are administered over the course of treatment. While in some embodiments a complete dose of radiation can be administered over the course of one day, the total dose is ideally fractionated and administered over several days. Desirably, radiotherapy is administered over the course of at least about 3 days, *e.g*., at least 5, 7, 10, 14, 17, 21, 25, 28, 32, 35, 38, 42, 46, 52, or 56 days (about 1-8 weeks). Accordingly, a daily dose of radiation will comprise approximately 1-5 Gy (*e.g*., about 1 Gy, 1.5 Gy, 1.8 Gy, 2 Gy, 2.5 Gy, 2.8 Gy, 3 Gy, 3.2 Gy, 3.5 Gy, 3.8 Gy, 4 Gy, 4.2 Gy, or 4.5 Gy), or 1-2 Gy (*e.g*., 1.5-2 Gy). The daily dose of radiation should be sufficient to induce destruction of the targeted cells. If stretched over a period, in one embodiment, radiation is not administered every day, thereby allowing the animal to rest and the effects of the therapy to be realized. For example, radiation desirably is administered on 5 consecutive days, and not administered on 2 days, for each week of treatment, thereby allowing 2 days of rest per week. However, radiation can be administered 1 day/week, 2 days/week, 3 days/week, 4 days/week, 5 days/week, 6 days/week, or all 7 days/week, depending on the animal's responsiveness and any potential side effects. Radiation therapy can be initiated at any time in the therapeutic period. In one embodiment, radiation is initiated in week 1 or week 2, and is administered for the remaining duration of the therapeutic period. For example, radiation is administered in weeks 1-6 or in weeks 2-6 of a therapeutic period comprising 6 weeks for treating, for instance, a solid tumor. Alternatively, radiation is administered in weeks 1-5 or weeks 2-5 of a therapeutic period comprising 5 weeks. These exemplary radiotherapy administration schedules are not intended, however, to limit the present invention.

Antimicrobial therapeutic agents may also be used as therapeutic agents in the present invention. Any agent that can kill, inhibit, or otherwise attenuate the function of microbial organisms may be used, as well as any agent contemplated to have such activities. Antimicrobial agents include, but are not limited to, natural and synthetic antibiotics, antibodies, inhibitory proteins (*e.g*., defensins), antisense nucleic acids, membrane disruptive agents and the like, used alone or in combination. Indeed, any type of antibiotic may be used including, but not limited to, antibacterial agents, antiviral agents, antifungal agents, and the like.

In some embodiments of the present invention, a compound of the invention and one or more therapeutic agents or anticancer agents are for use in cancer therapy administered to an animal under one or more of the following conditions: at different periodicities, at different durations, at different concentrations, by different administration routes, *etc.* In some embodiments, the compound is administered prior to the therapeutic or anticancer agent, *e.g.,* 0.5, 1, 2, 3, 4, 5, 10, 12, or 18 hours, 1, 2, 3, 4, 5, or 6 days, or 1, 2, 3, or 4 weeks prior to the administration of the therapeutic or anticancer agent. In some embodiments, the compound is administered after the therapeutic or anticancer agent, *e.g.,* 0.5, 1, 2, 3, 4, 5, 10, 12, or 18 hours, 1, 2, 3, 4, 5, or 6 days, or 1, 2, 3, or 4 weeks after the administration of the anticancer agent. In some embodiments, the compound and the therapeutic or anticancer agent are administered concurrently but on different schedules, *e.g.,* the compound is administered daily while the therapeutic or anticancer agent is administered once a week, once every two weeks, once every three weeks, or once every four weeks. In other embodiments, the compound is administered once a week while the therapeutic or anticancer agent is administered daily, once a week, once every two weeks, once every three weeks, or once every four weeks.

Compositions within the scope of this invention include all compositions wherein the compounds of the present invention are contained in an amount which is effective to achieve its intended purpose. While individual needs vary, determination of optimal ranges of effective amounts of each component is within the skill of the art. Typically, the compounds may be administered to mammals, *e.g*. humans, orally at a dose of 0.0025 to 50 mg/kg, or an equivalent amount of the pharmaceutically acceptable salt thereof, per day of the body weight of the mammal being treated for disorders responsive to induction of apoptosis. In one embodiment, about 0.01 to about 25 mg/kg is orally administered to treat, ameliorate, or prevent such disorders. For intramuscular injection, the dose is generally about one-half of the oral dose. For example, a suitable intramuscular dose would be about 0.0025 to about 25 mg/kg, or from about 0.01 to about 5 mg/kg.

The unit oral dose may comprise from about 0.01 to about 1000 mg, for example, about 0.1 to about 100 mg of the compound. The unit dose may be administered one or more times daily as one or more tablets or capsules each containing from about 0.1 to about 10 mg, conveniently about 0.25 to 50 mg of the compound or its solvates.

In a topical formulation, the compound may be present at a concentration of about 0.01 to 100 mg per gram of carrier. In a one embodiment, the compound is present at a concentration of about 0.07-1.0 mg/ml, for example, about 0.1-0.5 mg/ml, and in one embodiment, about 0.4 mg/ml.

In addition to administering the compound as a raw chemical, the compounds of the invention may be administered as part of a pharmaceutical preparation containing suitable pharmaceutically acceptable carriers comprising excipients and auxiliaries which facilitate processing of the compounds into preparations which can be used pharmaceutically. The preparations, particularly those preparations which can be administered orally or topically and which can be used for one type of administration, such as tablets, dragees, slow release lozenges and capsules, mouth rinses and mouth washes, gels, liquid suspensions, hair rinses, hair gels, shampoos and also preparations which can be administered rectally, such as suppositories, as well as suitable solutions for administration by intravenous infusion, injection, topically or orally, contain from about 0.01 to 99 percent, in one embodiment from about 0.25 to 75 percent of active compound(s), together with the excipient.

The pharmaceutical compositions of the invention are for use in cancer therapy administered to any patient which may experience the beneficial effects of the compounds of the invention. Foremost among such patients are mammals, *e.g*., humans, although the invention is not intended to be so limited. Other patients include veterinary animals (cows, sheep, pigs, horses, dogs, cats and the like).

The compounds and pharmaceutical compositions thereof may be administered by any means that achieve their intended purpose. For example, administration may be by parenteral, subcutaneous, intravenous, intramuscular, intraperitoneal, transdermal, buccal, intrathecal, intracranial, intranasal or topical routes. Alternatively, or concurrently, administration may be by the oral route. The dosage administered will be dependent upon the age, health, and weight of the recipient, kind of concurrent treatment, if any, frequency of treatment, and the nature of the effect desired.

The pharmaceutical preparations of the present invention are manufactured in a manner which is itself known, for example, by means of conventional mixing, granulating, dragee-making, dissolving, or lyophilizing processes. Thus, pharmaceutical preparations for oral use can be obtained by combining the active compounds with solid excipients, optionally grinding the resulting mixture and processing the mixture of granules, after adding suitable auxiliaries, if desired or necessary, to obtain tablets or dragee cores.

Suitable excipients are, in particular, fillers such as saccharides, for example lactose or sucrose, mannitol or sorbitol, cellulose preparations and/or calcium phosphates, for example tricalcium phosphate or calcium hydrogen phosphate, as well as binders such as starch paste, using, for example, maize starch, wheat starch, rice starch, potato starch, gelatin, tragacanth, methyl cellulose, hydroxypropylmethylcellulose, sodium carboxymethylcellulose, and/or polyvinyl pyrrolidone. If desired, disintegrating agents may be added such as the above-mentioned starches and also carboxymethyl-starch, crosslinked polyvinyl pyrrolidone, agar, or alginic acid or a salt thereof, such as sodium alginate. Auxiliaries are, above all, flow-regulating agents and lubricants, for example, silica, talc, stearic acid or salts thereof, such as magnesium stearate or calcium stearate, and/or polyethylene glycol. Dragee cores are provided with suitable coatings which, if desired, are resistant to gastric juices. For this purpose, concentrated saccharide solutions may be used, which may optionally contain gum arabic, talc, polyvinyl pyrrolidone, polyethylene glycol and/or titanium dioxide, lacquer solutions and suitable organic solvents or solvent mixtures. In order to produce coatings resistant to gastric juices, solutions of suitable cellulose preparations such as acetylcellulose phthalate or hydroxypropylmethylcellulose phthalate, are used. Dye stuffs or pigments may be added to the tablets or dragee coatings, for example, for identification or in order to characterize combinations of active compound doses.

Other pharmaceutical preparations which can be used orally include push-fit capsules made of gelatin, as well as soft, sealed capsules made of gelatin and a plasticizer such as glycerol or sorbitol. The push-fit capsules can contain the active compounds in the form of granules which may be mixed with fillers such as lactose, binders such as starches, and/or lubricants such as talc or magnesium stearate and, optionally, stabilizers. In soft capsules, the active compounds are in one embodiment dissolved or suspended in suitable liquids, such as fatty oils, or liquid paraffin. In addition, stabilizers may be added.

Possible pharmaceutical preparations which can be used rectally include, for example, suppositories, which consist of a combination of one or more of the active compounds with a suppository base. Suitable suppository bases are, for example, natural or synthetic triglycerides, or paraffin hydrocarbons. In addition, it is also possible to use gelatin rectal capsules which consist of a combination of the active compounds with a base. Possible base materials include, for example, liquid triglycerides, polyethylene glycols, or paraffin hydrocarbons.

Suitable formulations for parenteral administration include aqueous solutions of the active compounds in water-soluble form, for example, water-soluble salts and alkaline solutions. In addition, suspensions of the active compounds as appropriate oily injection suspensions may be administered. Suitable lipophilic solvents or vehicles include fatty oils, for example, sesame oil, or synthetic fatty acid esters, for example, ethyl oleate or triglycerides or polyethylene glycol-400. Aqueous injection suspensions may contain substances which increase the viscosity of the suspension include, for example, sodium carboxymethyl cellulose, sorbitol, and/or dextran. Optionally, the suspension may also contain stabilizers.

The topical compositions of this invention are formulated in one embodiment as oils, creams, lotions, ointments and the like by choice of appropriate carriers. Suitable carriers include vegetable or mineral oils, white petrolatum (white soft paraffin), branched chain fats or oils, animal fats and high molecular weight alcohol (greater than C₁₂). The carriers may be those in which the active ingredient is soluble. Emulsifiers, stabilizers, humectants and antioxidants may also be included as well as agents imparting color or fragrance, if desired. Additionally, transdermal penetration enhancers can be employed in these topical formulations. Examples of such enhancers can be found in U.S. Pat. Nos. 3,989,816 and 4,444,762.

Ointments may be formulated by mixing a solution of the active ingredient in a vegetable oil such as almond oil with warm soft paraffin and allowing the mixture to cool. A typical example of such an ointment is one which includes about 30% almond oil and about 70% white soft paraffin by weight. Lotions may be conveniently prepared by dissolving the active ingredient, in a suitable high molecular weight alcohol such as propylene glycol or polyethylene glycol.

One of ordinary skill in the art will readily recognize that the foregoing represents merely a detailed description of certain preferred embodiments of the present invention. Various modifications and alterations of the compositions and methods described above can readily be achieved using expertise available in the art and are within the scope of the invention.

### EXAMPLES

The following examples are illustrative, but not limiting, of the compounds, compositions, and methods of the present invention. Other suitable modifications and adaptations of the variety of conditions and parameters normally encountered in clinical therapy are obvious to those skilled in the art.

### Example I.

This example describes various synthetic methtods for obtaining the compounds of the present invention.

A method of preparing bicyclic quinone derivatives of formula I (illustrative of compounds of formula XIV) is shown in the scheme 1, and scheme 2. 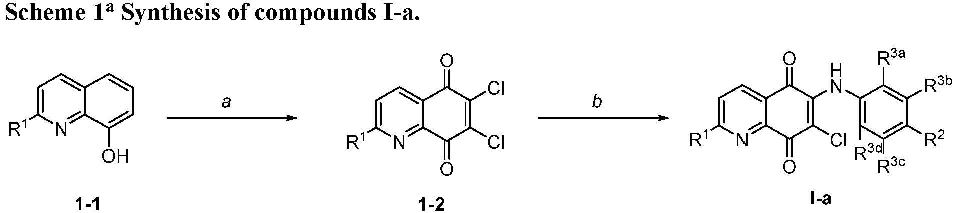 ^{a} Reagents and conditions: (a) NaClO₃, con. HCl, room temperature-50 °C, 2 h; (b) Arylamine, CeCl₃·7H₂O, EtOH, 60-90 °C, 2-6 h;
wherein R¹, R², R^{3a}, R^{3b}, R^{3c}, and R^{3d} are the same as defined above.

The detailed synthetic methods in scheme 1 are described below.

The preparation of the compound of formula **I-a** is as follow (Scheme 1): Chloroxidation of 8-hydroxyquinoline derivatives **1-1** with sodium chlorate in the presence of concentrated hydrochloric acid under simple magnetic stirring afforded the 6,7-dichloro-5,8-quinolinediones **1-2.** The compound of formula I is prepared by condensation reaction of 6,7-dichloro-5,8-quinolinediones **1-2** and arylamines. A solvent used in this reaction is C₁-C₃ lower alcohol such as methanol, ethanol and isopropanol. Additive used in this regioselective condensation of appropriate aminobenzenes to 6 position of 6,7-dichloroquinoline-5,8-diones **1-2** in the presence of Lewis acid such as cerium(III) chloride heptahydrate (CeCl₇·7H₂O), nickel(II) chloride hexahydrate (NiCl₂·6H₂O), and iron(III) chloride hexahydrate FeCl₃·6H₂O. The reaction is carried at reflux temperature for 2-6 hours. 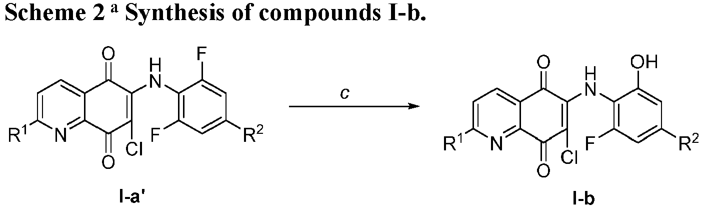 ^{a} Reagents and conditions: (c) KOH, MeOH, H₂O, 80-100 °C, 2-5 hours.
wherein R¹, and R² are the same as defined above.

The detailed synthetic methods in scheme 2 are described below.

The compound of formula **1-b** is prepared by reacting compound **1-a'** dissolved in methanol and water with potassium hydroxide at 80 °C for 2 hours (Scheme 2).

A method of preparing tetracyclic quinone derivatives of formula III **(Illustrative of compounds of Formula XV)** is shown in scheme 4. The detailed synthetic methods are described below. 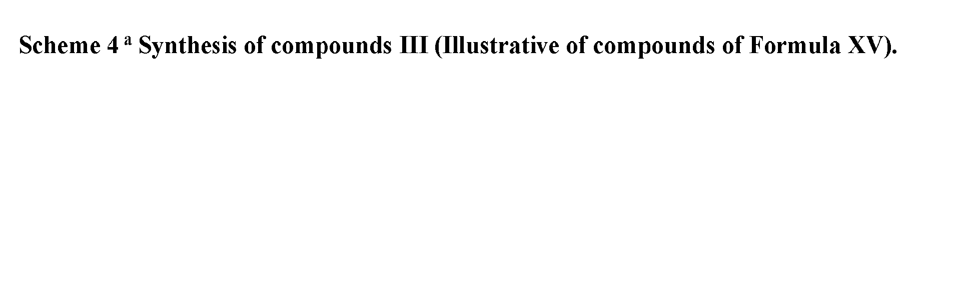 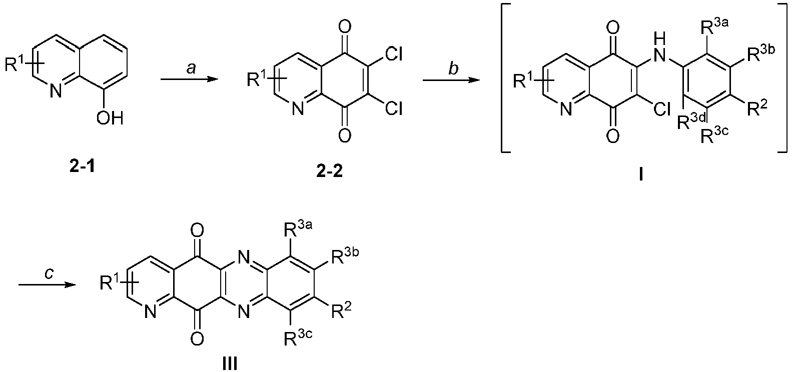 ^{a} Reagents and conditions, (a) NaClO₃, con. HCl, room temperature-50 °C, 2 h; (b) Arylamine, CeCl₃ ·7H₂O, EtOH, 60-80 °C, 2-5 h; (c) NaN₃, H₂O, DMF, 80-95 °C, 2-8 h.
wherein R¹, R², R^{3a}, R^{3b}, R^{3c}, and R^{3d} are the same as defined above.

The detailed synthesis of compounds in scheme 4 is described hereunder. The preparation of the compound of formula **III** is as follow: Chloroxidation of 8-hydroxyquinoline derivatives **2-1** with sodium chlorate in the presence of concentrated hydrochloric acid under simple magnetic stirring afforded the 6,7-dichloro-5,8-quinolinediones **2-2.** Condensation reaction of 6,7-dichloro-5,8-quinolinediones **I** with arylamines in the presence of Lewis acid, such as cerium(III) chloride heptahydrate (CeCl₇·7H₂O), nickel(II) chloride hexahydrate (NiCl₂·6H₂O), or iron(III) chloride hexahydrate FeCl₃·6H₂O. The solvent used in this reaction is C₁-C₃ lower alcohol such as methanol, ethanol and isopropanol at 60- 90 °C for 2-6 hours. After the reaction is completed, the solvent was removed under reduced pressure. Then the sodium azide, DMF and a drop of water were added. The mixture was stirred at 65-90 °C for 2-6 hours to obtain the compounds of formula **III (Illustrative of compounds of Formula XV).**

### General Experimental Methods

General Methods. Reagents and anhydrous solvents were used without further purification and purchased from commercial sources. Reaction progress was monitored by UV absorbance using thin-layer chromatography (TLC) on aluminum-backed precoated silica plates from Silicycle (SiliaPlate, 200 µm thickness, F254). Purifications using flash chromatography were performed using Silicycle silica gel (SiliaFlash F60, 40-63 µm, 230-400 mesh, PN R10030B), and a small percentage of compounds were purified using a Biotage Isolera chromatography system equipped with 10 and 25 g Ultra-SNAP Cartridge columns (25 µM spherical silica). 1H NMR spectra were obtained using a Varian (300 or 400 MHz) instrument. Spectral data are reported using the following abbreviations: s = singlet, d = doublet, t = triplet, q = quartet, m = multiplet, dd = doublet of doublets, and coupling constants are reported in Hz, followed by integration. A Shimadzu LCMS 20-20 system was utilized for generating HPLC traces, obtaining mass spectrometry data, and evaluating purity. The system is equipped with a PDA UV detector and Kinetex 2.6 µm, XB-C18 100 Å, 75 mm × 4.6 mm column, which was used at room temperature. HPLC gradient method utilized a 1% to 90% MeCN in H2O with 0.01% formic acid over 20 min with a 0.50 mL/min flow rate. Purity of final compounds (≥95%) was assessed at 254 nm using the described column and method. Reverse-phase preparatory purifications were performed on a Shimadzu LC-20 modular HPLC system. This system utilized a PDA detector and a Kinetex 5 µm XB-C18 100 Å, 150 mm × 21.2 mm column. Purification methods used a 27 min gradient from 10% to 90% MeCN in H2O with 0.02% trifluoroacetic acid.
**General Protocol A:** Chloroxidation. Sodium chlorate (5.3 g, 50 mmol) was added over a period of 1 h to a stirred solution of 8-hydroxyquinoline derivatives (10 mmol) in concentrated HCl (100 mL) at 50 °C. The reaction mixture then was allowed to stir for 2 h and there after diluted to 200 mL with distilled water. The yellow precipitate that formed was removed by filtration and discarded. The filtrate was extracted with CH₂Cl₂ (3 × 50 mL), and the organic phases were combined, washed with water and brine, dried (Na₂SO₄), and concentrated in vacuo. The solid was recrystallized in MeOH to afford pure bright yellow crystals of 6,7-dichloro-5,8-quinolinediones **1-2** and **2-2.**
**General Protocol B:** Regioselective condensation. A solution of 6,7-dichloro-5,8-quinolinediones **1-2 or 2-2** (1.0 equiv.), cerium (III) chloride heptahydrate (CeCl₃·7 H₂O, 1.1 equiv) and appropriate arylamines (1.0 equiv) in ethanol was stirred at 60-90 °C for 2-6 h. After completion of reaction by TLC, it was cooled, filtered, and recrystallized with ethanol to give dark purple powder of the desire compounds **I-a.**
**General Protocol C:** Hydrolysis reaction. 7-chloro-6-((2,6-difluoro-4-(4-methylpiperazin-1-yl) phenyl)amino)quinoline-5,8-diones **1-a'** (0.66 mmol) was added to a stirred solution of KOH (0.37 g, 6.6 mmol) in MeOH: H₂O (3:1) and heated to 80 °C. After 2 h, the dark red solution was cooled to rt. 10 mL of ethyl acetate was added, which was washed with water (10 mL) and saturated aqueous NaCl solution (10 mL). The organic layer was dried over sodium sulfate, and subsequently the solvent was distilled off under reduced pressure. This was purified by silica gel column chromatography, the desired compound **28 (1-b)** was obtained.

### General Protocol N: Cytotoxicity.

### MTT assay

The evaluation of cytotoxicity was based on the reduction of MTT dye by viable cells to give purple formazan products, which can be measured spectrophotometrically at 540 nm. One hundred eighty microliters of cancer cells were seeded into 96-well plates at 3,500-4,000 cells/well and incubated at 37°C overnight before the indicated treatments. After 72h, 20 µl of MTT solution (3 mg/ml) was added and incubated again for 3 h. After removal of media and solubilization of formazan crystals in 150 µL of DMSO, the absorbance was measured at 570 nm. Percentage of cell growth inhibition was expressed as 1-[(A-B)/(C-B)]x100% (A, B and C were the absorbance values from experimental, blank and control cells, respectively).

Representative compounds were tested in 60 cell lines (NCI60) at the National Cancer Institute, Developmental Therapeutics Program.

### Example II.

¹HNMR data, LC/MS data and purity of the compounds of formulas XII, XIII, XIV and XV prepared by the above procedure are summarized as follow:
**Example 1:** ***7-Chloro-6-((2,6-difluoro-4-(4-methylpiperazin-1-yl)phenyl)amino)quinoline-5,8-dione (1)**.* Following general protocol B. A solution of 2,6-difluoro-4-(4-methylpiperazin-1-yl)aniline (22.7 mg, 0.1 mmol), 6,7-dichloroquinoline-5,8-dione (22.7 mg, 0.1 mmol), and Cerium(III) chloride heptahydrate (41 mg, 0.11 mmol) in ethanol (2 mL) was stirred at 65 °C for 3 h. 7-chloro-6-((2,6-difluoro-4-(4-methylpiperazin-1-yl)phenyl)amino)quinoline-5,8-dione was recovered as a dark purple solid (28.8 mg, 69% yield). ¹H NMR (300 MHz, Methanol-*d₄*) δ 9.01 (s, 1H), 8.55 (d, *J* = 7.7 Hz, 1H), 7.89 (s, 1H), 6.75 (d, *J* = 10.8 Hz, 2H), 3.97 (s, 2H), 3.64 (s, 2H), 3.35 (d, *J* = 11.5 Hz, 4H), 3.00 (s, 3H). LCMS (ESI) 419.00 [M + H]⁺. HPLC purity at 254 nm, 100%.
**Example 2: *7-Chloro-6-((4-(4-(methylsulfonyl)piperazin-1-yl)phenyl)amino)quinoline-5,8-dione (2)***. Following general protocol B. A solution of 4-(4-(methylsulfonyl)piperazin-1-yl)aniline (25.5 mg, 0.1 mmol), 6,7-dichloroquinoline-5,8-dione (22.7 mg, 0.1 mmol), and Cerium(III) chloride heptahydrate (41 mg, 0.11 mmol) in ethanol (2 mL) was stirred at 65 °C for 3 h. 7-chloro-6-((4-(4-(methylsulfonyl)piperazin-1-yl)phenyl)amino)quinoline-5,8-dione was recovered as a dark purple solid (31.2 mg, 70% yield). ¹H NMR (300 MHz, Methanol-*d₄*) δ 8.91 (dd, *J* = 4.8, 1.7 Hz, 1H), 8.47 (dd, *J* = 7.9, 1.7 Hz, 1H), 7.76 (dd, *J* = 7.9, 4.8 Hz, 1H), 7.09 (d, *J* = 9.0 Hz, 2H), 6.98 (d, *J* = 9.0 Hz, 2H), 3.39 - 3.32 (m, 8H), 2.87 (s, 3H). LCMS (ESI) 447.00 [M + H]⁺. HPLC purity at 254 nm, 95.6%.
**Example 3: *7-Chloro-6-((4-(4-methylpiperazin-1-yl)-3-(trifluoromethyl)phenyl)amino) quinoline-5,8-dione (3).*** Following general protocol B. A solution of 4-(4-methylpiperazin-1-yl)-3-(trifluoromethyl)aniline (25.9 mg, 0.1 mmol), 6,7-dichloroquinoline-5,8-dione (22.7 mg, 0.1 mmol), and Cerium(III) chloride heptahydrate (41 mg, 0.11 mmol) in ethanol (2 mL) was stirred at 65 °C for 3 h. 7-chloro-6-((4-(4-methylpiperazin-1-yl)-3-(trifluoromethyl)phenyl)amino)quinoline-5,8-dione was recovered as a dark purple solid (18.5 mg, 41% yield). ¹H NMR (300 MHz, Methanol-*d₄*) δ 8.96 (d, *J* = 5.1 Hz, 1H), 8.53 (d, *J* = 6.4 Hz, 1H), 7.87 - 7.77 (m, 1H), 7.59 - 7.47 (m, 2H), 7.42 (dd, *J =* 8.6, 2.3 Hz, 1H), 3.62 (d, *J* = 9.9 Hz, 2H), 3.28 - 3.20 (m, 6H), 3.01 (s, 3H). LCMS (ESI) 451.10 [M + H]⁺. HPLC purity at 254 nm, 98.3%.
**Example 4: *5-((7-Chloro-5,8-dioxo-5,8-dihydroquinolin-6-yl)amino)-2-(4-methylpiperazin-1-yl) benzonitrile (4)*.** Following general protocol B. A solution of 5-amino-2-(4-methylpiperazin-1-yl)benzonitrile (21.6 mg, 0.1 mmol), 6,7-dichloroquinoline-5,8-dione (22.7 mg, 0.1 mmol), and Cerium(III) chloride heptahydrate (41 mg, 0.11 mmol) in ethanol (2 mL) was stirred at 65 °C for 3 h. 5-((7-chloro-5,8-dioxo-5,8-dihydroquinolin-6-yl)amino)-2-(4-methylpiperazin-1-yl) benzonitrile was recovered as a dark purple solid (22.0 mg, 54% yield). ¹H NMR (300 MHz, Methanol-*d₄*) δ 8.97 (d, *J* = 4.4 Hz, 1H), 8.52 (d, *J* = 7.8 Hz, 1H), 7.82 (dd, *J* = 7.8, 4.7 Hz, 1H), 7.53 (d, *J* = 2.3 Hz, 1H), 7.44 (dd, *J* = 8.7, 2.3 Hz, 1H), 7.27 (d, *J* = 8.8 Hz, 1H), 3.72 (t, *J* = 12.5 Hz, 4H), 3.41 (t, *J* = 11.5 Hz, 2H), 3.24 (d, *J* = 11.0 Hz, 2H), 3.04 (s, 3H). LCMS (ESI) 408.10 [M + H]⁺. HPLC purity at 254 nm, 99.2%.
**Example 5: *7-Chloro-6-((4-(4-methylpiperazin-1-yl)phenyl)amino)quinoline-5,8-dione* (5).** Following general protocol B. A solution of 4-(4-methylpiperazin-1-yl)aniline (19.1 mg, 0.1 mmol), 6,7-dichloroquinoline-5,8-dione (22.7 mg, 0.1 mmol), and Cerium(III) chloride heptahydrate (41 mg, 0.11 mmol) in ethanol (2 mL) was stirred at 65 °C for 3 h. 7-chloro-6-((4-(4-methylpiperazin-1-yl)phenyl)amino)quinoline-5,8-dione was recovered as a dark purple solid (27.1 mg, 71% yield). ¹H NMR (400 MHz, Methanol-*d₄*) δ 8.92 (s, 1H), 8.51 (dd, *J* = 7.9, 1.1 Hz, 1H), 7.79 (dd, *J* = 7.8, 4.8 Hz, 1H), 7.21 - 7.12 (m, 2H), 7.09 - 6.99 (m, 2H), 3.90 (d, *J=* 13.1 Hz, 2H), 3.70 - 3.60 (m, 2H), 3.36 - 3.26 (m, 2H), 3.09 (t, *J* = 12.1 Hz, 2H), 3.01 (s, 3H). LCMS (ESI) 383.1 [M + H]⁺. HPLC purity at 254 nm, 98.8%.
**Example 6: *7-Chloro-6-((4-(piperidin-1-yl)phenyl)amino)quinoline-5,8-dione (6).*** Following general protocol B. A solution of 4-(piperidin-1-yl)aniline (17.6 mg, 0.1 mmol), 6,7-dichloroquinoline-5,8-dione (22.7 mg, 0.1 mmol), and Cerium(III) chloride heptahydrate (41 mg, 0.11 mmol) in ethanol (2 mL) was stirred at 65 °C for 3 h. 7-chloro-6-((4-(piperidin-1-yl)phenyl)amino)quinoline-5,8-dione was recovered as a dark purple solid (22.4 mg, 61% yield). ¹H NMR (300 MHz, Methanol-*d₄*) δ 8.97 (d, *J* = 4.4 Hz, 1H), 8.53 (d, *J* = 7.6 Hz, 1H), 7.83 (dd, *J* = 7.2, 4.6 Hz, 1H), 7.63 (d, *J* = 8.1 Hz, 2H), 7.34 (d, *J* = 8.6 Hz, 2H), 3.71 - 3.61 (m, 4H), 2.13 - 1.99 (m, 4H), 1.89 - 1.72 (m, 2H). LCMS (ESI) 368.00 [M + H]⁺. HPLC purity at 254 nm, 98.9%.
**Example 7:** ***6-((4-(1H-Imidazol-1-yl)phenyl)amino)-7-chloroquinoline-5,8-dione** (7).* Following general protocol B. A solution of 4-(1H-imidazol-1-yl)aniline (15.9 mg, 0.1 mmol), 6,7-dichloroquinoline-5,8-dione (22.7 mg, 0.1 mmol), and Cerium(III) chloride heptahydrate (41 mg, 0.11 mmol) in ethanol (2 mL) was stirred at 65 °C for 3 h. 6-((4-(1H-imidazol-1-yl)phenyl)amino)-7-chloroquinoline-5,8-dione was recovered as a dark purple solid (19.2 mg, 55% yield). ¹H NMR (300 MHz, Methanol-*d₄*) δ 9.43 (s, 1H), 8.98 (dd, *J* = 4.8, 1.6 Hz, 1H), 8.55 (dd, *J* = 7.9, 1.7 Hz, 1H), 8.09 (t, *J* = 1.7 Hz, 1H), 7.84 (dd, *J* = 7.9, 4.8 Hz, 1H), 7.79 - 7.76 (m, 1H), 7.73 (d, *J* = 8.9 Hz, 2H), 7.41 (d, *J* = 8.9 Hz, 2H). LCMS (ESI) 351.00 [M + H]⁺. HPLC purity at 254 nm, 96.3%.
**Example 8: *7-Chloro-6-((4-(pyridin-4-yl)phenyl)amino)quinoline-5,8-dione (8).*** Following general protocol B. A solution of 4-(pyridin-4-yl)aniline (17.0 mg, 0.1 mmol), 6,7-dichloroquinoline-5,8-dione (22.7 mg, 0.1 mmol), and Cerium(III) chloride heptahydrate (41 mg, 0.11 mmol) in ethanol (2 mL) was stirred at 65 °C for 3 h. 7-chloro-6-((4-(pyridin-4-yl)phenyl)amino)quinoline-5,8-dione was recovered as a dark purple solid (17.7 mg, 49% yield). ¹H NMR (300 MHz, Methanol-*d₄*) δ 8.99 (dd, *J* = 4.8, 1.6 Hz, 1H), 8.82 (d, *J* = 7.0 Hz, 2H), 8.56 (dd, *J* = 7.9, 1.7 Hz, 1H), 8.40 (d, *J* = 7.0 Hz, 2H), 8.07 - 8.00 (m, 2H), 7.85 (dd, *J* = 7.9, 4.8 Hz, 1H), 7.37 (d, *J* = 8.8 Hz, 2H). LCMS (ESI) 361.90 [M + H]⁺. HPLC purity at 254 nm, 96.0%.
**Example 9: *7-Chloro-6-((4-(thiazol-2-yl)phenyl)amino)quinoline-5,8-dione (9).*** Following general protocol B. A solution of 4-(thiazol-2-yl)aniline (17.6 mg, 0.1 mmol), 6,7-dichloroquinoline-5,8-dione (22.7 mg, 0.1 mmol), and Cerium(III) chloride heptahydrate (41 mg, 0.11 mmol) in ethanol (2 mL) was stirred at 65 °C for 3 h. 7-chloro-6-((4-(thiazol-2-yl)phenyl)amino)quinoline-5,8-dione was recovered as a dark purple solid (18.0 mg, 49% yield). ¹H NMR (300 MHz, Methanol-*d₄*) δ 8.98 (d, *J* = 4.8 Hz, 1H), 8.55 (d, *J* = 8.7 Hz, 1H), 7.96 (d, *J* = 8.4 Hz, 2H), 7.89 (d, *J* = 3.7 Hz, 1H), 7.84 (dd, *J* = 8.5, 4.2 Hz, 1H), 7.63 (d, *J* = 2.9 Hz, 1H), 7.27 (d, *J* = 8.6 Hz, 2H). LCMS (ESI) 368.00 [M + H]⁺. HPLC purity at 254 nm, 97.9%.
**Example 10: *6-((4-(4-Acetylpiperazin-1-yl)phenyl)amino)-7-chloroquinoline-5,8-dione*** (10). Following general protocol B. A solution of 1-(4-(4-aminophenyl)piperazin-1-yl)ethan-1-one (21.9 mg, 0.1 mmol), 6,7-dichloroquinoline-5,8-dione (22.7 mg, 0.1 mmol), and Cerium(III) chloride heptahydrate (41 mg, 0.11 mmol) in ethanol (2 mL) was stirred at 65 °C for 3 h. 6-((4-(4-acetylpiperazin-1-yl)phenyl)amino)-7-chloroquinoline-5,8-dione was recovered as a dark purple solid (25.4 mg, 62% yield). ¹H NMR (300 MHz, Methanol*d₄*) δ 8.95 (s, 1H), 8.52 (d, *J* = 7.6 Hz, 1H), 7.85 - 7.76 (m, 1H), 7.15 (d, *J* = 8.9 Hz, 2H), 7.08 (d, *J* = 8.9 Hz, 2H), 3.84 - 3.71 (m, 4H), 3.29 - 3.23 (m, 4H), 2.18 (s, 3H). LCMS (ESI) 411.10 [M + H]⁺. HPLC purity at 254 nm, 99.4%.
**Example 11: *7-Chloro-6-((3-fluoro-4-(piperazin-1-yl)phenyl)amino)quinoline-5,8-dione*** (11). Following general protocol B. A solution of tert-butyl 4-(4-amino-2-fluorophenyl)piperazine-1-carboxylate (29.5 mg, 0.1 mmol), 6,7-dichloroquinoline-5,8-dione (22.7 mg, 0.1 mmol), and Cerium(III) chloride heptahydrate (41 mg, 0.11 mmol) in ethanol (2 mL) was stirred at 65 °C for 3 h. 7-chloro-6-((3-fluoro-4-(piperazin-1-yl)phenyl)amino)quinoline-5,8-dione was recovered as a dark purple solid (23.5 mg, 61% yield). ¹H NMR (300 MHz, Methanol-*d₄*) δ 8.96 (d, *J* = 4.8 Hz, 1H), 8.52 (dd, *J* = 7.9, 1.5 Hz, 1H), 7.81 (dd, *J* = 7.8, 4.7 Hz, 1H), 7.10 (t, *J* = 9.1 Hz, 1H), 7.05 - 6.94 (m, 2H), 3.46 - 3.40 (m, 4H), 3.38 - 3.34 (m, 4H). LCMS (ESI) 378.00 [M + H]⁺. HPLC purity at 254 nm, 99.5%.
**Example 12: *7-Chloro-6-((4-(4-(4-methoxyphenyl)piperazin-1-yl)phenyl)amino)quinoline-5,8-dione* (12).** Following general protocol B. A solution of 4-(4-(4-methoxyphenyl)piperazin-1-yl)aniline (28.3 mg, 0.1 mmol), 6,7-dichloroquinoline-5,8-dione (22.7 mg, 0.1 mmol), and Cerium(III) chloride heptahydrate (41 mg, 0.11 mmol) in ethanol (2 mL) was stirred at 65 °C for 3 h. 7-chloro-6-((4-(4-(4-methoxyphenyl)piperazin-1-yl)phenyl)amino)quinoline-5,8-dione was recovered as a dark purple solid (28.4 mg, 60% yield). ¹H NMR (300 MHz, Methanol-*d₄*) δ 8.96 (d, *J* = 5.5 Hz, 1H), 8.52 (d, *J* = 7.4 Hz, 1H), 7.85 - 7.77 (m, 1H), 7.55 (d, *J* = 8.6 Hz, 2H), 7.14 (t, *J* = 10.1 Hz, 6H), 3.87 (s, 3H), 3.80 - 3.74 (m, 4H), 3.67 - 3.60 (m, 4H). LCMS (ESI) 475.10 [M + H]⁺. HPLC purity at 254 nm, 98.9%.
**Example 13: *7-Chloro-2-methyl-6-((4-(4-methylpiperazin-1-yl)phenyl)amino)quinoline-5,8-dione (13).*** Following general protocol B. A solution of 4-(4-methylpiperazin-1-yl)aniline (19.1 mg, 0.1 mmol), 6,7-dichloro-2-methylquinoline-5,8-dione (24.1 mg, 0.1 mmol), and Cerium(III) chloride heptahydrate (41 mg, 0.11 mmol) in ethanol (2 mL) was stirred at 65 °C for 3 h. 7-chloro-2-methyl-6-((4-(4-methylpiperazin-1-yl)phenyl)amino)quinoline-5,8-dione was recovered as a dark purple solid (23.4 mg, 59% yield). ¹H NMR (300 MHz, Methanol-*d₄*) δ 8.37 (d, *J* = 8.0 Hz, 1H), 7.65 (d, *J* = 8.0 Hz, 1H), 7.17 - 7.12 (m, 2H), 7.04 (d, *J* = 9.1 Hz, 2H), 4.97 (s, 2H), 3.88 (s, 2H), 3.59 (d, *J* = 20.2 Hz, 2H), 3.08 (s, 2H), 3.00 (s, 3H), 2.72 (s, 3H). LCMS (ESI) 397.00 [M + H]⁺. HPLC purity at 254 nm, 99.3%.
**Example 14: *7-Chloro-6-((2,6-difluoro-4-(4-methylpiperazin-1-yl)phenyl)andno)-2-methylquinoline-5,8-dione* (14).** Following general protocol B. A solution of 2,6-difluoro-4-(4-methylpiperazin-1-yl)aniline (22.7 mg, 0.1 mmol), 6,7-dichloro-2-methylquinoline-5,8-dione (24.1 mg, 0.1 mmol), and Cerium(III) chloride heptahydrate (41 mg, 0.11 mmol) in ethanol (2 mL) was stirred at 65 °C for 3 h. 7-chloro-6-((2,6-difluoro-4-(4-methylpiperazin-1-yl)phenyl)amino)-2-methylquinoline-5,8-dione was recovered as a dark purple solid (30.2 mg, 70% yield). ¹H NMR (300 MHz, Methanol-*d₄*) δ 8.36 (d, *J* = 8.1 Hz, 1H), 7.66 (d, *J* = 8.2 Hz, 1H), 6.76 (d, *J* = 10.2 Hz, 2H), 3.96 (s, 2H), 3.60 (s, 2H), 3.18 (dd, *J* = 35.3, 14.7 Hz, 4H), 3.00 (s, 3H), 2.72 (s, 3H). LCMS (ESI) 433.20 [M + H]⁺. HPLC purity at 254 nm, 99.4%.
**Example 15:** ***7-Chloro-2-methyl-6-((4-(4-(methylsulfonyl)piperazin-1-yl)phenyl)amino) quinoline-5,8-dione** (15).* Following general protocol B. A solution of 4-(4-(methylsulfonyl)piperazin-1-yl)aniline (25.5 mg, 0.1 mmol), 6,7-dichloro-2-methylquinoline-5,8-dione (24.1 mg, 0.1 mmol),and Cerium(III) chloride heptahydrate (41 mg, 0.11 mmol) in ethanol (2 mL) was stirred at 65 °C for 3 h. 7-chloro-2-methyl-6-((4-(4-(methylsulfonyl)piperazin-1-yl)phenyl)amino)quinoline-5,8-dione was recovered as a dark purple solid (35.4 mg, 77% yield). ¹H NMR (300 MHz, Methanol-*d₄*) δ 8.37 (d, *J* = 7.5 Hz, 1H), 7.65 (d, *J=* 8.7 Hz, 1H), 7.11 (d, *J=* 8.4 Hz, 2H), 7.02 (d, *J=* 8.8 Hz, 2H), 3.40 (d, *J* = 8.8 Hz, 8H), 2.91 (s, 3H), 2.72 (s, 3H). LCMS (ESI) 461.10 [M + H]⁺. HPLC purity at 254 nm, 98.8%.
**Example 16:** ***5-(7-Chloro-2-methyl-5,8-dioxo-5,8-dihydroquinolin-6-yl)-2-(4-methylpiperazin-1-yl)** benzonitrile (16).* Following general protocol B. A solution of 5-amino-2-(4-methylpiperazin-1-yl)benzonitrile (21.6 mg, 0.1 mmol), 6,7-dichloro-2-methylquinoline-5,8-dione (21.6 mg, 0.1 mmol), and Cerium(III) chloride heptahydrate (41 mg, 0.11 mmol) in ethanol (2 mL) was stirred at 65 °C for 3 h. 5-((7-chloro-2-methyl-5,8-dioxo-5,8-dihydroquinolin-6-yl)amino)-2-(4-methylpiperazin-1-yl)benzonitrile was recovered as a dark purple solid (28.6 mg, 68% yield). ¹H NMR (300 MHz, Methanol-*d₄*) δ 8.35 (d, *J=* 7.7 Hz, 1H), 7.65 (d, *J* = 7.6 Hz, 1H), 7.50 (s, 1H), 7.41 (d, *J* = 8.5 Hz, 1H), 7.24 (d, *J=* 8.8 Hz, 1H), 3.70 (t, *J=* 14.1 Hz, 6H), 3.45 - 3.36 (m, 2H), 3.01 (s, 3H), 2.71 (s, 3H). LCMS (ESI) 422.10 [M + H]⁺. HPLC purity at 254 nm, 99.2%.
**Example 17: *7-Chloro-2-methyl-6-((4-(4-methylpiperazin-1 yl)-3-(trifluoromethyl)phenyl) amino)quinoline-5,8-dione* (17)**. Following general protocol B. A solution of 4-(4-methylpiperazin-1-yl)-3-(trifluoromethyl)aniline (25.9 mg, 0.1 mmol), 6,7-dichloro-2-methylquinoline-5,8-dione (24.1 mg, 0.1 mmol),and Cerium(III) chloride heptahydrate (41 mg, 0.11 mmol) in ethanol (2 mL) was stirred at 65 °C for 3 h. 7-chloro-2-methyl-6-((4-(4-methylpiperazin-1-yl)-3-(trifluoromethyl) phenyl)amino) quinoline-5,8-dione was recovered as a dark purple solid (31.0 mg, 67% yield). ¹H NMR (300 MHz, Methanol-*d₄*) δ 8.38 (d, *J=* 8.3 Hz, 1H), 7.68 (d, *J* = 8.3 Hz, 1H), 7.56 (d, *J* = 8.9 Hz, 1H), 7.51 (s, 1H), 7.41 (d, *J=* 6.8 Hz, 1H), 3.62 (d, *J=* 10.7 Hz, 2H), 3.25 (s, 6H), 3.01 (s, 3H), 2.73 (s, 3H). LCMS (ESI) 456.20 [M + H]⁺. HPLC purity at 254 nm, 97.9%.
**Example 18**: ***7-Chloro-6-[2-methoxy-4-(4-methylpiperazin-1-yl)phenyl]-2-methylquinoline-5,8-dione (18)***. Following general protocol B. A solution of 2-methoxy-4-(4-methylpiperazin-1-yl)aniline (22.1 mg, 0.1 mmol), 6,7-dichloro-2-methylquinoline-5,8-dione (24.1 mg, 0.1 mmol),and Cerium(III) chloride heptahydrate (41 mg, 0.11 mmol) in ethanol (2 mL) was stirred at 65 °C for 3 h. 7-chloro-6-((2-methoxy-4-(4-methylpiperazin-1-yl)phenyl)amino)-2-methylquinoline-5,8-dione was recovered as a dark purple solid (32.0 mg, 75% yield). ¹H NMR (300 MHz, Methanol-*d₄*) δ 8.31 (d, *J=* 7.8 Hz, 1H), 7.61 (d, *J=* 8.0 Hz, 1H), 7.08 (d, *J=* 8.0 Hz, 1H), 6.65 (s, 1H), 6.59 (d, *J=* 9.2 Hz, 1H), 3.90 (s, 2H), 3.79 (s, 3H), 3.62 (s, 2H), 3.28 - 3.21 (m, 2H), 3.12 (d, *J=* 12.4 Hz, 2H), 2.99 (s, 3H), 2.70 (s, 3H). LCMS (ESI) 427.10 [M + H]⁺. HPLC purity at 254 nm, 98.8%.
**Example 19: *7-Chloro-6-[3,5-difluoro-4-(4-methylpiperazin-1-yl)phenyl]quinoline-5,8-dione (19).*** Following general protocol B. A solution of 3,5-difluoro-4-(4-methylpiperazin-1-yl)aniline (22.7 mg, 0.1 mmol), 6,7-dichloroquinoline-5,8-dione (22.7 mg, 0.1 mmol), and Cerium(III) chloride heptahydrate (41 mg, 0.11 mmol) in ethanol (2 mL) was stirred at 65 °C for 3 h. 7-chloro-6-((3,5-difluoro-4-(4-methylpiperazin-1-yl)phenyl)amino)quinoline-5,8-dione was recovered as a dark purple solid (33.0 mg, 79% yield). ¹H NMR (400 MHz, DMSO-*d*₆) δ 9.78 (brs, 1H), 9.39 (s, 1H), 9.00 (dd, *J* = 4.7, 1.7 Hz, 1H), 8.39 (dd, *J* = 7.9, 1.7 Hz, 1H), 7.81 (dd, *J* = 7.9, 4.7 Hz, 1H), 6.88 (d, *J=* 11.0 Hz, 2H), 3.37 - 3.10 (m, 8H), 2.87 (s, 3H). LCMS (ESI) 419.00 [M + H]⁺. HPLC purity at 254 nm, 98.2%.
**Example 22: *7-Chloro-6-[4-(ethylamino)-2-(trifluoromethyl)phenyl]quinoline-5,8-dione* (22).** Following general protocol B. A solution of N¹-ethyl-3-(trifluoromethyl)benzene-1,4-diamine (20.4 mg, 0.1 mmol), 6,7-dichloroquinoline-5,8-dione (22.7 mg, 0.1 mmol), and Cerium(III) chloride heptahydrate (41 mg, 0.11 mmol) in ethanol (2 mL) was stirred at 65 °C for 3 h. 7-chloro-6-((4-(ethylamino)-2-(trifluoromethyl)phenyl)amino)quinoline-5,8-dione was recovered as a dark purple solid (17.8 mg, 45% yield). ¹H NMR (400 MHz, Methanol-*d₄*) δ 8.93 (s, 1H), 8.45 (dd, *J* = 7.9, 1.6 Hz, 1H), 7.81 (dd, *J* = 7.9, 4.6 Hz, 1H), 7.16 - 7.07 (m, 2H), 6.89 (dt, *J=* 8.7, 0.7 Hz, 1H), 4.05 - 3.94 (m, 2H), 1.34 - 1.29 (m, 3H). LCMS (ESI) 396.00 [M + H]⁺. HPLC purity at 254 nm, 97.4%.
**Example 28: *7-Chloro-6-[2-fluoro-6-hydroxy-4-(4-methylpiperazin-1-yl)phenyl]quinoline-5,8-dione (28).*** Following general protocol C. 7-chloro-6-((2,6-difluoro-4-(4-methylpiperazin-1-yl) phenyl)amino)quinoline-5,8-dione 1 (0.66 mmol) was added to a stirring solution of KOH (0.37 g, 6.6 mmol) in MeOH: H₂O (3:1) and heated to 80 °C. After 2 h, the dark red solution was cooled to rt. 10 mL of ethyl acetate was added, which was washed with water (10 mL) and saturated aqueous NaCl solution (10 mL). The organic layer was dried over sodium sulfate, and subsequently the solvent was distilled off under reduced pressure. This was purified by silica gel column chromatography, the desired compound 28 was obtained (71%). ¹H NMR (400 MHz, Methanol-*d₄*) δ 8.73 (s, 1H), 8.46 (t, *J* = 8.2 Hz, 1H), 7.73 (s, 1H), 6.77 - 6.51 (m, 2H), 3.86 (s, 2H), 3.62 (s, 2H), 3.37 - 3.32 (m, 2H), 3.07 (s, 2H), 3.00 (s, 3H). LCMS (ESI) 417.00 [M + H]⁺. HPLC purity at 254 nm, 100%.
**Example 42: *9-(4-methylpiperazin-1-yl)pyrido[2,3-b]phenazine-5,12-dione (42).*** Following general protocol J. A solution of 6,7-dichloro-5,8-quinolinediones (0.1 equiv.), cerium(III) chloride heptahydrate (CeCl₃·7 H₂O, 0.11 equiv.) and 4-(4-methylpiperazin-1-yl)aniline (19.1 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 9-(4-methylpiperazin-1-yl)pyrido[2,3-b]phenazine-5,12-dione was recovered as a dark purple solid (9.7 mg, 27% yield). ¹H NMR (300 MHz, Methanol-*d*₄) δ 9.13 (d, *J* = 5.0 Hz, 1H), 8.85 (d, *J* = 8.4 Hz, 1H), 8.24 (d, *J*= 9.5 Hz, 1H), 8.11 - 7.94 (m, 2H), 7.58 (s, 1H), 3.91 (s, 4H), 3.52 (s, 4H), 3.02 (s, 3H). LCMS (ESI) 360.00 [M + H]⁺. HPLC purity at 254 nm, 99.2%.
**Example 43: *7-methoxy-9-(4-methylpiperazin-1-yl)pyrido[2,3-b]phenazine-5,12-dione*** (43). Following general protocol J. A solution of 6,7-dichloro-5,8-quinolinediones (0.1 equiv.), cerium(III) chloride heptahydrate (CeCl₃·7 H₂O, 0.11 equiv.) and 2-methoxy-4-(4-methylpiperazin-1-yl)aniline (22.1 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 7-methoxy-9-(4-methylpiperazin-1-yl)pyrido[2,3-b]phenazine-5,12-dione was recovered as a dark purple solid (19.1 mg, 49% yield). ¹H NMR (300 MHz, Methanol-*d*₄) δ 9.13 (s, 1H), 8.82 (d, *J=* 6.9 Hz, 1H), 8.03 (d, *J=* 7.6 Hz, 1H), 7.12 (s, 1H), 6.93 (s, 1H), 4.06 (s, 3H), 3.59 (s, 8H), 3.09 (s, 3H). LCMS (ESI) 390.10 [M + H]⁺. HPLC purity at 254 nm, 99.3%.
**Example 44: *2-methyl-9-(4-methylpiperazin-1-yl)-5,12-dioxo-5,12-dihydropyrido[2,3-b]phenazine-10-carbonitrile* (44)**. Following general protocol J. A solution of 6,7-dichloro-2-methylquinoline-5,8-dione (0.1 equiv.), cerium(III) chloride heptahydrate (CeCl₃·7 H₂O, 0.11 equiv.) and 5-amino-2-(4-methylpiperazin-1-yl)benzonitrile (21.6 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 2-methyl-9-(4-methylpiperazin-1-yl)-5,12-dioxo-5,12-dihydropyrido[2,3-b]phenazine-10-carbonitrile was recovered as a dark purple solid (19.9 mg, 50% yield). ¹H NMR (400 MHz, Methanol-*d*₄) δ 8.72 (d, *J* = 8.1 Hz, 1H), 8.36 (d, *J* = 9.5 Hz, 1H), 8.00 (d, *J* = 9.5 Hz, 1H), 7.89 (d, *J=* 8.2 Hz, 1H), 3.82 - 3.46 (m, 6H), 3.37 (s, 2H), 3.09 (s, 3H), 2.84 (s, 3H). LCMS (ESI) 399.05 [M + H]⁺. HPLC purity at 254 nm, 100%.
**Example 45: *9-(4-methylpiperazin-1-yl)-5,12-dioxo-5,12-dihydropyrido[2,3-b]phenazine-10-carbonitrile* (45).** Following general protocol J. A solution of 6,7-dichloro-5,8-quinolinediones (0.1 equiv.), cerium(III) chloride heptahydrate (CeCl₃·7 H₂O, 0.11 equiv.) and 5-amino-2-(4-methylpiperazin-1-yl)benzonitrile (21.6 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 9-(4-methylpiperazin-1-yl)-5,12-dioxo-5,12-dihydropyrido[2,3-b]phenazine-10-carbonitrile was recovered as a dark purple solid (11.1 mg, 29% yield). ¹H NMR (400 MHz, DMSO-*d*₆) δ 9.17 (dd, *J* = 4.6, 1.7 Hz, 1H), 9.07 (s, 1H), 8.70 (dd, *J* = 7.9, 1.7 Hz, 1H), 8.06 (s, 1H), 8.00 (dd, *J* = 7.9, 4.6 Hz, 1H), 4.00 (s, 6H), 2.90 (d, *J* = 17.4 Hz, 5H). LCMS (ESI) 385.25 [M + H]⁺. HPLC purity at 254 nm, 98.5%.
**Example 46: *9-(4-methylpiperazin-1-yl)-5,12-dioxo-5,12-dihydropyrido[2,3-b]phenazine-8-carbonitrile* (46)**. Following general protocol J. A solution of 6,7-dichloro-5,8-quinolinediones (0.1 equiv.), cerium(III) chloride heptahydrate (CeCl₃·7 H₂O, 0.11 equiv.) and 5-amino-2-(4-methylpiperazin-1-yl)benzonitrile (21.6 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 9-(4-methylpiperazin-1-yl)-5,12-dioxo-5,12-dihydropyrido[2,3-b]phenazine-8-carbonitrile was recovered as a dark purple solid (8.4 mg, 22% yield). ¹H NMR (400 MHz, DMSO-*d*₆) δ 9.17 (dd, *J* = 4.6, 1.7 Hz, 1H), 9.07 (s, 1H), 8.70 (dd, *J* = 7.9, 1.7 Hz, 1H), 8.06 (s, 1H), 8.00 (dd, *J* = 7.9, 4.6 Hz, 1H), 4.00 (s, 6H), 2.90 (d, *J* = 17.4 Hz, 5H). LCMS (ESI) 384.95 [M + H]⁺. HPLC purity at 254 nm, 98.1%.
**Example 47: *9-(4-(methylsulfonyl)piperazin-1-yl)-5,12-dioxo-5,12-dihydropyrido[2,3-b]phenazine-10-carbonitrile*** (47). Following general protocol J. A solution of 6,7-dichloro-5,8-quinolinediones (0.1 equiv.), cerium(III) chloride heptahydrate (CeCl₃·7 H₂O, 0.11 equiv.) and 5-amino-2-(4-(methylsulfonyl)piperazin-1-yl)benzonitrile (28.0 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 9-(4-(methylsulfonyl)piperazin-1-yl)-5,12-dioxo-5,12-dihydropyrido[2,3-b]phenazine-10-carbonitrile was recovered as a dark purple solid (22.0 mg, 49% yield). ¹H NMR (400 MHz, DMSO-*d*₆) δ 9.16 (dd, *J=* 4.6, 1.6 Hz, 1H), 8.67 (dd, *J=* 7.9, 1.6 Hz, 1H), 8.42 (d, *J* = 9.8 Hz, 1H), 8.03 (d, *J=* 9.5 Hz, 1H), 7.98 (dd, *J=* 7.8, 4.6 Hz, 1H), 3.98 (s, 4H), 3.42 (s, 4H), 3.00 (s, 3H). LCMS (ESI) 448.90 [M + H]⁺. HPLC purity at 254 nm, 96.0%.
**Example 48: *9-(4-ethylpiperazin-1-yl)pyrido[2,3-b]phenazine-5,12-dione* (48).** Following general protocol J. A solution of 6,7-dichloro-5,8-quinolinediones (0.1 equiv.), cerium(III) chloride heptahydrate (CeCl₃·7 H₂O, 0.11 equiv.) and 4-(4-ethylpiperazin-1-yl)aniline (20.5 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 9-(4-ethylpiperazin-1-yl)pyrido[2,3-b]phenazine-5,12-dione was recovered as a dark purple solid (11.6 mg, 31% yield). ¹H NMR (400 MHz, DMSO-*d*₆) δ 9.14 (dd, *J=* 4.6, 1.7 Hz, 1H), 8.66 (dd, *J=* 7.9, 1.7 Hz, 1H), 8.26 (d, *J=* 9.5 Hz, 1H), 8.08 (d, *J* = 2.7 Hz, 1H), 7.96 (dd, *J* = 7.9, 4.6 Hz, 1H), 7.67 *(d, J=* 2.7 Hz, 1H), 4.44 (s, 2H), 3.66 (s, 4H), 3.24 (q, *J=* 7.1 Hz, 4H), 1.29 (t, *J=* 7.3 Hz, 3H). LCMS (ESI) 374.05 [M + H]⁺. HPLC purity at 254 nm, 100%.
**Example 49: *9-(4-cyclopropylpiperazin-1-yl)pyrido[2,3-b]phenazine-5,12-dione (49).*** Following general protocol J. A solution of 6,7-dichloro-5,8-quinolinediones (0.1 equiv.), cerium(III) chloride heptahydrate (CeCl₃·7 H₂O, 0.11 equiv.) and 4-(4-cyclopropylpiperazin-1-yl)aniline (21.7 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 9-(4-cyclopropylpiperazin-1-yl)pyrido[2,3-b]phenazine-5,12-dione was recovered as a dark purple solid (23.5 mg, 61% yield). ¹H NMR (400 MHz, Methanol-*d*₄) δ 9.13 (dd, *J=* 4.6, 1.6 Hz, 1H), 8.85 (dd, *J=* 8.0, 1.7 Hz, 1H), 8.24 (d, *J=* 9.5 Hz, 1H), 8.03 (ddd, *J=* 12.6, 8.8, 3.7 Hz, 2H), 7.58 (d, *J* = 2.7 Hz, 1H), 3.94 (s, 4H), 3.69 (s, 4H), 3.04 - 2.90 (m, 1H), 1.16 - 1.02 (m, 4H). LCMS (ESI) 386.05 [M + H]⁺. HPLC purity at 254 nm, 100%.
**Example 50: *9-(4-methylpiperazin-1-yl)-10-(trifluoromethyl)pyrido[2,3-b]phenazine-5,12-dione (50).*** Following general protocol J. A solution of 6,7-dichloro-5,8-quinolinediones (0.1 equiv.), cerium(III) chloride heptahydrate (CeCl₃·7 H₂O, 0.11 equiv.) and 4-(4-methylpiperazin-1-yl)-3-(trifluoromethyl)aniline (25.9 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 9-(4-methylpiperazin-1-yl)-10-(trifluoromethyl)pyrido[2,3-b]phenazine-5,12-dione was recovered as a dark purple solid (14.5 mg, 34% yield). ¹H NMR (400 MHz, Methanol-d4) δ 9.15 (dd, J = 4.6, 1.5 Hz, 1H), 8.85 (dd, J = 8.0, 1.7 Hz, 1H), 8.39 (d, J = 9.5 Hz, 1H), 8.10 (d, J = 9.6 Hz, 1H), 8.02 (dd, J = 8.0, 4.7 Hz, 1H), 4.01 - 3.40 (m, 8H), 3.08 (s, 3H). LCMS (ESI) 428.10 [M + H]⁺. HPLC purity at 254 nm, 99.1%.
**Example 51: *9-(4-methylpiperazin-1-yl)-8-(trifluoromethyl)pyrido[2,3-b]phenazine-5,12-dione (51).*** Following general protocol J. A solution of 6,7-dichloro-5,8-quinolinediones (0.1 equiv.), cerium(III) chloride heptahydrate (CeCl₃·7 H₂O, 0.11 equiv.) and 4-(4-methylpiperazin-1-yl)-3-(trifluoromethyl)aniline (25.9 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 2-methyl-9-(4-methylpiperazin-1-yl)-8-(trifluoromethyl)pyrido[2,3-b] phenazine-5,12-dione was recovered as a dark purple solid (7.1 mg, 16% yield). ¹H NMR (400 MHz, Methanol-d4) δ 9.18 (dd, J = 4.6, 1.6 Hz, 1H), 8.89 (dd, J = 8.0, 1.7 Hz, 1H), 8.80 (s, 1H), 8.40 (s, 1H), 8.05 (dd, J = 8.0, 4.7 Hz, 1H), 3.380-3.71 (m, 2H), 3.65-3.57 (m, 2H), 3.50-3.39 (m, 4H), 3.08 (s, 3H). LCMS (ESI) 428.10 [M + H]⁺. HPLC purity at 254 nm, 99.8%.
**Example 52: *2-methyl-9-(4-methylpiperazin-1-yl)-10-(trifluoromethyl)pyrido[2,3-bJphenazine-5,12-dione* (52).** Following general protocol J. A solution of 6,7-dichloro-2-methylquinoline-5,8-dione (0.1 equiv, 24 mg), cerium(III) chloride heptahydrate (CeCl₃·7 H₂O, 0.11 equiv.) and 4-(4-methylpiperazin-1-yl)-3-(trifluoromethyl)aniline (25.9 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 2-methyl-9-(4-methylpiperazin-1-yl)-10-(trifluoromethyl)pyrido[2,3-b] phenazine-5,12-dione was recovered as a dark purple solid (11.0 mg, 25% yield). ¹H NMR (400 MHz, Methanol-*d*₄) δ 8.69 (d, *J* = 8.1 Hz, 1H), 8.39 (d, *J* = 9.5 Hz, 1H), 8.10 (d, *J* = 9.5 Hz, 1H), 7.87 (d, *J=* 8.1 Hz, 1H), 3.93 (s, 2H), 3.71 (s, 4H), 3.53 - 3.38 (m, 2H), 3.08 (s, 3H), 2.83 (s, 3H). LCMS (ESI) 442.10 [M + H]⁺. HPLC purity at 254 nm, 95.8%.
**Example 53: *2-methyl-9-(4-methylpiperazin-1-yl)-8-(trifluoromethyl)pyrido[2,3-b]phenazine-5,12-dione (53).*** Following general protocol J. A solution of 6,7-dichloro-2-methylquinoline-5,8-dione (0.1 equiv, 24 mg), cerium(III) chloride heptahydrate (CeCl₃·7 H₂O, 0.11 equiv.) and 4-(4-methylpiperazin-1-yl)-3-(trifluoromethyl)aniline (25.9 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 2-methyl-9-(4-methylpiperazin-1-yl)-8-(trifluoromethyl) pyrido[2,3-b] phenazine-5,12-dione was recovered as a dark purple solid (7.1 mg, 16% yield). ¹H NMR (400 MHz, Methanol-*d*₄) δ 8.81 (s, 1H), 8.75 (d, *J=* 8.1 Hz, 1H), 8.40 (s, 1H), 7.91 (d, *J=* 8.2 Hz, 1H), 3.72 (s, 2H), 3.61 (s, 2H), 3.42 (d, *J=* 8.4 Hz, 4H), 3.08 (s, 3H), 2.85 (s, 3H). LCMS (ESI) 441.95 [M + H]⁺. HPLC purity at 254 nm, 98.7%.
**Example 54: *9-(4-cyclopropylpiperazin-1-yl)-2-methylpyrido[2,3-b]phenazine-5,12-dione* (54).** Following general protocol J. A solution of 6,7-dichloro-2-methylquinoline-5,8-dione (0.1 equiv, 24 mg), cerium(III) chloride heptahydrate (CeCl₃·7 H₂O, 0.11 equiv.) and 4-(4-cyclopropylpiperazin-1-yl)aniline (21.7 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 9-(4-cyclopropylpiperazin-1-yl)-2-methylpyrido[2,3-b]phenazine-5,12-dione was recovered as a dark purple solid (21.9 mg, 55% yield). ¹H NMR (400 MHz, Methanol-*d*₄) δ 8.70 (d, *J=* 8.1 Hz, 1H), 8.23 (d, *J=* 9.5 Hz, 1H), 8.04 (dd, *J* = 9.6, 2.8 Hz, 1H), 7.87 (d, *J* = 8.2 Hz, 1H), 7.58 (d, *J* = 2.7 Hz, 1H), 3.92 (s, 4H), 3.64 (s, 4H), 2.90 (s, 1H), 2.83 (s, 3H), 1.05 (dd, *J* = 11.1, 6.1 Hz, 4H). LCMS (ESI) 400.15 [M + H]⁺. HPLC purity at 254 nm, 99.4%.
**Example 55: *10-fluoro-9-(4-methylpiperazin-1-yl)pyrido[2,3-b]phenazine-5,12-dione* (55).** Following general protocol J. A solution of 6,7-dichloro-5,8-quinolinediones (0.1 equiv.), cerium(III) chloride heptahydrate (CeCl₃·7 H₂O, 0.11 equiv.) and 3-fluoro-4-(4-methylpiperazin-1-yl)aniline (20.9 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 10-fluoro-9-(4-methylpiperazin-1-yl)pyrido[2,3-b]phenazine-5,12-dione was recovered as a dark purple solid (7.9 mg, 21% yield). ¹H NMR (400 MHz, Methanol-*d*₄) δ 9.15 (dd, *J=* 4.8, 1.7 Hz, 1H), 8.87 (dd, *J=* 8.0, 1.7 Hz, 1H), 8.18 (dd, *J=* 9.5, 1.4 Hz, 1H), 8.10 - 7.96 (m, 2H), 4.15 (s, 2H), 3.81 - 3.44 (m, 6H), 3.07 (s, 3H). LCMS (ESI) 378.10 [M + H]⁺. HPLC purity at 254 nm, 100%.
**Example 56: *10-chloro-9-(4-methylpiperazin-1-yl)pyrido[2,3-b]phenazine-5,12-dione* (56).** Following general protocol J. A solution of 6,7-dichloro-5,8-quinolinediones (0.1 equiv.), cerium(III) chloride heptahydrate (CeCl₃·7 H₂O, 0.11 equiv.) and 3-chloro-4-(4-methylpiperazin-1-yl)aniline (22.5 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 10-chloro-9-(4-methylpiperazin-1-yl)pyrido[2,3-b]phenazine-5,12-dione was recovered as a dark purple solid (13.0 mg, 33% yield). ¹H NMR (499 MHz, Methanol-*d*₄) δ 9.14 (d, *J=* 3.3 Hz, 1H), 8.85 (dd, *J=* 7.9, 1.6 Hz, 1H), 8.30 (d, *J=* 9.3 Hz, 1H), 8.04 (d, *J* = 9.3 Hz, 1H), 8.01 (dd, *J* = 8.0, 4.7 Hz, 1H), 4.03 (s, 2H), 3.72 (s, 2H), 3.49 (s, 4H), 3.06 (s, 3H). ¹⁹F NMR (470 MHz, Methanol-*d*₄) δ -76.99. LCMS (ESI) 393.90 [M + H]⁺. HPLC purity at 254 nm, 99.0%.
**Example 57: *9-(4-(methylsulfonyl)piperazin-1-yl)pyrido[2,3-b]phenazine-5,12-dione* (57).** Following general protocol J. A solution of 6,7-dichloro-5,8-quinolinediones (0.1 equiv.), cerium(III) chloride heptahydrate (CeCl₃·7 H₂O, 0.11 equiv.) and 4-(4-(methylsulfonyl)piperazin-1-yl)aniline (25.5 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 9-(4-(methylsulfonyl)piperazin-1-yl)pyrido[2,3-b]phenazine-5,12-dione was recovered as a dark purple solid (17.3 mg, 41% yield). ¹H NMR (300 MHz, Chloroform-*d*) δ 9.24 (dd, *J* = 4.6, 1.7 Hz, 1H), 8.84 (dd, *J*= 8.0, 1.8 Hz, 1H), 8.34 (d, *J=* 9.6 Hz, 1H), 7.87 (dd, *J=* 8.0, 4.6 Hz, 1H), 7.79 (dd, *J=* 9.6, 2.9 Hz, 1H), 7.63 (d, *J=* 3.0 Hz, 1H), 3.85 - 3.68 (m, 4H), 3.56 - 3.39 (m, 4H), 2.89 (s, 3H). LCMS (ESI) 424.10 [M + H]⁺. HPLC purity at 254 nm, 99.2%.
**Example 58: *2-methyl-9-(4-(methylsulfonyl)piperazin-1-yl)pyrido[2,3-b]phenazine-5,12-dione (58).*** Following general protocol J. A solution of 6,7-dichloro-2-methylquinoline-5,8-dione (0.1 equiv, 24 mg), cerium(III) chloride heptahydrate (CeCl₃·7 H₂O, 0.11 equiv.) and 4-(4-(methylsulfonyl)piperazin-1-yl)aniline (25.5 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 2-methyl-9-(4-(methylsulfonyl)piperazin-1-yl)pyrido[2,3-b]phenazine-5,12-dione was recovered as a dark purple solid (20.1 mg, 46% yield). ¹H NMR (300 MHz, Chloroform-*d*) δ 8.69 (d, *J* = 7.7 Hz, 1H), 8.32 (d, *J=* 9.5 Hz, 1H), 7.78 (s, 1H), 7.72 - 7.62 (m, 2H), 3.72 (s, 4H), 3.49 (s, 4H), 2.88 (s, 3H), 2.86 (s, 3H). LCMS (ESI) 438.05 [M + H]⁺. HPLC purity at 254 nm, 100%.
**Example 59: *10-chloro-2-methyl-9-(4-methylpiperazin-1-yl)pyrido[2,3-b]phenazine-5,12-dione (59).*** Following general protocol J. A solution of 6,7-dichloro-2-methylquinoline-5,8-dione (0.1 equiv, 24 mg), cerium(III) chloride heptahydrate (CeCl₃·7 H₂O, 0.11 equiv.) and 3-chloro-4-(4-methylpiperazin-1-yl)aniline (22.5 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 10-chloro-2-methyl-9-(4-methylpiperazin-1-yl)pyrido[2,3-b]phenazine-5,12-dione was recovered as a dark purple solid (15.9 mg, 39% yield). ¹H NMR (300 MHz, DMSO-*d*₆) δ 8.57 (d, *J=* 8.0 Hz, 1H), 8.35 (d, *J* = 9.2 Hz, 1H), 8.09 (d, *J* = 9.4 Hz, 1H), 7.85 (d, *J* = 8.1 Hz, 1H), 3.92 (s, 4H), 3.37 (s, 4H), 2.95 (s, 3H), 2.76 (s, 3H). LCMS (ESI) 408.05 [M + H]⁺. HPLC purity at 254 nm, 95.5%.
**Example 60: *9-((4-ethylpiperazin-1-yl)methyl)-2-methylpyrido[2,3-b]phenazine-5,12-dione (60).*** Following general protocol J. A solution of 6,7-dichloro-2-methylquinoline-5,8-dione (0.1 equiv, 24 mg), cerium(III) chloride heptahydrate (CeCl₃·7 H₂O, 0.11 equiv.) and 4-((4-ethylpiperazin-1-yl)methyl)aniline (21.9 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 9-((4-ethylpiperazin-1-yl)methyl)-2-methylpyrido[2,3-b]phenazine-5,12-dione was recovered as a dark purple solid (9.6 mg, 24% yield). ¹H NMR (400 MHz, Methanol-*d*₄) δ 8.74 (d, *J* = 8.1 Hz, 1H), 8.42 (d, *J=* 8.6 Hz, 2H), 8.19 (dd, *J=* 8.9, 1.7 Hz, 1H), 7.90 (d, *J=* 8.1 Hz, 1H), 4.02 (s, 2H), 3.72 - 3.46 (m, 2H), 3.30 - 3.13 (m, 6H), 2.84 (s, 3H), 2.74 - 2.55 (m, 2H), 1.39 (t, *J=* 7.3 Hz, 3H). LCMS (ESI) 402.20 [M + H]⁺. HPLC purity at 254 nm, 99.5%.
**Example 61: *9-(4-methylpiperazine-1-carbonyl)-8-(trifluoromethyl)pyrido[2,3-b]phenazine-5,12-dione (61).*** Following general protocol J. A solution of 6,7-dichloro-5,8-quinolinediones (0.1 equiv.), cerium(III) chloride heptahydrate (CeCl₃·7 H₂O, 0.11 equiv.) and (4-amino-2-(trifluoromethyl)phenyl)(4-ethylpiperazin-1-yl)methanone (30.1 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 9-(4-methylpiperazine-1-carbonyl)-8-(trifluoromethyl) pyrido[2,3-b]phenazine-5,12-dione was recovered as a dark purple solid (10.0 mg, 22% yield). ¹H NMR (300 MHz, Methanol-*d*₄) δ 9.19 (dd, *J* = 4.6, 1.7 Hz, 1H), 8.99 - 8.85 (m, 2H), 8.64 (s, 1H), 8.06 (dd, *J* = 8.0, 4.7 Hz, 1H), 3.67 (d, *J=* 74.0 Hz, 8H), 3.03 (s, 3H). LCMS (ESI) 456.05 [M + H]⁺. HPLC purity at 254 nm, 98.8%.
**Example 62: *9-morpholino-10-(trifluoromethyl)pyrido[2,3-b]phenazine-5,12-dione (62).*** Following general protocol J. A solution of 6,7-dichloro-5,8-quinolinediones (0.1 equiv.), cerium(III) chloride heptahydrate (CeCl₃·7 H₂O, 0.11 equiv.) and 4-morpholino-3-(trifluoromethyl)aniline (24.6 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 9-morpholino-10-(trifluoromethyl)pyrido[2,3-b]phenazine-5,12-dione was recovered as a dark purple solid (12 mg, 29% yield). ¹H NMR (300 MHz, Chloroform-*d*) δ 9.25 (d, *J=* 4.6 Hz, 1H), 8.83 (dd, *J=* 8.0, 1.7 Hz, 1H), 8.42 (d, *J=* 9.6 Hz, 1H), 7.95 - 7.79 (m, 2H), 4.01 - 3.89 (m, 4H), 3.58 (d, *J=* 5.1 Hz, 4H). LCMS (ESI) 415.00 [M + H]⁺. HPLC purity at 254 nm, 99.7%.
**Example 63: *9-morpholino-8-(trifluoromethyl)pyrido[2,3-b]phenazine-5,12-dione (63).*** Following general protocol J. A solution of 6,7-dichloro-5,8-quinolinediones (0.1 equiv.), cerium(III) chloride heptahydrate (CeCl₃·7 H₂O, 0.11 equiv.) and 4-morpholino-3-(trifluoromethyl)aniline (24.6 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 9-morpholino-8-(trifluoromethyl)pyrido[2,3-b]phenazine-5,12-dione was recovered as a dark purple solid (7.9 mg, 19% yield). ¹H NMR (300 MHz, Chloroform-*d*) δ 9.30 (d, *J=* 2.7 Hz, 1H), 8.93 - 8.83 (m, 2H), 8.19 (s, 1H), 7.93 (dd, *J* = 8.0, 4.6 Hz, 1H), 4.05 - 3.90 (m, 4H), 3.34 - 3.20 (m, 4H). LCMS (ESI) 414.95 [M + H]⁺. HPLC purity at 254 nm, 98.7%.
**Example 64: *9-(4-methylpiperidin-1-yl)pyrido[2,3-b]phenazine-5,12-dione (64).*** Following general protocol J. A solution of 6,7-dichloro-5,8-quinolinediones (0.1 equiv.), cerium(III) chloride heptahydrate (CeCl₃·7 H₂O, 0.11 equiv.) and 4-(4-methylpiperidin-1-yl)aniline (19.0 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 9-(4-methylpiperidin-1-yl)pyrido[2,3-b]phenazine-5,12-dione was recovered as a dark purple solid (19.3 mg, 54% yield). ¹H NMR (400 MHz, Methanol-*d*₄) δ 9.10 (s, 1H), 8.82 (d, *J=* 7.7 Hz, 1H), 8.13 (d, *J=* 9.6 Hz, 1H), 8.00 (d, *J* = 9.3 Hz, 2H), 7.43 (d, *J* = 2.7 Hz, 1H), 4.29 (d, *J* = 13.2 Hz, 2H), 3.27 - 3.11 (m, 2H), 1.90 (d, *J=* 13.6 Hz, 2H), 1.45 - 1.27 (m, 3H), 1.04 (d, J= 6.4 Hz, 3H). LCMS (ESI) 359.00 [M + H]⁺. HPLC purity at 254 nm, 100%.
**Example 65: *9-(4-methylpiperazin-1-yl)-7-(trifluoromethyl)pyrido[2,3-b]phenazine-5,12-dione (65).*** Following general protocol J. A solution of 6,7-dichloro-5,8-quinolinediones (0.1 equiv.), cerium(III) chloride heptahydrate (CeCl₃·7 H₂O, 0.11 equiv.) and 4-(4-methylpiperazin-1-yl)-2-(trifluoromethyl)aniline (25.8 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 9-(4-methylpiperazin-1-yl)-7-(trifluoromethyl)pyrido[2,3-b]phenazine-5,12-dione was recovered as a dark purple solid (20.9 mg, 49% yield). ¹H NMR (400 MHz, Methanol-*d*₄) δ 9.13 (dd, *J* = 4.6, 1.7 Hz, 1H), 8.90 - 8.77 (m, 1H), 8.34 (s, 1H), 8.02 (dd, *J* = 7.9, 4.6 Hz, 1H), 7.70 (s, 1H), 3.61 (s, 8H), 3.12 - 3.01 (m, 2H). LCMS (ESI) 428.00 [M + H]⁺. HPLC purity at 254 nm, 99.8%.
**Example 66: *9-morpholino-7-(trifluoromethyl)pyrido[2,3-b]phenazine-5,12-dione (66).*** Following general protocol J. A solution of 6,7-dichloro-5,8-quinolinediones (0.1 equiv.), cerium(III) chloride heptahydrate (CeCl₃·7 H₂O, 0.11 equiv.) and 4-morpholino-2-(trifluoromethyl)aniline (24.6 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture was stirred at 90 °C for 2 h. The reaction mixture was chilled, the filtered precipitate was extracted with methylene chloride and concentrated, and then the residue was purified by column chromatography, 9-morpholino-7-(trifluoromethyl)pyrido[2,3-b]phenazine-5,12-dione was recovered as a dark purple solid (16.6 mg, 40% yield). ¹H NMR (400 MHz, Methanol-*d*₄) δ 9.12 (s, 1H), 8.84 (d, *J=* 7.9 Hz, 1H), 8.31 (d, *J=* 2.4 Hz, 1H), 8.00 (dd, *J* = 8.1, 4.1 Hz, 1H), 7.66 (d, *J=* 2.8 Hz, 1H), 4.01 - 3.91 (m, 2H), 3.88 (dt, *J* = 10.6, 4.7 Hz, 2H), 3.74 (t, *J* = 5.7 Hz, 1H), 3.66 (t, *J* = 4.9 Hz, 2H), 3.45 - 3.38 (m, 1H). LCMS (ESI) 415.05 [M + H]⁺. HPLC purity at 254 nm, 100%.
**Example 68: *9-(4-(tert-butyl)piperazin-1-yl)pyrido[2,3-b]phenazine-5,12-dione (68).*** Following general protocol J. A solution of 6,7-dichloro-5,8-quinolinediones (0.1 equiv.), cerium(III) chloride heptahydrate (CeCl₃·7 H₂O, 0.11 equiv.) and 4-(4-(tert-butyl)piperazin-1-yl)aniline (23.3 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 9-(4-(tert-butyl)piperazin-1-yl)pyrido[2,3-b]phenazine-5,12-dione was recovered as a dark purple solid (12 mg, 30% yield). ¹H NMR (400 MHz, Methanol-*d*₄) δ 9.14 (dd, *J=* 4.7, 1.7 Hz, 1H), 8.85 (dd, *J=* 8.0, 1.7 Hz, 1H), 8.24 (d, *J=* 9.5 Hz, 1H), 8.10 - 7.97 (m, 2H), 7.57 (d, *J* = 2.8 Hz, 1H), 4.50 (d, *J* = 21.8 Hz, 2H), 3.81 (d, *J=* 32.5 Hz, 2H), 3.61 - 3.40 (m, 4H), 1.55 (s, 9H). LCMS (ESI) 402.20 [M + H]⁺. HPLC purity at 254 nm, 98.4%.
**Example 69: *9-(4-(4-methoxyphenyl)piperazin-1-yl)pyrido[2,3-b]phenazine-5,12-dione* (69).** Following general protocol J. A solution of 6,7-dichloro-5,8-quinolinediones (0.1 equiv.), cerium(III) chloride heptahydrate (CeCl₃·7 H₂O, 0.11 equiv.) and 4-(4-(4-methoxyphenyl)piperazin-1-yl)aniline (28.3 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 9-(4-(4-methoxyphenyl)piperazin-1-yl)pyrido[2,3-b]phenazine-5,12-dione was recovered as a dark purple solid (22.0 mg, 49% yield). ¹H NMR (400 MHz, Chloroform-*d*) δ 9.20 (dd, *J* = 4.6, 1.7 Hz, 1H), 8.81 (dd, *J*= 7.9, 1.7 Hz, 1H), 8.29 (d, *J* = 9.6 Hz, 1H), 7.92 - 7.78 (m, 2H), 7.61 (d, *J* = 2.9 Hz, 1H), 7.03 - 6.92 (m, 2H), 6.92 - 6.82 (m, 2H), 3.79 (s, 3H), 3.78 - 3.73 (m, 4H), 3.29 (dd, *J=* 6.4, 3.8 Hz, 4H). LCMS (ESI) 451.90 [M + H]⁺. HPLC purity at 254 nm, 99.3%.
**Example 70: *9-((3aS,5S,7aS)-octahydro-7aH-2,5-methanoinden-7a-yl)pyrido[2,3-bJphenazine-5,12-dione (70).*** Following general protocol J. A solution of 6,7-dichloro-5,8-quinolinediones (0.1 equiv.), cerium(III) chloride heptahydrate (CeCl₃·7 H₂O, 0.11 equiv.) and 4-((3aS,5S,7aS)-octahydro-7aH-2,5-methanoinden-7a-yl)aniline (22.7 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 9-((3aS,5S,7aS)-octahydro-7aH-2,5-methanoinden-7a-yl)pyrido[2,3-b]phenazine-5,12-dione was recovered as a dark purple solid (24.1 mg, 61% yield). ¹H NMR (400 MHz, Chloroform-*d*) δ 9.26 (s, 1H), 8.86 (d, *J=* 7.9 Hz, 1H), 8.53 - 8.37 (m, 2H), 8.20 (dd, *J=* 9.0, 2.2 Hz, 1H), 7.89 (dd, *J=* 7.8, 4.3 Hz, 1H), 2.21 (s, 3H), 2.15 (s, 2H), 2.07 (d, *J=* 2.9 Hz, 4H), 1.85 (q, *J=* 12.6 Hz, 6H). LCMS (ESI) 396.25 [M + H]⁺. HPLC purity at 254 nm, 95.8%.
**Example 71: *9-(thiazol-2-yl)pyrido[2,3-b]phenazine-5,12-dione* (71).** Following general protocol J. A solution of 6,7-dichloro-5,8-quinolinediones (0.1 equiv.), cerium(III) chloride heptahydrate (CeCl₃·7 H₂O, 0.11 equiv.) and 4-(thiazol-2-yl)aniline (17.6 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 9-(thiazol-2-yl)pyrido[2,3-b]phenazine-5,12-dione was recovered as a dark purple solid (22.7 mg, 66% yield). ¹H NMR (400 MHz, Chloroform-*d*) δ 9.26 (dd, *J* = 4.6, 1.8 Hz, 1H), 9.01 (d, *J* = 2.0 Hz, 1H), 8.83 (ddd, *J* = 15.7, 8.5, 1.9 Hz, 2H), 8.55 (d, *J* = 8.9 Hz, 1H), 8.04 (d, *J* = 3.2 Hz, 1H), 7.88 (dd, *J=* 8.0, 4.5 Hz, 1H), 7.56 (d, *J=* 3.2 Hz, 1H). LCMS (ESI) 344.95 [M + H]⁺. HPLC purity at 254 nm, 100%.
**Example 72: *5,12-dioxo-N,N-dipropyl-5,12-dihydropyrido[2,3-b]phenazine-9-sulfonamide* (72).** Following general protocol J. A solution of 6,7-dichloro-5,8-quinolinediones (0.1 equiv.), cerium(III) chloride heptahydrate (CeCl₃·7 H₂O, 0.11 equiv.) and 4-amino-*N*,*N*-dipropylbenzenesulfonamide (25.6 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 5,12-dioxo-N,N-dipropyl-5,12-dihydropyrido[2,3-b]phenazine-9-sulfonamide was recovered as a dark purple solid (21.6 mg, 51% yield). ¹H NMR (400 MHz, Chloroform-*d*) δ 9.30 (dd, *J=* 4.6, 1.8 Hz, 1H), 9.01 (dd, J= 2.0, 0.6 Hz, 1H), 8.88 (dd, *J=* 8.0, 1.7 Hz, 1H), 8.63 (dd, *J* = 9.0, 0.6 Hz, 1H), 8.38 (dd, *J* = 8.9, 2.0 Hz, 1H), 7.92 (dd, *J* = 8.0, 4.6 Hz, 1H), 3.31 - 3.17 (m, 4H), 1.70 - 1.53 (m, 4H), 0.90 (t, *J* = 7.4 Hz, 6H). LCMS (ESI) 424.95 [M + H]⁺. HPLC purity at 254 nm, 96.8%.
**Example 73: *9-cyclopropylpyrido[2,3-b]phenazine-5,12-dione* (73).** Following general protocol J. A solution of 6,7-dichloro-5,8-quinolinediones (0.1 equiv.), cerium(III) chloride heptahydrate (CeCl₃·7 H₂O, 0.11 equiv.) and 4-cyclopropylaniline (13.3 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 9-cyclopropylpyrido[2,3-b]phenazine-5,12-dione was recovered as a dark purple solid (21.0 mg, 70% yield). ¹H NMR (400 MHz, Chloroform-*d*) δ 9.25 (dd, *J* = 4.6, 1.8 Hz, 1H), 8.85 (dd, *J* = 8.0, 1.7 Hz, 1H), 8.38 (d, *J* = 8.9 Hz, 1H), 8.13 (d, *J* = 2.0 Hz, 1H), 7.87 (dd, *J* = 8.0, 4.5 Hz, 1H), 7.79 (dd, *J* = 8.9, 2.0 Hz, 1H), 2.22 (tt, *J* = 8.2, 5.0 Hz, 1H), 1.37 - 1.24 (m, 2H), 1.12 - 0.94 (m, 2H). LCMS (ESI) 302.00 [M + H]⁺. HPLC purity at 254 nm, 95.9%.
**Example 74: *9-cyclohexylpyrido[2,3-b]phenazine-5,12-dione* (74)**. Following general protocol J. A solution of 6,7-dichloro-5,8-quinolinediones (0.1 equiv.), cerium(III) chloride heptahydrate (CeCl₃·7 H₂O, 0.11 equiv.) and 4-cyclohexylaniline (17.5 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 9-cyclohexylpyrido[2,3-b]phenazine-5,12-dione was recovered as a dark purple solid (23.3 mg, 68% yield). ¹H NMR (400 MHz, Chloroform-*d*) δ 9.25 (dd, *J* = 4.6, 1.8 Hz, 1H), 8.85 (dd, *J* = 7.9, 1.7 Hz, 1H), 8.41 (d, *J* = 8.8 Hz, 1H), 8.34 (dt, *J* = 2.1, 0.7 Hz, 1H), 7.96 (dd, *J* = 9.0, 1.9 Hz, 1H), 7.87 (dd, *J* = 7.9, 4.5 Hz, 1H), 2.96 - 2.75 (m, 1H), 2.07 (d, *J* = 12.0 Hz, 2H), 1.95 (d, *J=* 12.5 Hz, 2H), 1.84 (dd, *J=* 12.9, 3.1 Hz, 1H), 1.63 - 1.43 (m, 4H), 1.40 - 1.27 (m, 1H). LCMS (ESI) 344.05 [M + H]⁺. HPLC purity at 254 nm, 100%.
**Example 75: *9-((2-(diethylamino)ethyl)amino)pyrido[2,3-b]phenazine-5,12-dione (75).*** Following general protocol J. A solution of 6,7-dichloro-5,8-quinolinediones (0.1 equiv.), cerium(III) chloride heptahydrate (CeCl₃·7 H₂O, 0.11 equiv.) and *N*¹-(2-(diethylamino)ethyl)benzene-1,4-diamine (20.7 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 9-((2-(diethylamino)ethyl)amino)pyrido[2,3-b]phenazine-5,12-dione was recovered as a dark purple solid (7.1 mg, 19% yield). ¹H NMR (400 MHz, Methanol-*d*₄) δ 9.18 - 9.08 (m, 1H), 8.84 (dd, *J* = 8.0, 1.7 Hz, 1H), 8.14 (d, *J* = 9.3 Hz, 1H), 8.01 (dd, *J* = 8.0, 4.7 Hz, 1H), 7.61 (dd, *J* = 9.3, 2.6 Hz, 1H), 7.24 (d, *J* = 2.6 Hz, 1H), 3.86 (t, *J* = 6.6 Hz, 4H), 3.55 (t, *J* = 6.6 Hz, 2H), 3.45 - 3.37 (m, 4H), 1.41 (t, *J=* 7.3 Hz, 6H). LCMS (ESI) 376.10 [M + H]⁺. HPLC purity at 254 nm, 97.5%.
**Example 78: *9-(1-methylpiperidin-4-yl)pyrido[2,3-b]phenazine-5,12-dione (78).*** Following general protocol J. A solution of 6,7-dichloro-5,8-quinolinediones (0.1 equiv.), cerium(III) chloride heptahydrate (CeCl₃·7 H₂O, 0.11 equiv.) and 4-(1-methylpiperidin-4-yl)aniline (19.1 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 9-(1-methylpiperidin-4-yl)pyrido[2,3-b]phenazine-5,12-dione was recovered as a dark purple solid (16.5 mg, 46% yield). ¹H NMR (400 MHz, DMSO-*d*₆) δ 9.17 (dd, *J* = 4.6, 1.7 Hz, 1H), 8.70 (dd, *J* = 7.9, 1.7 Hz, 1H), 8.42 (d, *J=* 8.8 Hz, 1H), 8.23 (d, *J=* 2.0 Hz, 1H), 8.08 (dd, *J=* 8.8, 2.0 Hz, 1H), 7.99 (dd, *J=* 7.9, 4.6 Hz, 1H), 3.63 (d, *J=* 12.0 Hz, 2H), 3.58 - 3.39 (m, 3H), 2.88 (s, 3H), 2.26 (d, *J* = 13.8 Hz, 2H), 2.18 - 2.03 (m, 2H). LCMS (ESI) 359.15 [M + H]⁺. HPLC purity at 254 nm, 98.5%.
**Example 79: *9-(4-methyl-1,4-diazepan-1-yl)pyrido[2,3-b]phenazine-5,12-dione (79).*** Following general protocol J. A solution of 6,7-dichloro-5,8-quinolinediones (0.1 equiv.), cerium(III) chloride heptahydrate (CeCl₃·7 H₂O, 0.11 equiv.) and 4-(4-methyl-1,4-diazepan-1-yl)aniline (20.5 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 9-(4-methyl-1,4-diazepan-1-yl)pyrido[2,3-b]phenazine-5,12-dione was recovered as a dark purple solid (10.8 mg, 29% yield). ¹H NMR (400 MHz, Methanol-*d*₄) δ 9.13 (dd, *J=* 4.7, 1.7 Hz, 1H), 8.83 (dd, *J=* 8.0, 1.7 Hz, 1H), 8.14 (d, *J=* 9.6 Hz, 1H), 8.01 (dd, *J=* 8.0, 4.7 Hz, 1H), 7.89 (dd, *J=* 9.6, 2.9 Hz, 1H), 7.34 (d, *J* = 2.8 Hz, 1H), 4.31 - 3.41 (m, 10H), 3.06 (s, 3H). LCMS (ESI) 374.15 [M + H]⁺. HPLC purity at 254 nm, 97.4%.
**Example 80: *9-(pyrrolidin-1-yl)-10-(trifluoromethyl)pyrido[2,3-b]phenazine-5,12-dione (80).*** Following general protocol J. A solution of 6,7-dichloro-5,8-quinolinediones (0.1 equiv.), cerium(III) chloride heptahydrate (CeCl₃·7 H₂O, 0.11 equiv.) and 4-(pyrrolidin-1-yl)-3-(trifluoromethyl)aniline (23.0 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 9-(pyrrolidin-1-yl)-10-(trifluoromethyl)pyrido[2,3-b]phenazine-5,12-dione was recovered as a dark purple solid (7.2 mg, 18% yield). ¹H NMR (400 MHz, Methanol-*d*₄) δ 9.11 (d, *J* = 4.5 Hz, 1H), 8.81 (dd, *J* = 7.9, 1.7 Hz, 1H), 8.12 (d, *J=* 9.8 Hz, 1H), 8.03 - 7.91 (m, 2H), 3.76 (s, 4H), 2.14 - 2.03 (m, 4H). LCMS (ESI) 399.00 [M + H]⁺. HPLC purity at 254 nm, 99.2%.
**Example 81: *9-(pyrrolidin-1-yl)-8-(trifluoromethyl)pyrido[2,3-b]phenazine-5,12-dione* (81).** Following general protocol J. A solution of 6,7-dichloro-5,8-quinolinediones (0.1 equiv.), cerium(III) chloride heptahydrate (CeCl₃·7 H₂O, 0.11 equiv.) and 4-(pyrrolidin-1-yl)-3-(trifluoromethyl)aniline (23.0 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 9-(pyrrolidin-1-yl)-8-(trifluoromethyl)pyrido[2,3-b]phenazine-5,12-dione was recovered as a dark purple solid (6.0 mg, 15% yield). ¹H NMR (400 MHz, Methanol-*d*₄) δ 9.12 (dd, *J=* 4.7, 1.7 Hz, 1H), 8.84 (dd, *J* = 8.0, 1.7 Hz, 1H), 8.61 (s, 1H), 8.01 (dd, *J* = 8.0, 4.7 Hz, 1H), 7.57 (s, 1H), 3.70 (t, *J* = 6.3 Hz, 4H), 2.23 - 2.08 (m, 4H). LCMS (ESI) 399.00 [M + H]⁺. HPLC purity at 254 nm, 98.4%.
**Example 82: *10-fluoro-9-(4-methylpiperidin-1-yl)pyrido[2,3-b]phenazine-5,12-dione* (82).** Following general protocol J. A solution of 6,7-dichloro-5,8-quinolinediones (0.1 equiv.), cerium(III) chloride heptahydrate (CeCl₃·7 H₂O, 0.11 equiv.) and 3-fluoro-4-(4-methylpiperidin-1-yl)aniline (20.8 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 10-fluoro-9-(4-methylpiperidin-1-yl)pyrido[2,3-b]phenazine-5,12-dione was recovered as a dark purple solid (13.1 mg, 35% yield). ¹H NMR (400 MHz, Methanol-*d*₄) δ 9.12 (dd, *J* = 4.6, 1.7 Hz, 1H), 8.85 (dd, *J* = 8.0, 1.7 Hz, 1H), 8.12 - 8.06 (m, 1H), 8.04 - 7.93 (m, 2H), 4.09 (d, *J=* 12.8 Hz, 2H), 3.24 (t, *J=* 12.6 Hz, 2H), 1.88 (d, *J* = 13.1 Hz, 2H), 1.75 (s, 1H), 1.52 - 1.41 (m, 2H), 1.06 (d, *J* = 6.5 Hz, 3H). LCMS (ESI) 377.15 [M + H]⁺. HPLC purity at 254 nm, 98.5%.
**Example 83: *8-fluoro-9-(4-methylpiperidin-1-yl)pyrido[2,3-b]phenazine-5,12-dione (83).*** Following general protocol J. A solution of 6,7-dichloro-5,8-quinolinediones (0.1 equiv.), cerium(III) chloride heptahydrate (CeCl₃·7 H₂O, 0.11 equiv.) and 3-fluoro-4-(4-methylpiperidin-1-yl)aniline (20.8 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 8-fluoro-9-(4-methylpiperidin-1-yl)pyrido[2,3-b]phenazine-5,12-dione was recovered as a dark purple solid (0.98 mg, 26% yield). ¹H NMR (400 MHz, Methanol-*d*₄) δ 9.12 (dd, *J* = 4.7, 1.7 Hz, 1H), 8.82 (dd, *J* = 7.9, 1.7 Hz, 1H), 8.00 (dd, *J=* 7.9, 4.6 Hz, 1H), 7.88 (s, 1H), 7.57 (s, 1H), 3.86 (d, *J=* 12.3 Hz, 2H), 3.03 - 2.80 (m, 2H), 1.86 (d, *J* = 12.9 Hz, 2H), 1.75 - 1.61 (m, 1H), 1.49 (qd, *J=* 12.3, 3.8 Hz, 2H), 1.07 (d, *J* = 6.5 Hz, 3H). LCMS (ESI) 377.15 [M + H]⁺. HPLC purity at 254 nm, 98.8%.
**Example 84: *9-(4-acetylpiperazin-1-yl)-10-(trifluoromethyl)pyrido[2,3-b]phenazine-5,12-dione (84).*** Following general protocol J. A solution of 6,7-dichloro-5,8-quinolinediones (0.1 equiv.), cerium(III) chloride heptahydrate (CeCl₃·7 H₂O, 0.11 equiv.) and 1-(4-(4-amino-2-(trifluoromethyl)phenyl)piperazin-1-yl)ethan-1-one (28.7 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 9-(4-acetylpiperazin-1-yl)-10-(trifluoromethyl)pyrido[2,3-b]phenazine-5,12-dione was recovered as a dark purple solid (22.3 mg, 49% yield). ¹H NMR (400 MHz, Chloroform-d) δ 9.28 (dd, J = 4.6, 1.8 Hz, 1H), 8.85 (dd, J = 8.0, 1.8 Hz, 1H), 8.46 (d, J = 9.6 Hz, 1H), 7.93 - 7.82 (m, 2H), 3.92 (s, 2H), 3.79 - 3.73 (m, 2H), 3.56 (dd, J = 11.6, 6.9 Hz, 2H), 2.23 (s, 3H). LCMS (ESI) 456.10 [M + H]⁺. HPLC purity at 254 nm, 99.0%.
**Example 85: *9-(4-acetylpiperazin-1-yl)-8-(trifluoromethyl)pyrido[2,3-b]phenazine-5,12-dione (85).*** Following general protocol J. A solution of 6,7-dichloro-5,8-quinolinediones (0.1 equiv.), cerium(III) chloride heptahydrate (CeCl₃·7 H₂O, 0.11 equiv.) and 1-(4-(4-amino-2-(trifluoromethyl) phenyl) piperazin-1-yl)ethan-1-one (28.7 mg, 0.1 mmol) in ethanol (2 mL) was stirred at 60-80 °C for 2 h. Next, most of the ethanol was removed under vacuum. Then DMF (2 mL), H₂O (10 µL) and sodium azide (13 mg, 0.2 mmol) were added to above reaction system. The mixture solution was stirred at 90 °C for 2 h. The reaction mixture was chilled, the filtered precipitate was extracted with methylene chloride and concentrated, and then the residue was purified by column chromatography, 9-(4-acetylpiperazin-1-yl)-8-(trifluoromethyl) pyrido[2,3-b]phenazine-5,12-dione was recovered as a dark purple solid (21.0 mg, 46% yield). ¹H NMR (400 MHz, Methanol-d4) δ 9.16 (dd, J = 4.7, 1.7 Hz, 1H), 8.89 (dd, J = 8.0, 1.7 Hz, 1H), 8.78 (s, 1H), 8.29 (s, 1H), 8.04 (dd, J = 8.0, 4.7 Hz, 1H), 3.86-3.79 (m, 4H), 3.31-3.22 (m, 4H), 2.22 (s, 3H). LCMS (ESI) 456.10 [M + H]⁺. HPLC purity at 254 nm, 99.8%.

### Example III.

Representative compounds were tested in a panel of 60 cancer cells (NCI 60) at the National Cancer Institute. Growth inhibition are shown in table 2, table 3, table 4, table 5, and Fig. 1, Fig. 2, Fig. 3, Fig. 4, Fig. 5.

**Table 2. Growth inhibition (% control) of compound 1 in the NCI60 cell lines.**

| 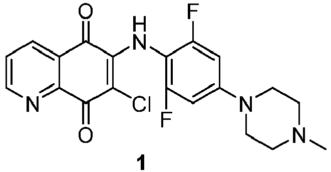 | | | | | |
|---|---|---|---|---|---|
| **PANELNME** | **CELLNAME** | **GIPRCNT** | **PANELNME** | **CELLNAME** | **GIPRCNT** |
| Leukemia | CCRF-CEM | -18.2389 | Melanoma | LOX IMVI | -70.2674 |
| Leukemia | HL-60(TB) | -26.4294 | Melanoma | MALME-3M | -96.0656 |
| Leukemia | K-562 | -0.83445 | Melanoma | M14 | -40.5376 |
| Leukemia | MOLT-4 | -22.6883 | Melanoma | MDA-MB-435 | -75.7981 |
| Leukemia | RPMI-8226 | -20.7257 | Melanoma | SK-MEL-2 | -49.6035 |
| Leukemia | SR | -0.81342 | Melanoma | SK-MEL-28 | -55.0718 |
| Non-Small Cell Lung Cancer | A549/ATCC | 74.56334 | Melanoma | SK-MEL-5 | -91.7268 |
| Non-Small Cell Lung Cancer | EKVX | -82.6636 | Melanoma | UACC-257 | -62.2412 |
| Non-Small Cell Lung Cancer | HOP-62 | 83.28052 | Melanoma | UACC-62 | -45.6278 |
| Non-Small Cell Lung Cancer | HOP-92 | -79.9407 | Ovarian Cancer | IGROV1 | -90.4429 |
| Non-Small Cell Lung Cancer | NCI-H226 | 85.31879 | Ovarian Cancer | OVCAR-3 | -98.2241 |
| Non-Small Cell Lung Cancer | NCI-H23 | -61.2055 | Ovarian Cancer | OVCAR-4 | -99.2407 |
| Non-Small Cell Lung Cancer | NCI-H322M | 101.8249 | Ovarian Cancer | OVCAR-5 | -59.8079 |
| Non-Small Cell Lung Cancer | NCI-H460 | 9.912259 | Ovarian Cancer | OVCAR-8 | 1.948805 |
| Non-Small Cell Lung Cancer | NCI-H522 | -59.746 | Ovarian Cancer | NCI/ADR-RES | 37.87226 |
| Colon Cancer | COLO 205 | 33.97826 | Ovarian Cancer | SK-OV-3 | 82.66932 |
| Colon Cancer | HCC-2998 | 61.85349 | Renal Cancer | 786-0 | -72.0362 |
| Colon Cancer | HCT-116 | -69.7462 | Renal Cancer | A498 | 80.11417 |
| Colon Cancer | HCT-15 | -8.53119 | Renal Cancer | ACHN | -93.058 |
| Colon Cancer | HT29 | 11.1525 | Renal Cancer | CAKI-1 | -85.6094 |
| Colon Cancer | KM12 | 82.36567 | Renal Cancer | SN12C | -97.4828 |
| Colon Cancer | SW-620 | -76.6429 | Renal Cancer | TK-10 | 8.46166 |
| CNS Cancer | SF-268 | 48.73363 | Renal Cancer | UO-31 | -100 |
| CNS Cancer | SF-295 | 78.81483 | Prostate Cancer | PC-3 | 11.82948 |
| CNS Cancer | SF-539 | -25.0889 | Prostate Cancer | DU-145 | -67.5681 |
| CNS Cancer | SNB-19 | 96.33177 | Breast Cancer | MCF7 | -58.1831 |
| CNS Cancer | SNB-75 | 38.01025 | Breast Cancer | MDA-MB-231/ATCC | -31.9795 |
| CNS Cancer | U251 | -9.87723 | Breast Cancer | HS 578T | -16.4793 |
| | | | Breast Cancer | BT-549 | 80.51756 |
| | | | Breast Cancer | T-47D | -32.5382 |
| | | | Breast Cancer | MDA-MB-468 | -73.911 |

**Table 3. Growth inhibition (% control) of compound 13 in the NCI60 cell lines.**

| 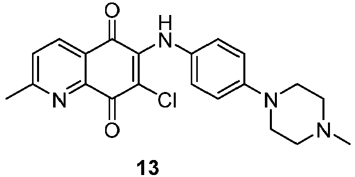 | | | | | |
|---|---|---|---|---|---|
| PANELNME | CELLNAME | GIPRCNT | PANELNME | CELLNAME | GIPRCNT |
| Leukemia | CCRF-CEM | -6.23727 | Melanoma | LOX IMVI | -95.4404 |
| Leukemia | HL-60(TB) | -46.6759 | Melanoma | MALME-3M | -75.4881 |
| Leukemia | K-562 | -12.2778 | Melanoma | M14 | 73.82868 |
| Leukemia | MOLT-4 | -25.588 | Melanoma | MDA-MB-435 | -99.1118 |
| Leukemia | RPMI-8226 | -32.551 | Melanoma | SK-MEL-2 | 68.35914 |
| Leukemia | SR | -5.05435 | Melanoma | SK-MEL-28 | 30.32254 |
| Non-Small Cell Lung Cancer | A549/ATCC | 97.63702 | Melanoma | SK-MEL-5 | 10.38648 |
| Non-Small Cell Lung Cancer | EKVX | -81.7903 | Melanoma | UACC-257 | 57.23161 |
| Non-Small Cell Lung Cancer | HOP-62 | 63.65498 | Melanoma | UACC-62 | -49.3593 |
| Non-Small Cell Lung Cancer | HOP-92 | -86.301 | Ovarian Cancer | IGROV1 | -83.5269 |
| Non-Small Cell Lung Cancer | NCI-H226 | 105.4813 | Ovarian Cancer | OVCAR-3 | -100 |
| Non-Small Cell Lung Cancer | NCI-H23 | 16.65413 | Ovarian Cancer | OVCAR-4 | -100 |
| Non-Small Cell Lung Cancer | NCI-H322M | 97.34827 | Ovarian Cancer | OVCAR-5 | -7.54758 |
| Non-Small Cell Lung Cancer | NCI-H460 | 97.22202 | Ovarian Cancer | OVCAR-8 | -62.572 |
| Non-Small Cell Lung Cancer | NCI-H522 | -63.7561 | Ovarian Cancer | NCI/ADR-RES | 27.60809 |
| Colon Cancer | COLO 205 | 82.4617 | Ovarian Cancer | SK-OV-3 | 89.88163 |
| Colon Cancer | HCC-2998 | 95.31216 | Renal Cancer | 786-0 | 52.17685 |
| Colon Cancer | HCT-116 | -64.939 | Renal Cancer | A498 | 100.8258 |
| Colon Cancer | HCT-15 | 70.43851 | Renal Cancer | ACHN | -93.75 |
| Colon Cancer | HT29 | 66.44319 | Renal Cancer | CAKI-1 | -100 |
| Colon Cancer | KM12 | 92.08078 | Renal Cancer | RXF 393 | 62.77679 |
| Colon Cancer | SW-620 | -69.7878 | Renal Cancer | SN12C | -88.5465 |
| CNS Cancer | SF-268 | 77.71986 | Renal Cancer | TK-10 | 100.0838 |
| CNS Cancer | SF-295 | 99.36131 | Renal Cancer | UO-31 | -97.3532 |
| CNS Cancer | SF-539 | -24.5048 | Prostate Cancer | PC-3 | 68.66255 |
| CNS Cancer | SNB-19 | 94.60122 | Prostate Cancer | DU-145 | -18.6864 |
| CNS Cancer | SNB-75 | -53.8363 | Breast Cancer | MCF7 | -61.9194 |
| CNS Cancer | U251 | 8.46619 | Breast Cancer | MDA-MB- | -62.7773 |
| | | | Breast Cancer | HS 578T | 58.00374 |
| | | | Breast Cancer | T-47D | -61.1209 |
| | | | Breast Cancer | MDA-MB-468 | -84.6413 |

**Table 4. Growth inhibition (% control) of compound 49 in the NCI60 cell lines.**

| 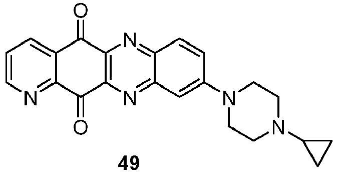 | | | | | |
|---|---|---|---|---|---|
| PANELNME | CELLNAME | GIPRCNT | PANELNME | CELLNAME | GIPRCNT |
| Leukemia | CCRF-CEM | 26.16653365 | Melanoma | LOX IMVI | -46.5487 |
| Leukemia | HL-60(TB) | -43.08739255 | Melanoma | MALME-3M | -100 |
| Leukemia | K-562 | 0.865850512 | Melanoma | M14 | -91.1652 |
| Leukemia | MOLT-4 | 26.1993216 | Melanoma | MDA-MB-435 | -87.4148 |
| Leukemia | RPMI-8226 | 25.33047339 | Melanoma | SK-MEL-2 | -16.262 |
| Leukemia | SR | -10.03125 | Melanoma | SK-MEL-28 | -76.8851 |
| Non-Small Cell Lung | A549/ATCC | -53.26612903 | Melanoma | SK-MEL-5 | -98.1218 |
| Non-Small Cell Lung | EKVX | 27.36687168 | Melanoma | UACC-257 | -84.4508 |
| Non-Small Cell Lung | HOP-62 | -56.63533835 | Melanoma | UACC-62 | -98.5184 |
| Non-Small Cell Lung | HOP-92 | 62.4576734 | Ovarian Cancer | IGROV1 | -13.7639 |
| Non-Small Cell Lung | NCI-H226 | -49.1 | Ovarian Cancer | OVCAR-3 | -1.5625 |
| Non-Small Cell Lung | NCI-H23 | -56.52460457 | Ovarian Cancer | OVCAR-4 | -87.768 |
| Non-Small Cell Lung | NCI-H322M | -96.67388614 | Ovarian Cancer | OVCAR-5 | -82.2933 |
| Non-Small Cell Lung | NCI-H460 | -54.12162162 | Ovarian Cancer | OVCAR-8 | -15.1765 |
| Non-Small Cell Lung | NCI-H522 | -50.54491413 | Ovarian Cancer | NCI/ADR-RES | 20.14936 |
| Colon Cancer | COLO 205 | -68.33964646 | Ovarian Cancer | SK-OV-3 | -78.6244 |
| Colon Cancer | HCC-2998 | -80.83237327 | Renal Cancer | 786-0 | 28.12127 |
| Colon Cancer | HCT-116 | 0.850852918 | Renal Cancer | A498 | -88.1317 |
| Colon Cancer | HCT-15 | -58.80584192 | Renal Cancer | ACHN | -35.0589 |
| Colon Cancer | HT29 | -21.30829016 | Renal Cancer | CAKI-1 | -5.26768 |
| Colon Cancer | KM12 | 9.272487374 | Renal Cancer | SN12C | -94.7287 |
| Colon Cancer | SW-620 | -37.75201613 | Renal Cancer | TK-10 | 0.122365 |
| CNS Cancer | SF-268 | 12.4597104 | Renal Cancer | UO-31 | -99.9594 |
| CNS Cancer | SF-295 | -88.88513514 | Prostate Cancer | PC-3 | 11.58377 |
| CNS Cancer | SF-539 | -99.3694362 | Prostate Cancer | DU-145 | -75.9298 |
| CNS Cancer | SNB-19 | -81.51574803 | Breast Cancer | MCF7 | -56.1231 |
| CNS Cancer | SNB-75 | -64.69113842 | Breast Cancer | MDA-MB- | 15.11298 |
| CNS Cancer | U251 | -32.64358108 | Breast Cancer | HS 578T | -36.5499 |
| | | | Breast Cancer | BT-549 | -93.6913 |
| | | | Breast Cancer | T-47D | 22.46038 |
| | | | Breast Cancer | MDA-MB-468 | -21.9933 |

**Table 5. Growth inhibition (% control) of compound 50 in the NCI60 cell lines.**

| 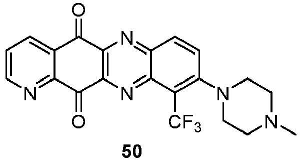 | | | | | |
|---|---|---|---|---|---|
| PANELNME | CELLNAME | GIPRCNT | PANELNME | CELLNAME | GIPRCNT |
| Leukemia | CCRF-CEM | 5.712771 | Melanoma | LOX IMVI | -50.1492 |
| Leukemia | HL-60(TB) | -50.3957 | Melanoma | MALME-3M | -99.1953 |
| Leukemia | K-562 | -6.71679 | Melanoma | M14 | -89.6374 |
| Leukemia | MOLT-4 | -36.4229 | Melanoma | MDA-MB-435 | -91.762 |
| Leukemia | RPMI-8226 | 27.56721 | Melanoma | SK-MEL-2 | -34.5495 |
| Leukemia | SR | -27.1607 | Melanoma | SK-MEL-28 | 5.494684 |
| Non-Small Cell Lung Cancer | A549/ATCC | -36.1751 | Melanoma | SK-MEL-5 | -99.7024 |
| Non-Small Cell Lung Cancer | EKVX | 5.132739 | Melanoma | UACC-257 | -82.2969 |
| Non-Small Cell Lung Cancer | HOP-62 | -63.3298 | Melanoma | UACC-62 | -82.4675 |
| Non-Small Cell Lung Cancer | HOP-92 | 27.49924 | Ovarian Cancer | IGROV1 | -32.57 |
| Non-Small Cell Lung Cancer | NCI-H226 | -78.8735 | Ovarian Cancer | OVCAR-3 | -27.9399 |
| Non-Small Cell Lung Cancer | NCI-H23 | -39.656 | Ovarian Cancer | OVCAR-4 | -91.1398 |
| Non-Small Cell Lung Cancer | NCI-H322M | 36.62518 | Ovarian Cancer | OVCAR-5 | -90.4043 |
| Non-Small Cell Lung Cancer | NCI-H460 | -56.5637 | Ovarian Cancer | OVCAR-8 | -10.8526 |
| Non-Small Cell Lung Cancer | NCI-H522 | -67.5222 | Ovarian Cancer | NCI/ADR-RES | 10.14274 |
| Colon Cancer | COLO 205 | -94.9856 | Ovarian Cancer | SK-OV-3 | 29.67778 |
| Colon Cancer | HCC-2998 | -26.9174 | Renal Cancer | 786-0 | 36.68945 |
| Colon Cancer | HCT-116 | 0.983829 | Renal Cancer | A498 | -21.1398 |
| Colon Cancer | HCT-15 | -47.4718 | Renal Cancer | ACHN | -44.1558 |
| Colon Cancer | HT29 | -55.5144 | Renal Cancer | CAKI-1 | 33.13822 |
| Colon Cancer | KM12 | 12.99617 | Renal Cancer | RXF 393 | 38.35717 |
| Colon Cancer | SW-620 | 4.708827 | Renal Cancer | SN12C | -95.418 |
| CNS Cancer | SF-268 | 1.957085 | Renal Cancer | TK-10 | 80.28756 |
| CNS Cancer | SF-295 | -61.5894 | Renal Cancer | UO-31 | -19.0631 |
| CNS Cancer | SF-539 | -52.4375 | Prostate Cancer | PC-3 | 8.007208 |
| CNS Cancer | SNB-19 | 17.8598 | Prostate Cancer | DU-145 | -85.7143 |
| CNS Cancer | SNB-75 | 5.29558 | Breast Cancer | MCF7 | -57.016 |
| CNS Cancer | U251 | 0.910837 | Breast Cancer | MDA-MB- | -5.99233 |
| | | | Breast Cancer | HS 578T | -15.9764 |
| | | | Breast Cancer | BT-549 | -97.0139 |
| | | | Breast Cancer | T-47D | -28.3961 |
| | | | Breast Cancer | MDA-MB-468 | -62.4106 |

Having now fully described the invention, it will be understood by those of skill in the art that the same can be performed within a wide and equivalent range of conditions, formulations, and other parameters without affecting the scope of the invention or any embodiment thereof.

## Claims

1. A compound of Formula XII, XIII, XIV or XV: 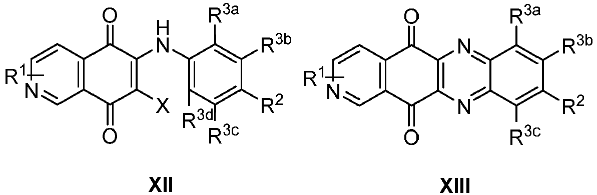 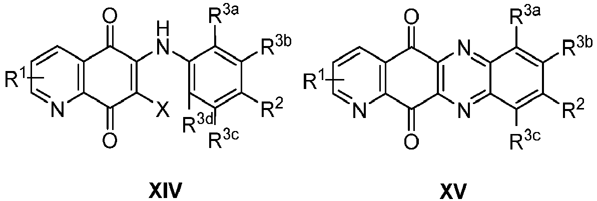 including pharmaceutically acceptable salts, and/or solvates thereof; wherein:
R¹ is selected from the group consisting of hydrogen, or optionally substituted alkyl, or halogen;
R² is substituted selected from the group consisting of: 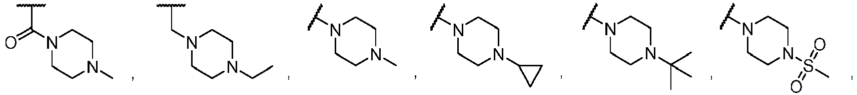 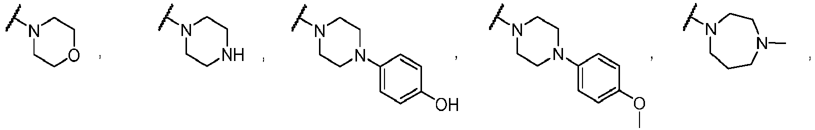 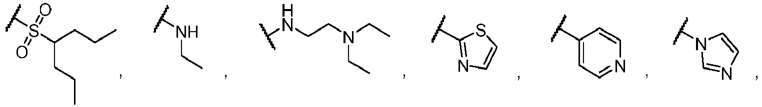 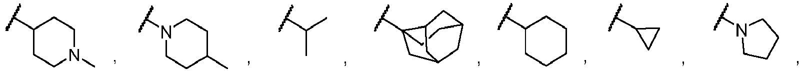 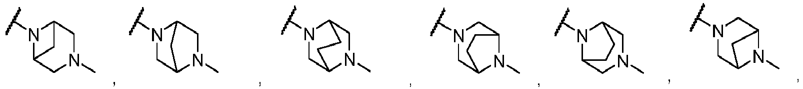 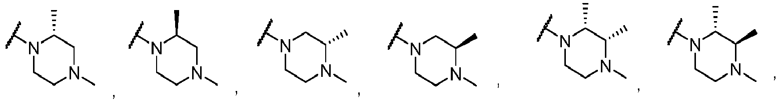 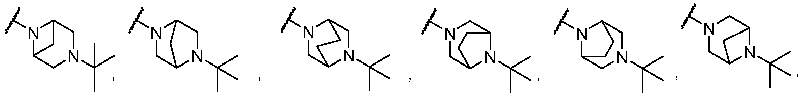 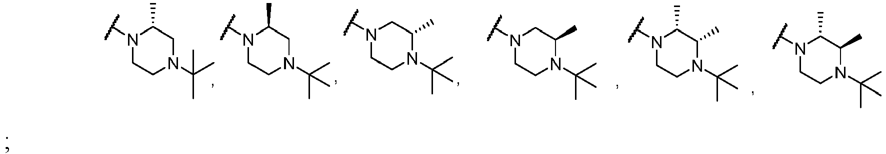
R^{3a}, R^{3b}, R^{3c}, or R^{3d} is selected from the group consisting of hydrogen, halogen, methyl, methoxy, trifluoromethyl, hydroxyl or cyano;
X is selected from the group consisting of hydrogen, halogen, amino, heterocycloalkyl, or hydroxyl.

2. The compound of claim 1 , wherein
R¹ is selected from the group consisting of hydrogen, halo, alkyl, heteroalkyl, optionally substituted C₆-C₁₄ aryl, and optionally substituted 5 to 14 membered heteroaryl,
and/or
R³ (R^{3a}, R^{3b}, R^{3c}, or R^{3d}) is monosubstituted or polysubstituted selected from the group consisting of: 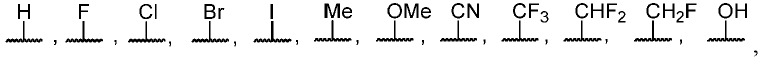 and/or
wherein X is substituted selected from the group consisting of:

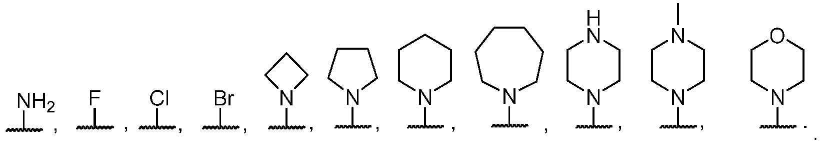

3. The compound of claim 1, wherein the compound is selected from the compounds selected from the group consisting of:
| | | |
|---|---|---|
| 1 | 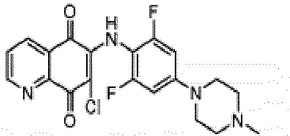 | **7-chloro-6-((2,6-difluoro-4-(4-methylpiperazin-1-yl)phenyl)amino)quinoline-5,8-dione** |
| 2 | 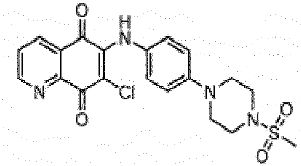 | **7-chloro-6-((4-(4-(methylsulfonyl)piperazin-1-yl)phenyl)amino)quinoline-5,8-dione** |
| 3 | 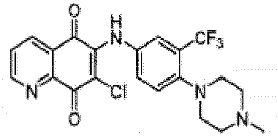 | **7-chloro-6-((4-methylpiperazin-1-yl)-3-(trifluoromelhyl)phenyl)amino)quinoline-5,8-dione** |
| 4 | 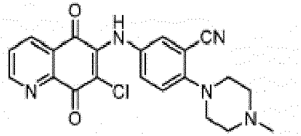 | **5-((7-chloro-5,8-dioxo-5,8-dihydroquinolin-6-yl)amino)-2-(4-methylpiperazin-1-yl)benzonitrile** |
| 5 | 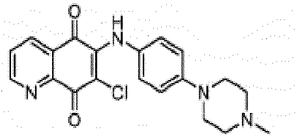 | **7-chloro-6-((4-(4-methylpiperazin-1-yl)phenyl)amino)quinoline-5,8-dione** |
| 6 | 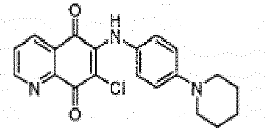 | **7-chloro-6-((4-(piperidin-1-yl)phenyl)amino)quinoline-5,8-dione** |
| 7 | 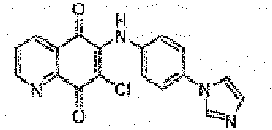 | **6-((4-(1H-imidazol-1-yl)phenyl )amino)-7-chloroquinoline-5,8-dione** |
| 8 | 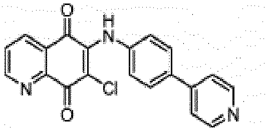 | **7-chloro-6-((4-(pyridin-4-yl)phenyl)amino)quinoline-5,8-dione** |
| 9 | 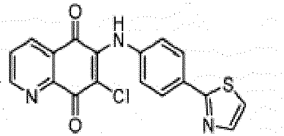 | **7-chloro-6-((4-(thiazol-2-yl)phenyl)amino)quinoline-5,8-dione** |
| 10 | 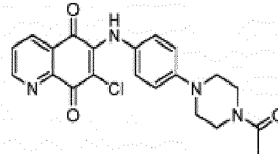 | **6-((4-(4-acetylpiperazin-1-yl)phenyl)amino)-7-chloroquinoline-5,8-dione** |
| 11 | 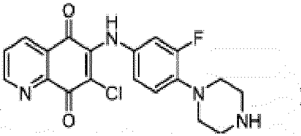 | **7-chloro-6-((3-fluoro-4-(piperazin-1-yl)phenyl)amino)quinoline-5,8-dione** |
| 12 | 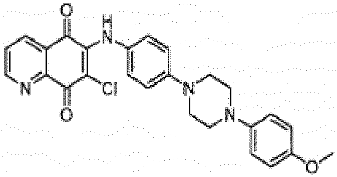 | **7-chloro-6-((4-(4-(4-methoxyphenyl)piperazin-1-yl)phenyl)amino)quinoline-5,8-dione** |
| 13 | 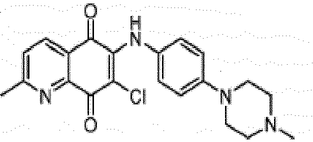 | **7-chloro-2-methyl-6-((4(4-methylpiperazin-1-yl)phenyl)amino)quinoline-5,8-dione** |
| 14 | 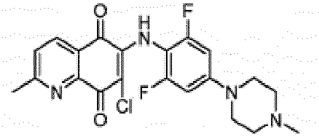 | **7-chloro-6-((2,6-difluoro-4-(4-methylpiperazin-1-yl)phenyl)amino)-2-methylquinoline-5,8-dione** |
| 15 | 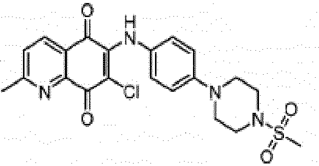 | **7-chloro-2-methyl-6-((4-(4-(methylsulfonyl)piperazin-1-yl)phenyl)amino)quinoline-5,8-dione** |
| 16 | 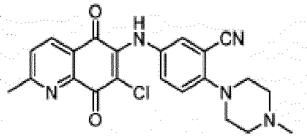 | **5-((7-chloro-2-methyl-5,8-dioxo-5,8-dihydroquinolin-6-yl)amino)-2-(4-methytpiperazin-1-yl)benzonitrile** |
| 17 | 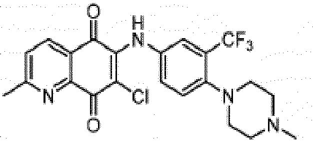 | **7-chloro-2-methyl-6-((4-(4-methylpiperazin-1-yl)-3-(trifluoromethyl)phenyl)amino)quinoline-5,8-dione** |
| 18 | 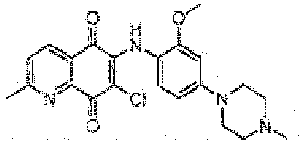 | **7-chloro-6-((2-methoxy-4-(4-methylpiperazin-1-yl)phenyl)amino)-2-methylquinoline-5,8-dione** |
| 19 | 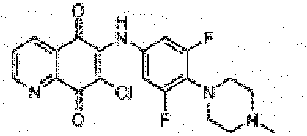 | **7-chloro-6-((3,5-difluoro-4-(4-methylpiperazin-1-yl)phenyl)amino)quinoline-5,8-dione** |
| 20 | 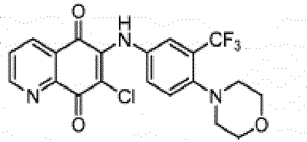 | **7-chloro-6-((4-morpholino-3-(trifluoromethyl)phenyl)amino)quindine-5,8-dione** |
| 21 | 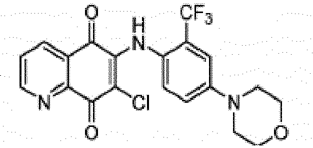 | **7-chloro-6-((4-morpholino-2-(trifluoromethyl)phenyl)amino)quinoline-5,8-dione** |
| 22 | 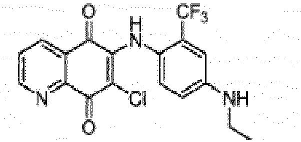 | **7-chloro-6-((4-(ethylamino)-2-(trifluoromethyl)phenyl)amino)quinoline-5,8-dione** |
| 23 | 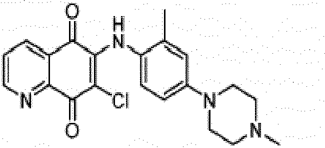 | **7-chloro-6-((2-methyl-4-(4-methylpiperazin-1-yl)phenyl)amino)quinoline-5,8-dione** |
| 24 | 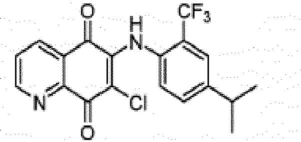 | **7-chloro-6-((4-isopropyl-2-(trifluorotnethyl)phenyl)amino)quinoline-5,8-dione** |
| 25 | 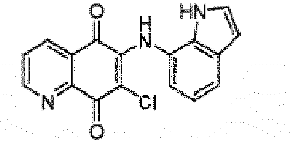 | **6-((1H-indol-7-yl)amino)-7-chloroquinoline-5,8-dione** |
| 26 | 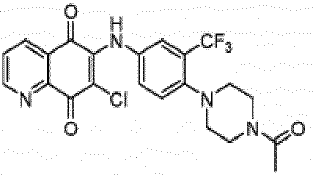 | **6-((4-(4-acetylpiperazin-1-yl)-3-(trifluoromelhyl)phenyl)amino)-7-chloroquinoline-5,8-dione** |
| 27 | 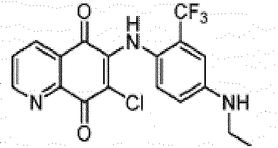 | **7-chloro-6-((4-(ethylamino)-2-(trifluoromethyl)phenyl)amino)quinoline-5,8-dione** |
| 28 | 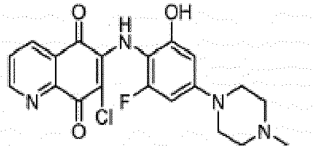 | **7-chloro-6-((2-fluoro-6-hydroxy-4-(4-methylpiperazin-1-yl)phenyl)amino)quinoline-5,8-dione** |
| 29 | 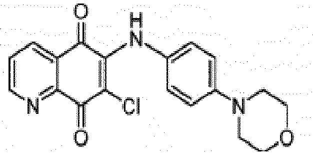 | **7-chloro-6-((4-morpholinophenyl)amino)quinoline-5,8-dione** |
| 30 | 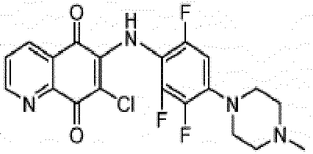 | **7-chloro-6-((2,3,6-trifluoro-4-(4-methylpiperazin-1-yl)phenyl)amino)quinoline-5,8-dione** |
| 31 | 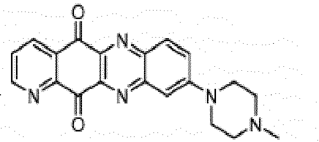 | **9-(4-methylpiperazin-1-yl)pyrido[2,3-b]phenazine-5,12-dione** |
| 43 | 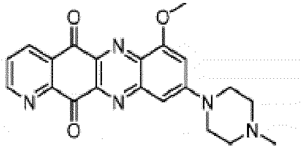 | **7-methoxy-9-(4-methylpiperazin-1-yl)pyrido[2,3-b]phenazine-5,12-dione** |
| 44 | 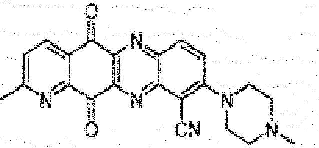 | **2-methyl-9-(4-methylpiperazin-1-yl)-5,12-dioxo-5,12-dihydropyrido[2,3-b]phenazine-10-carbonitrile** |
| 45 | 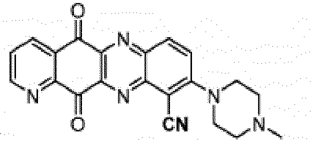 | **9-(4-methylpiperazin-1-yl)-5,12-dioxo-5,12-dihydropyrido[2,3-b]phenazine-10-carbonitrile** |
| 46 | 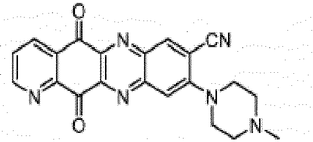 | **9-(4-methylpiperazin-1-yl )-5,12-dioxo-5,12-dihydropyrido[2,3-b]phenazine-8-carbonitrile** |
| 47 | 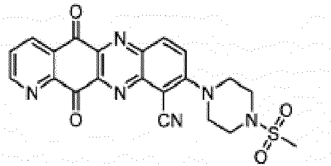 | **9-(4-(methylsulfonyl)piperazin-1-yl)-5,12-dioxo-5,12-dihydropyrido[2,3-b]phenazine-10-carbonitrile** |
| 48 | 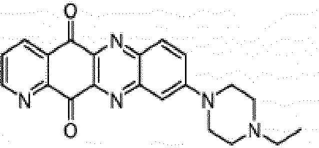 | **9-(4-ethylpiperazin-1-yl)pyrido[2,3-b]phenazine-5,12-dione** |
| 49 | 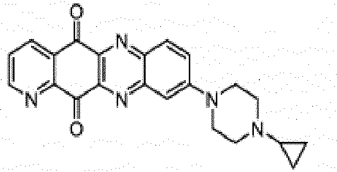 | **9-(4-cyclopropylpiperazin-1-yl)pyrido[2,3-b]phenazine-5,12-dione** |
| 50 | 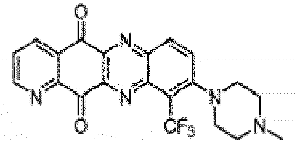 | **9-(4-methylpiperazin-1-yl)-10-(trifluoromethyl)pyrido[2,3-b]phenazine-5,12-dione** |
| 51 | 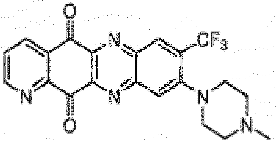 | **9-(4-methylpiperazin-1-yl)-8-(trifluoromethyl)pyrido[2,3-b]phenazine-5,12-dione** |
| 52 | 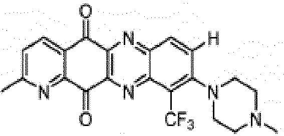 | **2-methyl-9-(4-methylpiperazin-1-yl)-10-(trifluoromethyl)pyrido[2,3-b]phenazine-5,12-dione** |
| 53 | 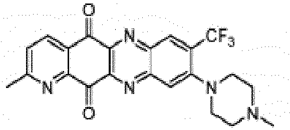 | **2-melhyl-9-(4-melhylpiperazin-1-yl-8-(tifluoromethylpyrido[2,3b]phenazine-5,12-dione** |
| 54 | 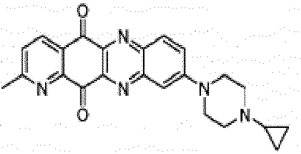 | **9-(4-cyclopropylpiperazin-1-yl)-2-metylpyrido[2,3-b]phenazine-5,12-dione** |
| 55 | 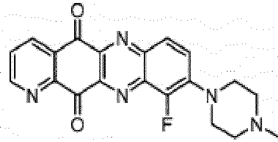 | **10-fluoro-9-(4-methylpiperazin-1-yl)pyrido[2,3-b]phenazine-5,12-dione** |
| 56 | 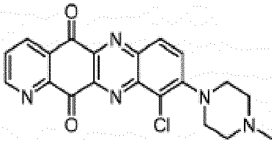 | **10-chloro-9-(4-melhylpiperazin-1-yl)pyrido[2,3-b]phenazine-5,12-dione** |
| 57 | 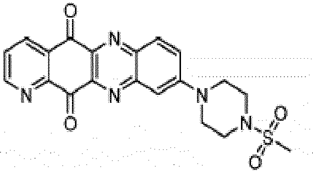 | **9-(4-(methylsulfonyl)piperazin-1-yl)pyrido[2,3-b]phenazine-5,12-dione** |
| 58 | 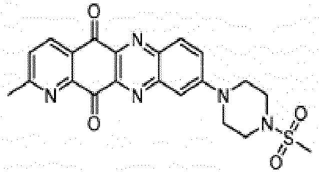 | **2-methyl-9-(4-(methylsulfonyl)piperazin-1-yl)pyrido[2,3-b]phenazine-5,12-dione** |
| 59 | 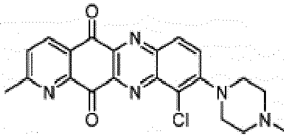 | **10-chloro-2-methyl-9-(4-methylpiperazin-1-yl)pyrido[2,3-b]phenazine-5.12-dione** |
| 60 | 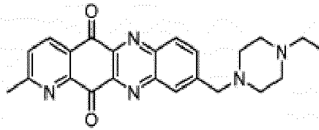 | **9-((4-ethylpiperazin-1-yl)methyl)-2-methylpyrido[2,3-b]phenazine-5,12-dione** |
| 61 | 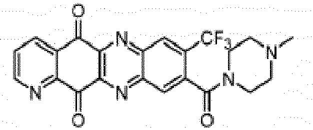 | **9-(4-methylpiperazine-1-carbonyl)-8-(trifluoromethyl)pyrido[2,3-b]phenazine-5,12-dione** |
| 62 | 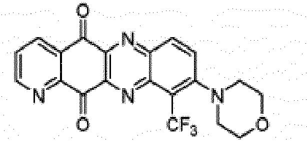 | **9-morpholino-10-(trifluoromethyl)pyrido[2,3-b]phenazine-5,12-dione** |
| 63 | 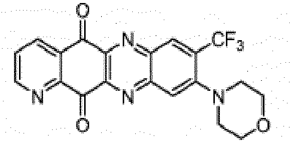 | **9-morpholino-8-(trifluoromethyl)pyrido[2,3-b]phenazine-5,12-dione** |
| 64 | 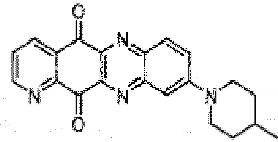 | **9-(4-methylpiperidin-1-yl)pyrido[2,3-b]phenazine-5,12-dione** |
| 65 | 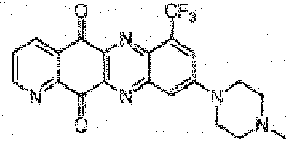 | **9-(4-methylpiperazin-1-yl)-7-(trifluoromethyl)pyrido[2,3-b]phenazine-5,12-dione** |
| 66 | 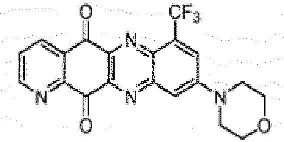 | **9-morpholino-7-(trifluoromethyl)pyrido[2,3-b]phenazine-5,12-dione** |
| 67 | 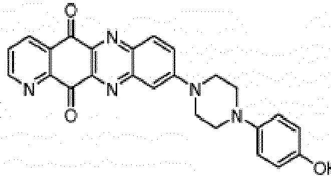 | **9-(4-(4-hydroxyphenyl)piperazin-1-yl)pyrido[2,3-b]phenazine-5,12-dione** |
| 68 | 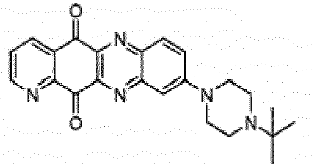 | **9-(4-(tert-butyl)piperazin-1- yl)pyrido[2,3-b]phenazine-5,12-dione** |
| 69 | 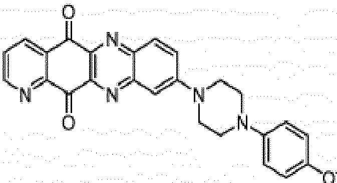 | **9)-(4-(4-methoxyphenyl)piperazin-1- yl)pyrido[2,3-b]phenazine-5,12-dione** |
| 70 | 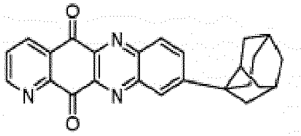 | **9-((3aS,5S,7aS)-octahydro-7aH-2,5-methanoinden-7a-yl)pyrido[2,3-b]phenazine-5,12-dione** |
| 71 | 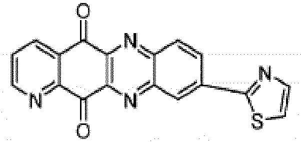 | **9-(thiazol-2-yl)pyrido[2,3-b]phenazine-5,12-dione** |
| 72 | 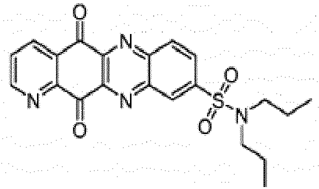 | **5,12-dioxo-N,N-dipropyl-5,12-dihydropyrido[2,3-b]phenazine-9-sulfonamide** |
| 73 | 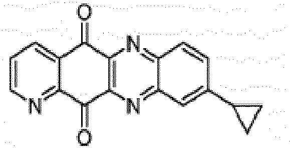 | **9-cyclopropylpyrido[2,3-b]phenazine-5,12-dione** |
| 74 | 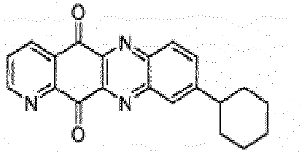 | **9-cyclohexylpyrido[2,3-b]phenazine-5,12-dione** |
| 75 | 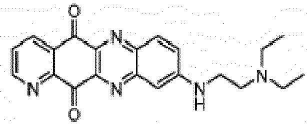 | **9-((2-(diethylamino)ethyl)amino)pyrido[2,3-b]phenazine-5,12-dione** |
| 78 | 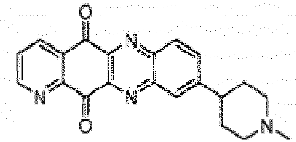 | **9-(1-methylpiperidin-4-yl)pyrido[2,3-b]phenazine-5,12-dione** |
| 79 | 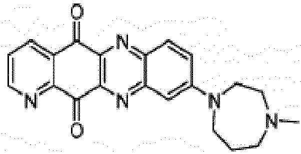 | **9-(4-methyl-1,4-diazepan-1-yl)pyrido[2,3-b]phenazine-5,12-dione** |
| 80 | 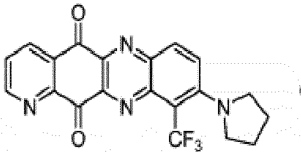 | **9-(pyrrolidin-1-yl)-10-(trifluoromethyl)pyrido[2,3-b]phenazine-5,12-dione** |
| 81 | 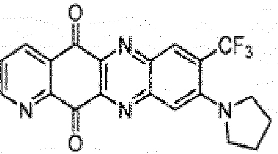 | **9-(pyrrolidin-1-yl)-8-(trifluoromethyl)pyrido[2,3-b]phenazine-5,12-dione** |
| 82 | 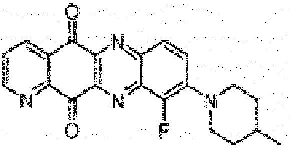 | **10-fluoro-9-(4-methylpiperidin-1-yl)pyrido[2,3-b]phenazine-5,12-dione** |
| 83 | 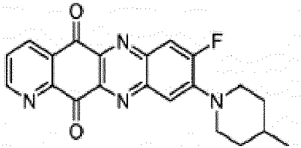 | **8-fluoro-9-(4-methylpiperidin-1-yl)pyrido[2,3-b]phenazine-5,12-dione** |
| 84 | 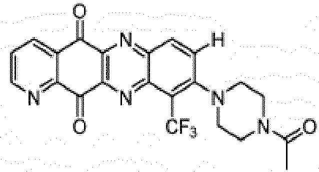 | **9-(4-acetylpiperazin-1-yl)-10-(trifluoromethyl) pyrido[2,3-b]phenazine-5,12-dione** |
| 85 | 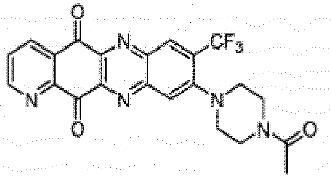 | **9-(4-acetylpiperazin-1-yl)-8-(trifluoromethyl)pyrido[2,3-b]phenazine-5,12-dione** |
| 86 | 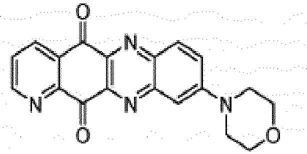 | **9-morpholinopyrido[2,3-b]phenazine-5,12-dione** |
| 87 | 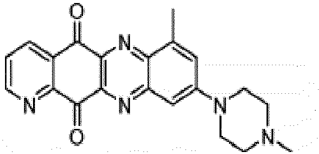 | **7-methyl-9-(4-methylpiperazin-1-yl)pyrido[2,3-b]phenazine-5,12-dione** |
| 88 | 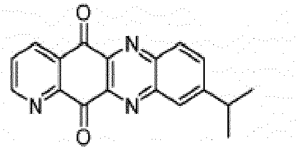 | **9-isopropylpyrido[2,3-b]phenazine-5,12-dione** |

4. A pharmaceutical composition comprising a compound of any of claims 1 - 3 for use in the treatment, amelioration, or prevention of a hyperproliferative disease in a patient wherein a therapeutically effective amount of said pharmaceutical composition is administered to said patient.

5. The pharmaceutical composition for use in the treatment, amelioration, or prevention of a hyperproliferative disease of claim 4, wherein said hyperproliferative disease is cancer,
preferably wherein said cancer is selected from breast cancer, prostate cancer, lymphoma, skin cancer, colon cancer, melanoma, malignant melanoma, ovarian cancer, brain cancer, primary brain carcinoma, head-neck cancer, glioma, glioblastoma, liver cancer, bladder cancer, non-small cell lung cancer, head or neck carcinoma, breast carcinoma, ovarian carcinoma, lung carcinoma, small-cell lung carcinoma, Wilms tumor, cervical carcinoma, testicular carcinoma, bladder carcinoma, pancreatic carcinoma, stomach carcinoma, colon carcinoma, prostatic carcinoma, genitourinary carcinoma, thyroid carcinoma, esophageal carcinoma, myeloma, multiple myeloma, adrenal carcinoma, renal cell carcinoma, endometrial carcinoma, adrenal cortex carcinoma, malignant pancreatic insulinoma, malignant carcinoid carcinoma, choriocarcinoma, mycosis fungoides, malignant hypercalcemia, cervical hyperplasia, leukemia, acute lymphocytic leukemia, chronic lymphocytic leukemia, acute myelogenous leukemia, chronic myelogenous leukemia, chronic granulocytic leukemia, acute granulocytic leukemia, hairy cell leukemia, neuroblastoma, rhabdomyosarcoma, Kaposi's sarcoma, polycythemia vera, essential thrombocytosis, Hodgkin's disease, non- Hodgkin's lymphoma, soft-tissue sarcoma, osteogenic sarcoma, primary macroglobulinemia, and retinoblastoma.

6. The pharmaceutical composition for use in the treatment, amelioration, or prevention of a hyperproliferative disease of claim 4, wherein said patient is a human patient.

7. The pharmaceutical composition for use in the treatment, amelioration, or prevention of a hyperproliferative disease of claim 4, wherein further one or more anticancer agents are administered.

8. The pharmaceutical composition for use in the treatment, amelioration, or prevention of a hyperproliferative disease of claim 7, wherein said anticancer agent is a chemotherapeutic agent.

9. The pharmaceutical composition for use in the treatment, amelioration, or prevention of a hyperproliferative disease of claim 7, wherein said anticancer agent is radiation therapy.

10. A kit comprising any of the compounds of Claims 1-3 and instructions for administering said compound to a patient having a hyperproliferative disease.

11. The kit of claim 10 wherein said hyperproliferative disease is cancer.

12. The kit of Claim 11, wherein said cancer is selected from breast cancer, prostate cancer, lymphoma, skin cancer, colon cancer, melanoma, malignant melanoma, ovarian cancer, brain cancer, primary brain carcinoma, head-neck cancer, glioma, glioblastoma, liver cancer, bladder cancer, non-small cell lung cancer, head or neck carcinoma, breast carcinoma, ovarian carcinoma, lung carcinoma, small-cell lung carcinoma, Wilms tumor, cervical carcinoma, testicular carcinoma, bladder carcinoma, pancreatic carcinoma, stomach carcinoma, colon carcinoma, prostatic carcinoma, genitourinary carcinoma, thyroid carcinoma, esophageal carcinoma, myeloma, multiple myeloma, adrenal carcinoma, renal cell carcinoma, endometrial carcinoma, adrenal cortex carcinoma, malignant pancreatic insulinoma, malignant carcinoid carcinoma, choriocarcinoma, mycosis fungoides, malignant hypercalcemia, cervical hyperplasia, leukemia, acute lymphocytic leukemia, chronic lymphocytic leukemia, acute myelogenous leukemia, chronic myelogenous leukemia, chronic granulocytic leukemia, acute granulocytic leukemia, hairy cell leukemia, neuroblastoma, rhabdomyosarcoma, Kaposi's sarcoma, polycythemia vera, essential thrombocytosis, Hodgkin's disease, non- Hodgkin's lymphoma, soft-tissue sarcoma, osteogenic sarcoma, primary macroglobulinemia, and retinoblastoma.

13. The kit of claim 10 further comprising one or more anticancer agents.

14. The kit of claim 13, wherein said compound is to be administered together with one or more anticancer agents.

## Patentansprüche

1. Verbindung der Formel XII, XIII, XIV oder XV: 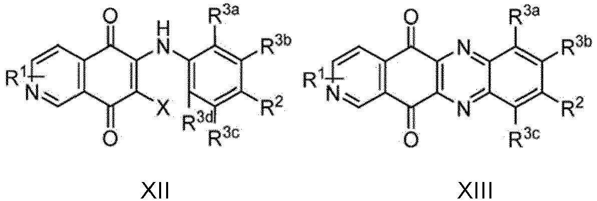 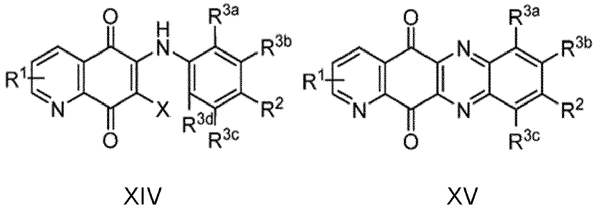 einschließlich pharmazeutisch unbedenklicher Salze und/oder Solvate davon, wobei:
R¹ aus der aus Wasserstoff, gegebenenfalls substituiertem Alkyl und Halogen bestehenden Gruppe ausgewählt ist,
R² ausgewählt aus der folgenden Gruppe substituiert ist: 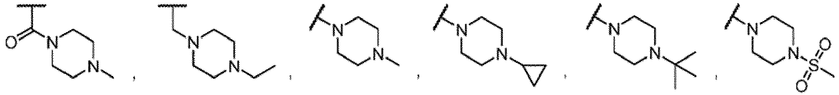 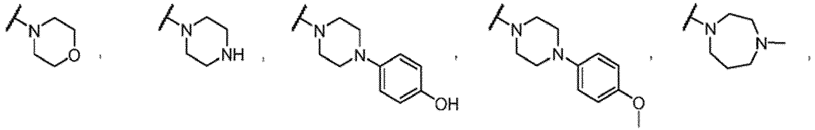 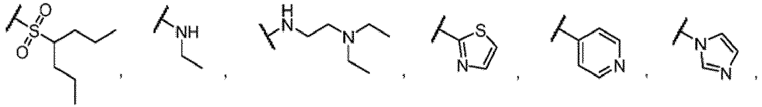 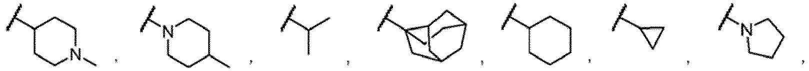 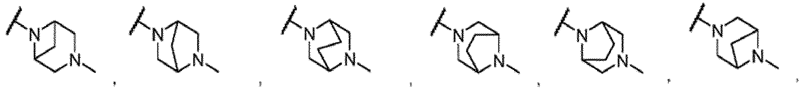 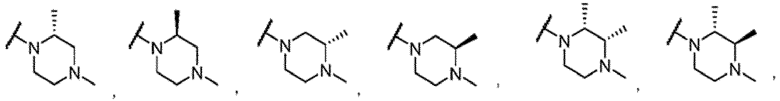 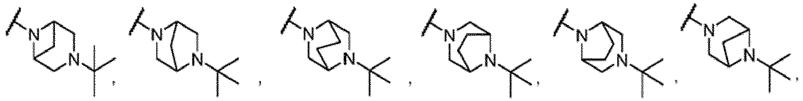 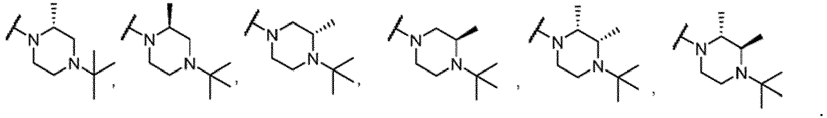
R^{3a} R^{3b}, R^{3c} oder R^{3d} aus der aus Wasserstoff, Halogen, Methyl, Methoxy, Trifluormethyl, Hydroxyl und Cyano bestehenden Gruppe ausgewählt ist,
X aus der aus Wasserstoff, Halogen, Amino, Heterocycloalkyl und Hydroxyl bestehenden Gruppe ausgewählt ist.

2. Verbindung nach Anspruch 1, wobei
R¹ aus der aus Wasserstoff, Halogen, Alkyl, Heteroalkyl, gegebenenfalls substituiertem C₆-C₁₄-Aryl und gegebenenfalls substituiertem 5- bis 14-gliedrigen Heteroaryl bestehenden Gruppe ausgewählt ist,
und/oder
R³ (R^{3a}, R^{3b}, R^{3c} oder R^{3d}) einfach oder mehrfach ausgewählt aus der folgenden Gruppe substituiert ist: 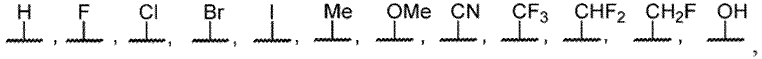 und/oder
wobei X ausgewählt aus der folgenden Gruppe substituiert ist:

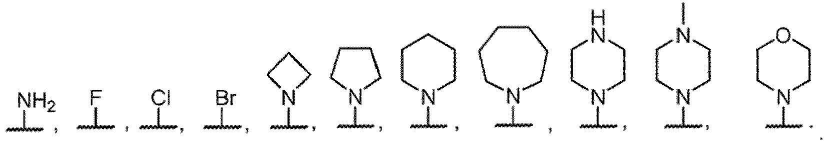

3. Verbindung nach Anspruch 1, wobei die Verbindung ausgewählt ist aus den Verbindungen ausgewählt aus der Gruppe bestehend aus:
| | | |
|---|---|---|
| 1 | 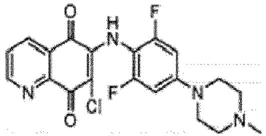 | 7-Chlor-6-((2,6-difluor-4-(4-methylpiperazin-1-yl)phenyl)amino)chinolin-5,8-dion |
| 2 | 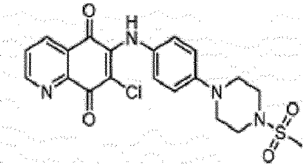 | 7-Chlor-6-((4-(4-(methylsulfonyl)piperazin-1-yl)phenyl)amino)chinolin-5,8-dion |
| 3 | 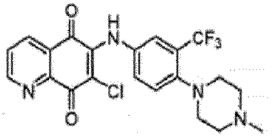 | 7-Chlor-6-((4-(4-methylpiperazin-1-yl)-3-(trifluormethyl)phenyl)amino)chinolin-5,8-dion |
| 4 | 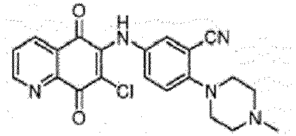 | 5-((7-Chlor-5,8-dioxo-5,8-dihydrochinolin-6-yl)amino)-2-(4-methylpiperazin-1-yl)benzonitril |
| 5 | 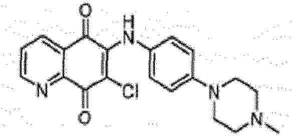 | 7-Chlor-6-((4-(4-methylpiperazin-1-yl)phenyl)amino)chinolin-5,8-dion |
| 6 | 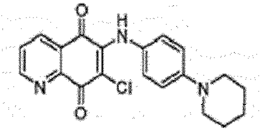 | 7-Chlor-6-((4-(piperidin-1-yl)phenyl)amino)chinolin-5,8-dion |
| 7 | 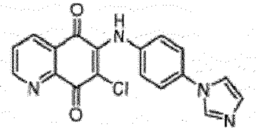 | 6-((4-(1H-Imidazol-1-yl)phenyl)amino)-7-chlorchinolin-5,8-dion |
| 8 | 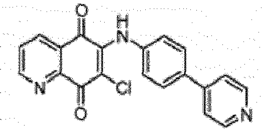 | 7-Chlor-6-((4-(pyridin-4-yl)phenyl)amino)chinolin-5,8-dion |
| 9 | 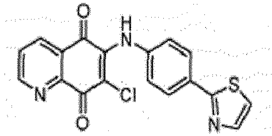 | 7-Chlor-6-((4-(thiazol-2-yl)phenyl)amino)chinolin-5,8-dion |
| 10 | 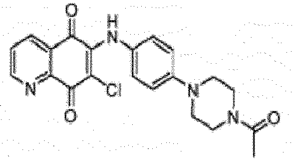 | 6-((4-(4-Acetylpiperazin-1-yl)phenyl)amino)-7-chlorchinolin-5,8-dion |
| 11 | 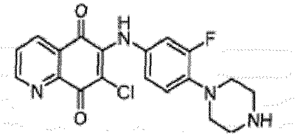 | 7-Chlor-6-((3-fluor-4-(piperazin-1-yl)phenyl)amino)chinolin-5,8-dion |
| 12 | 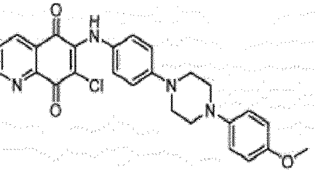 | 7-Chlor-6-((4-(4-(4-methoxyphenyl)piperazin-1-yl)phenyl)amino)chinolin-5,8-dion |
| 13 | 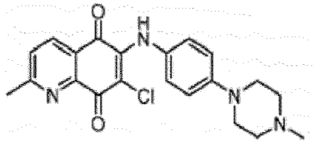 | 7-Chlor-2-methyl-6-((4-(4-methylpiperazin-1-yl)phenyl)amino)chinolin-5,8-dion |
| 14 | 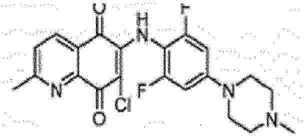 | 7-Chlor-6-((2,6-difluor-4-(4-methylpiperazin-1-yl)phenyl)amino)-2-methylchinolin-5,8-dion |
| 15 | 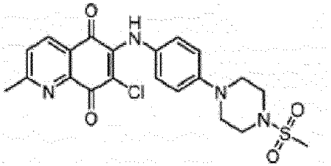 | 7-Chlor-2-methyl-6-((4-(4-(methylsulfonyl)piperazin-1-yl)phenyl)amino)chinolin-5,8-dion |
| 16 | 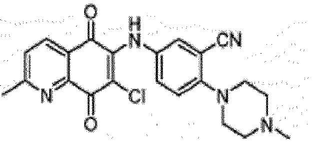 | 5-((7-Chlor-2-methyl-5,8-dioxo-5,8-dihydrochinolin-6-yl)amino)-2-(4-methylpiperazin-1-yl)benzonitril |
| 17 | 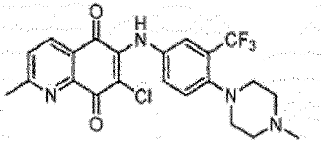 | 7-Chlor-2-methyl-6-((4-(4-methylpiperazin-1-yl)-3-(trifluormethyl)phenyl)amino)chinolin-5,8-dion |
| 18 | 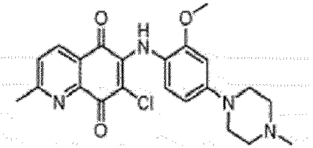 | 7-Chlor-6-((2-methoxy-4-(4-methylpiperazin-1-yl)phenyl)amino)-2-methylchinolin-5,8-dion |
| 19 | 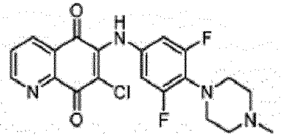 | 7-Chlor-6-((3,5-difluor-4-(4-methylpiperazin-1-yl)phenyl)amino)chinolin-5,8-dion |
| 20 | 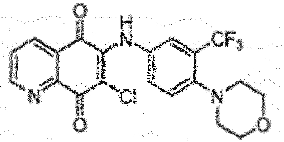 | 7-Chlor-6-((4-morpholino-3-(trifluormethyl)phenyl)amino)chinolin-5,8-dion |
| 21 | 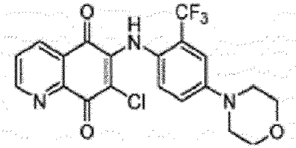 | 7-Chlor-6-((4-morpholino-2-(trifluormethyl)phenyl)amino)chinolin-5,8-dion |
| 22 | 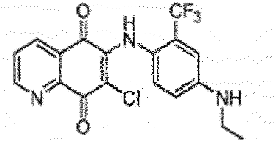 | 7-Chlor-6-((4-(ethylamino)-2-(trifluormethyl)phenyl)amino)chinolin-5,8-dion |
| 23 | 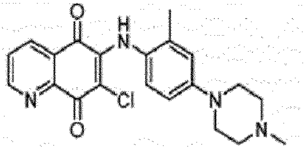 | 7-Chlor-6-((2-methyl-4-(4-methylpiperazin-1-yl)phenyl)amino)chinolin-5,8-dion |
| 24 | 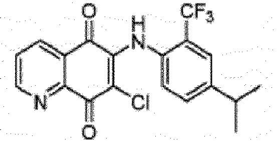 | 7-Chlor-6-((4-isopropyl-2-(trifluormethyl)phenyl)amino)chinolin-5,8-dion |
| 25 | 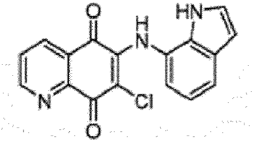 | 6-((1H-Indol-7-yl)amino)-7-chlorchinolin-5,8-dion |
| 26 | 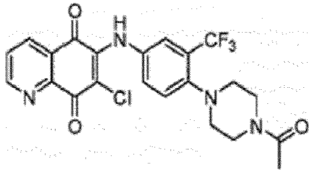 | 6-((4-(4-Acetylpiperazin-1-yl)-3-(trifluormethyl)phenyl)amino)-7-chlorchinolin-5,8-dion |
| 27 | 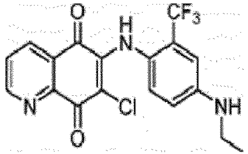 | 7-Chlor-6-((4-(ethylamino)-2-(trifluormethyl)phenyl)amino)chinolin-5,8-dion |
| 28 | 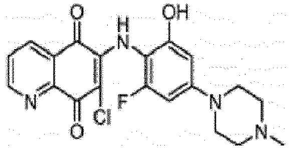 | 7-Chlor-6-((2-fluor-6-hydroxy-4-(4-methylpiperazin-1-yl)phenyl)amino)chinolin-5,8-dion |
| 29 | 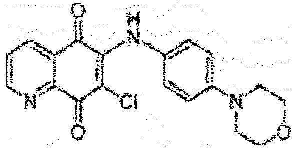 | 7-Chlor-6-((4-morpholinophenyl)amino)chinolin-5,8-dion |
| 30 | 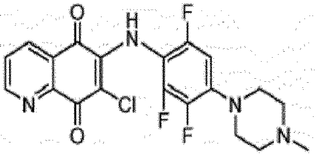 | 7-Chlor-6-((2,3,6-trifluor-4-(4-methylpiperazin-1-yl)phenyl)amino)chinolin-5,8-dion |
| 42 | 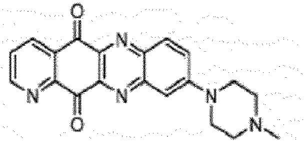 | 9-(4-Methylpiperazin-1-yl)pyrido[2,3-b]phenazin-5,12-dion |
| 43 | 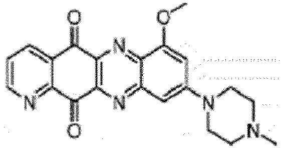 | 7-Methoxy-9-(4-methylpiperazin-1-yl)pyrido[2,3-b]phenazin-5,12-dion |
| 44 | 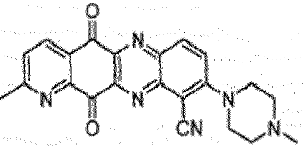 | 2-Methyl-9-(4-methylpiperazin-1-yl)-5,12-dioxo-5,12-dihydropyrido[2,3-b]phenazin-10-carbonsäurenitril |
| 45 | 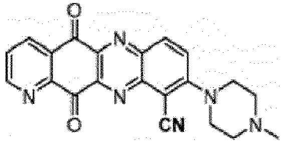 | 9-(4-Methylpiperazin-1-yl)-5,12-dioxo-5,12-dihydropyrido[2,3-b]phenazin-10-carbonsäurenitril |
| 46 | 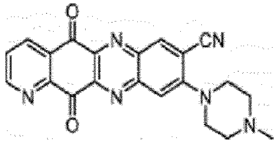 | 9-(4-Methylpiperazin-1-yl)-5,12-dioxo-5,12-dihydropyrido[2,3-b]phenazin-8-carbonsäurenitril |
| 47 | 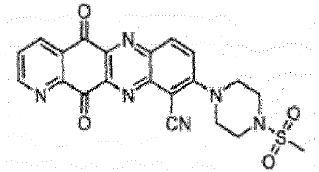 | 9-(4-(Methylsulfonyl)piperazin-1-yl)-5,12-dioxo-5,12-dihydropyrido[2,3-b]phenazin-10-carbonsäurenitril |
| 48 | 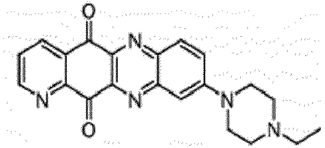 | 9-(4-Ethylpiperazin-1-yl)pyrido[2,3-b]phenazin-5,12-dion |
| 49 | 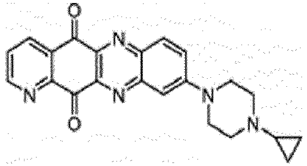 | 9-(4-Cyclopropylpiperazin-1-yl)pyrido[2,3-b]phenazin-5,12-dion |
| 50 | 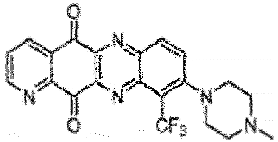 | 9-(4-Methylpiperazin-1-yl)-10-(trifluormethyl)pyrido[2,3-b]phenazin-5,12-dion |
| 51 | 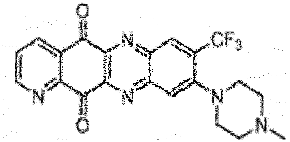 | 9-(4-Methylpiperazin-1-yl)-8-(trifluormethyl)pyrido[2,3-b]phenazin-5,12-dion |
| 52 | 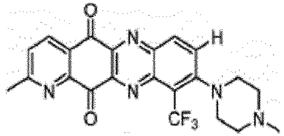 | 2-Methyl-9-(4-methylpiperazin-1-yl)-10-(trifluormethyl)pyrido[2,3-b]phenazin-5,12-dion |
| 53 | 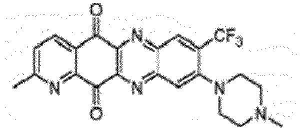 | 2-Methyl-9-(4-methylpiperazin-1-yl)-8-(trifluormethyl)pyrido[2,3-b]phenazin-5,12-dion |
| 54 | 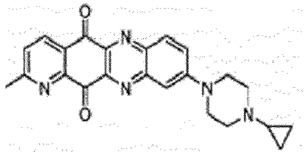 | 9-(4-Cyclopropylpiperazin-1-yl)-2-methylpyrido[2,3-b]phenazin-5,12-dion |
| 55 | 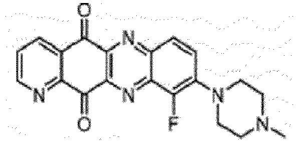 | 10-Fluor-9-(4-methylpiperazin-1-yl)pyrido[2,3-b]phenazin-5,12-dion |
| 56 | 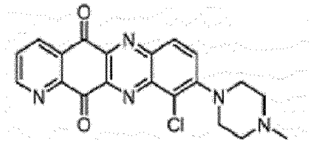 | 10-Chlor-9-(4-methylpiperazin-1-yl)pyrido[2,3-b]phenazin-5,12-dion |
| 57 | 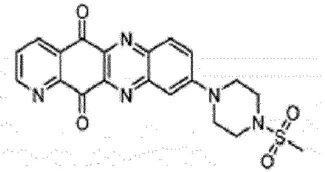 | 9-(4-(Methylsulfonyl)piperazin-1-yl)pyrido[2,3-b]phenazin-5,12-dion |
| 58 | 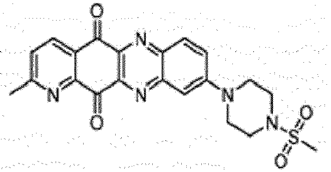 | 2-Methyl-9-(4-(methylsulfonyl)piperazin-1-yl)pyrido[2,3-b]phenazin-5,12-dion |
| 59 | 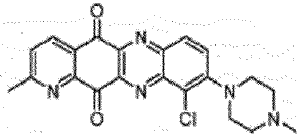 | 10-Chlor-2-methyl-9-(4-methylpiperazin-1-yl)pyrido[2,3-b]phenazin-5,12-dion |
| 60 | 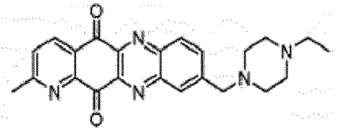 | 9-((4-Ethylpiperazin-1-yl)methyl)-2-methylpyrido[2,3-b]phenazin-5,12-dion |
| 61 | 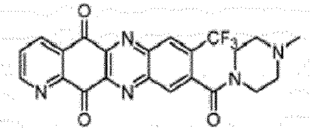 | 9-(4-Methylpiperazin-1-carbonyl)-8-(trifluormethyl)pyrido[2,3-b]phenazin-5,12-dion |
| 62 | 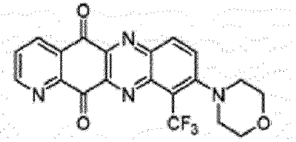 | 9-Morpholino-10-(trifluormethyl)pyrido[2,3-b]phenazin-5,12-dion |
| 63 | 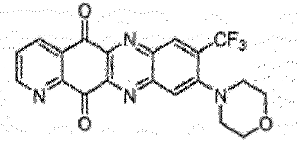 | 9-Morpholino-8-(trifluormethyl)pyrido[2,3-b]phenazin-5,12-dion |
| 64 | 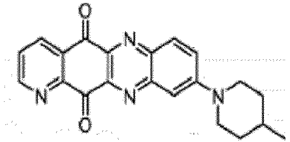 | 9-(4-Methylpiperidin-1-yl)pyrido[2,3-b]phenazin-5,12-dion |
| 65 | 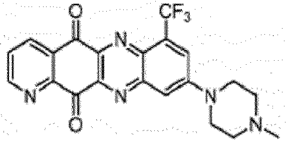 | 9-(4-Methylpiperazin-1-yl)-7-(trifluormethyl)pyrido[2,3-b]phenazin-5,12-dion |
| 66 | 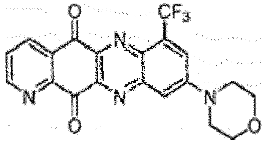 | 9-Morpholino-7-(trifluormethyl)pyrido[2,3-b]phenazin-5,12-dion |
| 67 | 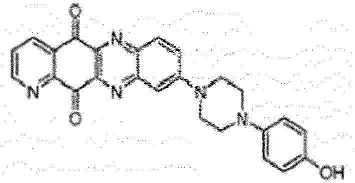 | 9-(4-(4-Hydroxyphenyl)piperazin-1-yl)pyrido[2,3-b]phenazin-5,12-dion |
| 68 | 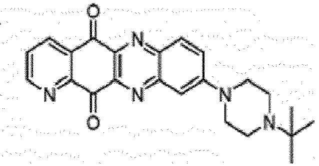 | 9-(4-(tert.-Butyl)piperazin-1-yl)pyrido[2,3-b]phenazin-5,12-dion |
| 69 | 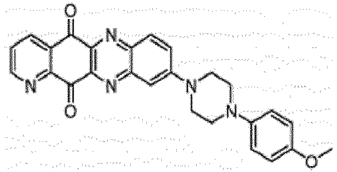 | 9-(4-(4-Methoxyphenyl)piperazin-1-yl)pyrido[2,3-b]phenazin-5,12-dion |
| 70 | 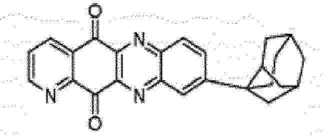 | 9-((3aS,5S,7aS)-Octahydro-7aH-2,5-methanoinden-7a-yl)pyrido[2,3-b]phenazin-5,12-dion |
| 71 | 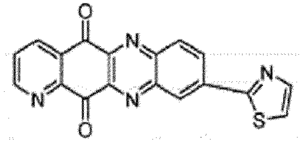 | 9-(Thiazol-2-yl)pyrido[2,3-b]phenazin-5,12-dion |
| 72 | 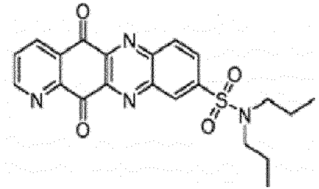 | 5,12-Dioxo-N,N-dipropyl-5,12-dihydropyrido[2,3-b]phenazin-9-sulfonamid |
| 73 | 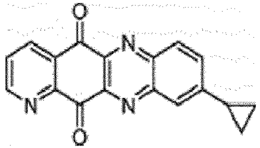 | 9-Cyclopropylpyrido[2,3-b]phenazin-5,12-dion |
| 74 | 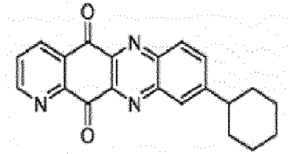 | 9-Cyclohexylpyrido[2,3-b]phenazin-5,12-dion |
| 75 | 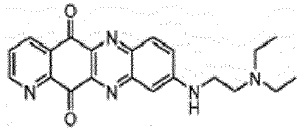 | 9-((2-(Diethylamino)ethyl)amino)pyrido[2,3-b]phenazin-5,12-dion |
| 78 | 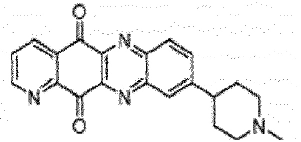 | 9-(1-Methylpiperidin-4-yl)pyrido[2,3-b]phenazin-5,12-dion |
| 79 | 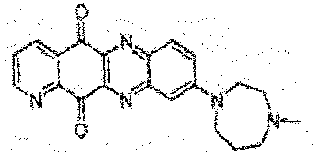 | 9-(4-Methyl-1,4-diazepan-1-yl)pyrido[2,3-b]phenazin-5,12-dion |
| 80 | 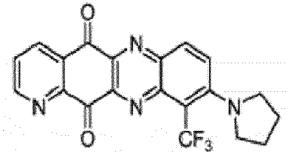 | 9-(Pyrrolidin-1-yl)-10-(trifluormethyl)pyrido[2,3-b]phenazin-5,12-dion |
| 81 | 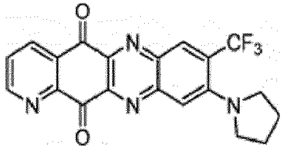 | 9-(Pyrrolidin-1-yl)-8-(trifluormethyl)pyrido[2,3-b]phenazin-5,12-dion |
| 82 | 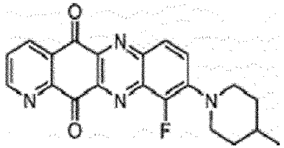 | 10-Fluor-9-(4-methylpiperidin-1-yl)pyrido[2,3-b]phenazin-5,12-dion |
| 83 | 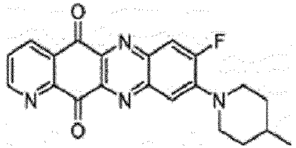 | 8-Fluor-9-(4-methylpiperidin-1-yl)pyrido[2,3-b]phenazin-5,12-dion |
| 84 | 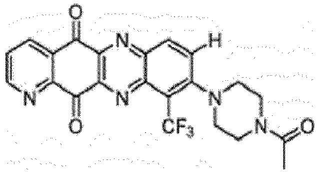 | 9-(4-Acetylpiperazin-1-yl)-10-(trifluormethyl)pyrido[2,3-b]phenazin-5,12-dion |
| 85 | 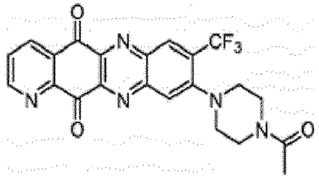 | 9-(4-Acetylpiperazin-1-yl)-8-(trifluormethyl)pyrido[2,3-b]phenazin-5,12-dion |
| 86 | 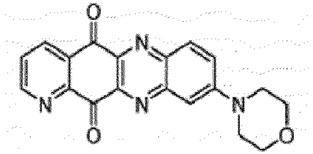 | 9-Morpholinopyrido[2,3-b]phenazin-5,12-dion |
| 87 | 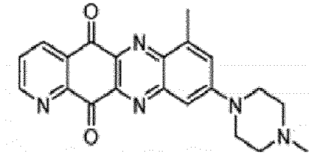 | 7-Methyl-9-(4-methylpiperazin-1-yl)pyrido[2,3-b]phenazin-5,12-dion |
| 88 | 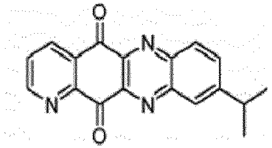 | 9-Isopropylpyrido[2,3-b]phenazin-5,12-dion |

4. Pharmazeutische Zusammensetzung, umfassend eine Verbindung nach einem der Ansprüche 1 - 3 zur Verwendung bei der Behandlung, Linderung oder Prävention einer hyperproliferativen Erkrankung bei einem Patienten, wobei dem Patienten eine therapeutisch wirksame Menge der pharmazeutischen Zusammensetzung verabreicht wird.

5. Pharmazeutische Zusammensetzung zur Verwendung bei der Behandlung, Linderung oder Prävention einer hyperproliferativen Erkrankung nach Anspruch 4, wobei es sich bei der hyperproliferativen Erkrankung um Krebs handelt,
wobei die Krebserkrankung vorzugsweise aus Brustkrebs, Prostatakrebs, Lymphom, Hautkrebs, Dickdarmkrebs, Melanom, malignem Melanom, Eierstockkrebs, Hirnkrebs, primärem Hirnkarzinom, Kopf-Hals-Krebs, Gliom, Glioblastom, Leberkrebs, Blasenkrebs, nichtkleinzelligem Lungenkrebs, Kopf- oder Halskarzinom, Brustkarzinom, Eierstockkarzinom, Lungenkarzinom, kleinzelligem Lungenkarzinom, Wilms-Tumor, Gebärmutterhalskarzinom, Hodenkarzinom, Blasenkarzinom, Pankreaskarzinom, Magenkarzinom, Dickdarmkarzinom, Prostatakarzinom, Urogenitalkarzinom, Schilddrüsenkarzinom, Speiseröhrenkarzinom, Myelom, multiplem Myelom, Nebennierenkarzinom, Nierenzellkarzinom, Endometriumkarzinom, Nebennierenrindenkarzinom, malignem Pankreasinsulinom, malignem Karzinoidkarzinom, Choriokarzinom, Mycosis fungoides, maligner Hyperkalzämie, Zervixhyperplasie, Leukämie, akuter lymphatischer Leukämie, chronischer lymphatischer Leukämie, akuter myeloischer Leukämie, chronischer myeloischer Leukämie, chronischer granulozytärer Leukämie, akuter granulozytärer Leukämie, Haarzellenleukämie, Neuroblastom, Rhabdomyosarkom, Kaposi-Sarkom, Polycythemia vera, essentieller Thrombozytose, Morbus Hodgkin, Non-Hodgkin-Lymphom, Weichteilsarkom, osteogenem Sarkom, primärer Makroglobulinämie und Retinoblastom ausgewählt ist.

6. Pharmazeutische Zusammensetzung zur Verwendung bei der Behandlung, Linderung oder Prävention einer hyperproliferativen Erkrankung nach Anspruch 4, wobei es sich bei dem Patienten um einen Menschen handelt.

7. Pharmazeutische Zusammensetzung zur Verwendung bei der Behandlung, Linderung oder Prävention einer hyperproliferativen Erkrankung nach Anspruch 4, wobei weiterhin ein oder mehrere Krebsmittel verabreicht werden.

8. Pharmazeutische Zusammensetzung zur Verwendung bei der Behandlung, Linderung oder Prävention einer hyperproliferativen Erkrankung nach Anspruch 7, wobei es sich bei dem Krebsmittel um ein Chemotherapeutikum handelt.

9. Pharmazeutische Zusammensetzung zur Verwendung bei der Behandlung, Linderung oder Prävention einer hyperproliferativen Erkrankung nach Anspruch 7, wobei es sich bei dem Krebsmittel um Strahlentherapie handelt.

10. Kit, umfassend eine der Verbindungen nach einem der Ansprüche 1-3 und Anweisungen zur Verabreichung der Verbindung an einen an einer hyperproliferativen Erkrankung leidenden Patienten.

11. Kit nach Anspruch 10, wobei es sich bei der hyperproliferativen Erkrankung um Krebs handelt.

12. Kit nach Anspruch 11, wobei die Krebserkrankung vorzugsweise aus Brustkrebs, Prostatakrebs, Lymphom, Hautkrebs, Dickdarmkrebs, Melanom, malignem Melanom, Eierstockkrebs, Hirnkrebs, primärem Hirnkarzinom, Kopf-Hals-Krebs, Gliom, Glioblastom, Leberkrebs, Blasenkrebs, nichtkleinzelligem Lungenkrebs, Kopf- oder Halskarzinom, Brustkarzinom, Eierstockkarzinom, Lungenkarzinom, kleinzelligem Lungenkarzinom, Wilms-Tumor, Gebärmutterhalskarzinom, Hodenkarzinom, Blasenkarzinom, Pankreaskarzinom, Magenkarzinom, Dickdarmkarzinom, Prostatakarzinom, Urogenitalkarzinom, Schilddrüsenkarzinom, Speiseröhrenkarzinom, Myelom, multiplem Myelom, Nebennierenkarzinom, Nierenzellkarzinom, Endometriumkarzinom, Nebennierenrindenkarzinom, malignem Pankreasinsulinom, malignem Karzinoidkarzinom, Choriokarzinom, Mycosis fungoides, maligner Hyperkalzämie, Zervixhyperplasie, Leukämie, akuter lymphatischer Leukämie, chronischer lymphatischer Leukämie, akuter myeloischer Leukämie, chronischer myeloischer Leukämie, chronischer granulozytärer Leukämie, akuter granulozytärer Leukämie, Haarzellenleukämie, Neuroblastom, Rhabdomyosarkom, Kaposi-Sarkom, Polycythemia vera, essentieller Thrombozytose, Morbus Hodgkin, Non-Hodgkin-Lymphom, Weichteilsarkom, osteogenem Sarkom, primärer Makroglobulinämie und Retinoblastom ausgewählt ist.

13. Kit nach Anspruch 10, weiterhin umfassend ein oder mehrere Krebsmittel.

14. Kit nach Anspruch 13, wobei die Verbindung zusammen mit einem oder mehreren Krebsmitteln verabreicht wird.

## Revendications

1. Composé de Formule XII, XIII, XIV ou XV : 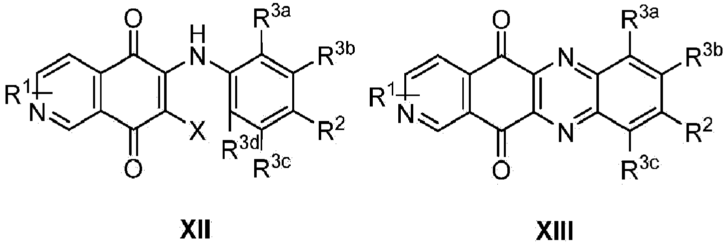 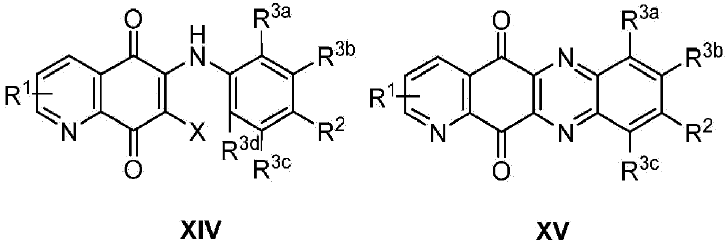 incluant des sels pharmaceutiquement acceptables et/ou des solvates de ceux-ci ; où :
R¹ est choisi dans le groupe constitué d'un hydrogène, ou d'un alkyle éventuellement substitué, ou d'un halogène ;
R² est substitué, choisi dans le groupe constitué de : 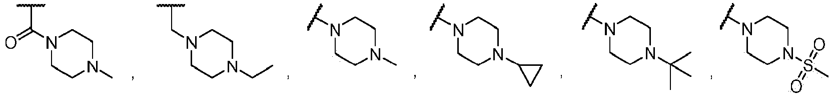 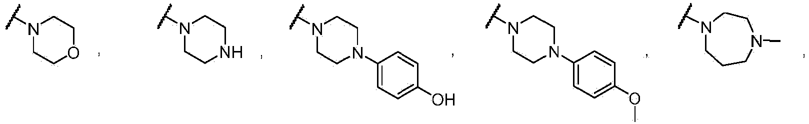 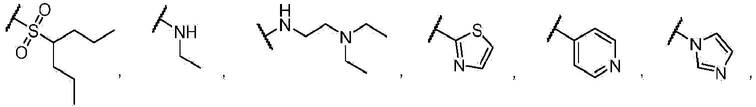 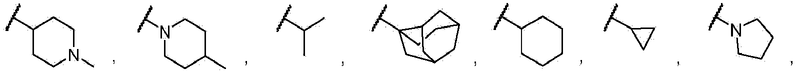 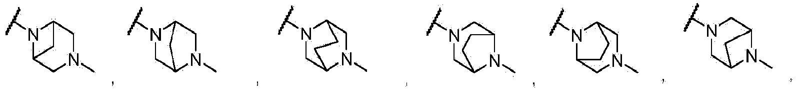 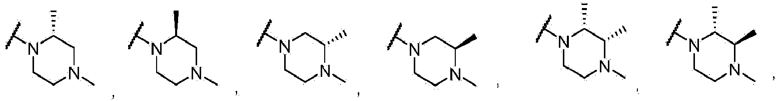 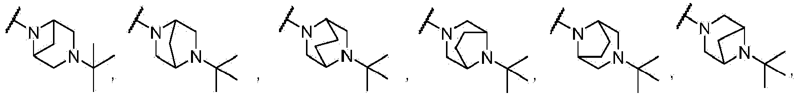 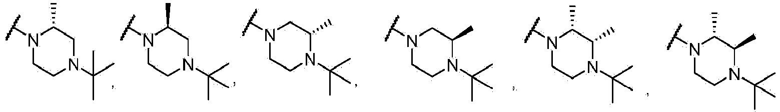
R^{3a}, R^{3b}, R^{3c}, ou R^{3d} est choisi dans le groupe constitué d'un hydrogène, d'un halogène, d'un méthyle, d'un méthoxy, d'un trifluorométhyle, d'un hydroxyle ou d'un cyano ;
X est choisi dans le groupe constitué d'un hydrogène, d'un halogène, d'un amino, d'un hétérocycloalkyle ou d'un hydroxyle.

2. Composé de la revendication 1, dans lequel
R¹ est choisi dans le groupe constitué d'un hydrogène, d'un halo, d'un alkyle, d'un hétéroalkyle, d'un aryle en C₆ à C₁₄ éventuellement substitué et d'un hétéroaryle de 5 à 14 chaînons éventuellement substitué,
et/ou
R³ (R^{3a}, R^{3b}, R^{3c} ou R^{3d}) est monosubstitué ou polysubstitué choisi dans le groupe constitué de : 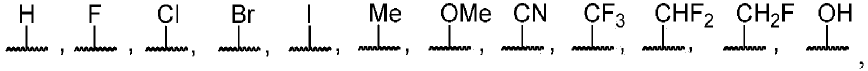 et/ou
dans lequel X est substitué, choisi dans le groupe constitué de :

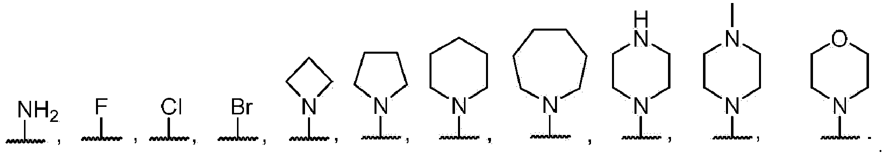

3. Composé de la revendication 1, dans lequel le composé est choisi parmi les composés choisis dans le groupe constitué de :
| | |
|---|---|
| 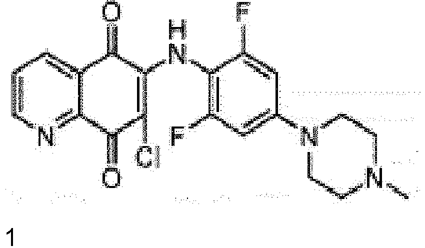 | 7-chloro-6-((2,6-difluoro-4-(4-méthylpipérazin-1-yl)phényl)amino)quinoléine-5,8-dione |
| 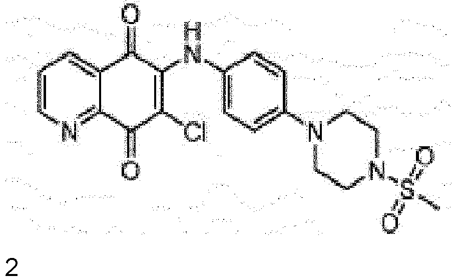 | 7-chloro-6-((4-(4-(méthylsulfonyl)pipérazin-1-yl)phényl)amino)quinoléine-5,8-dione |
| 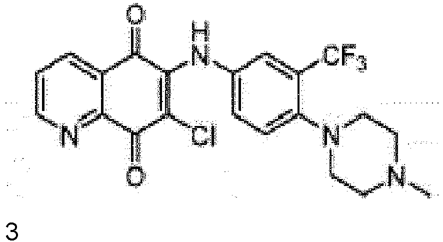 | 7-chloro-6-((4-(4-méthylpipérazin-1-yl)-3-(trifluorométhyl)phényl)amino)quinoléine-5,8-dione |
| 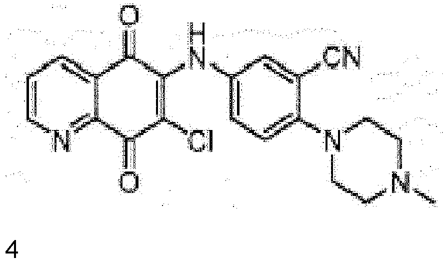 | 5-((7-chloro-5,8-dioxo-5,8-dihydroquinoléin-6-yl)amino)-2-(4-méthylpipérazin-1-yl)benzonitrile |
| 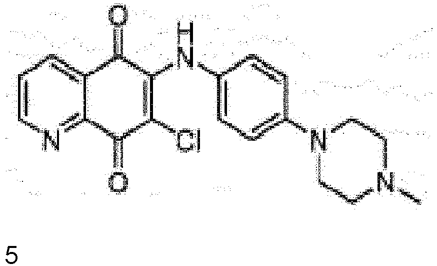 | 7-chloro-6-((4-(4-méthylpipérazin-1-yl)phényl)amino)quinoléine-5,8-dione |
| 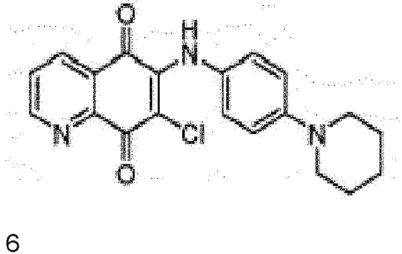 | 7-chloro-6-((4-(pipéridin-1-yl) phényl)amino)quinoléine-5,8-dione |
| 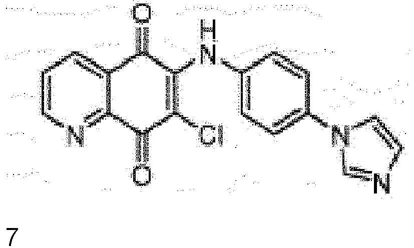 | 6-((4-(1H-imidazol-1-yl)phényl)amino)-7-chloroquinoléine-5,8-dione |
| 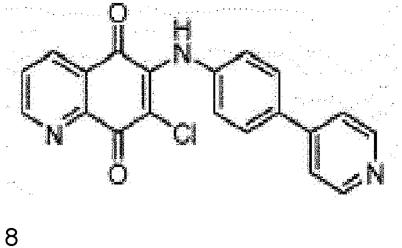 | 7-chloro-6-((4-(4-(pyridin-4-yl)phényl)amino)quinoléine-5,8-dione |
| 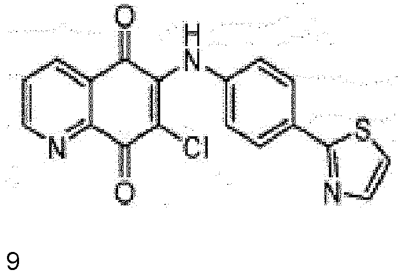 | 7-chloro-6-((4-(thiazol-2-yl)phényl)amino)quinoléine-5,8-dione |
| 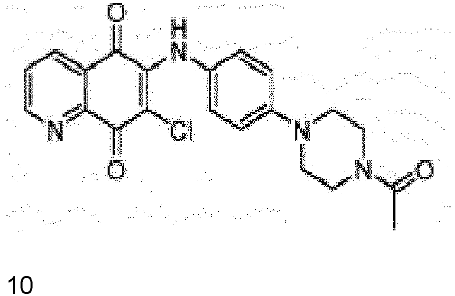 | 6-((4-(4-acétylpipérazin-1-yl)phényl)amino)-7-chloroquinoléine-5,8-dione |
| 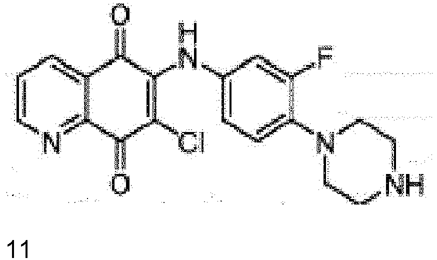 | 7-chloro-6-((3-fluoro-4-(pipérazin-1-yl)phényl)amino)quinoléine-5,8-dione |
| 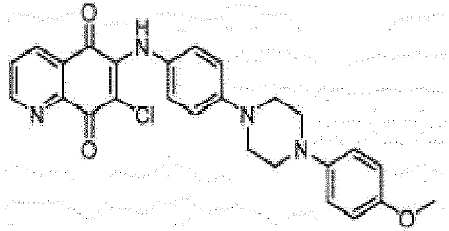 | 7-chloro-6-((4-(4-(4-méthoxyphényl)pipérazin-1-yl)phényl)amino)quinoléine-5,8-dione |
| 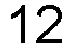 | |
| 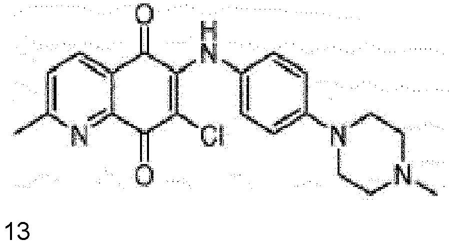 | 7-chloro-2-méthyl-6-((4-(4-méthylpipérazin-1-yl)phényl)amino)quinoléine-5,8-dione |
| 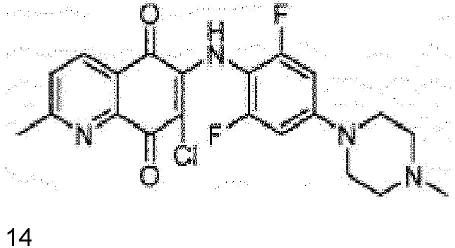 | 7-chloro-6-((2,6-difluoro-4-(4-méthylpipérazin-1-yl)phényl)amino)-2-méthylquinoléine-5,8-dione |
| 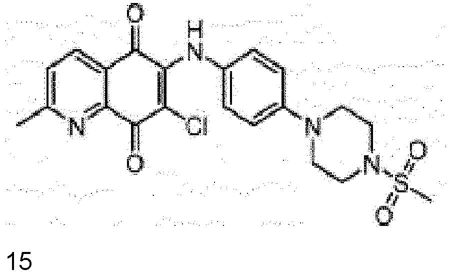 | 7-chloro-2-méthyl-6-((4-(4-(méthylsulfonyl)pipérazin-1-yl)phényl)amino)quinoléine-5,8-dione |
| 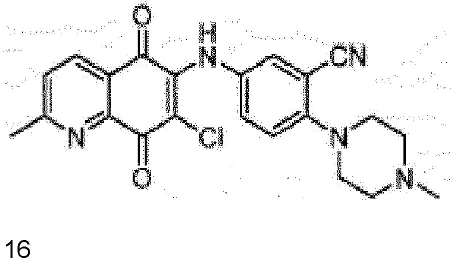 | 5-((7-chloro-2-méthyl-5,8-dioxo-5,8-dihydroquinoléin-6-yl)amino)-2-(4-méthylpipérazin-1-yl)benzonitrile |
| 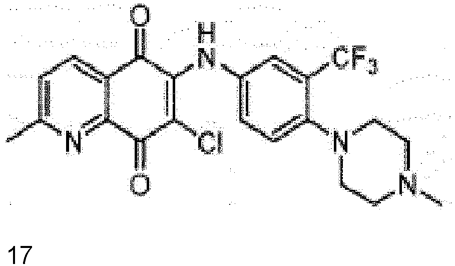 | 7-chloro-2-méthyl-6-((4-(4-méthylpipérazin-1-yl)-3-(trifluorométhyl)phényl)amino)quinoléine-5,8-dione |
| 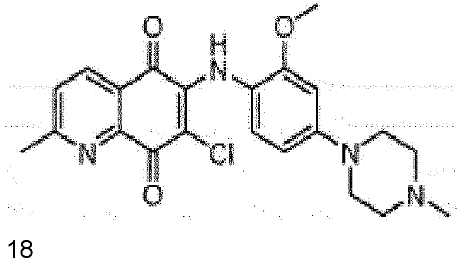 | 7-chloro-6-((2-méthoxy-4-(4-méthylpipérazin-1-yl)phényl)amino)-2-méthylquinoléine-5,8-dione |
| 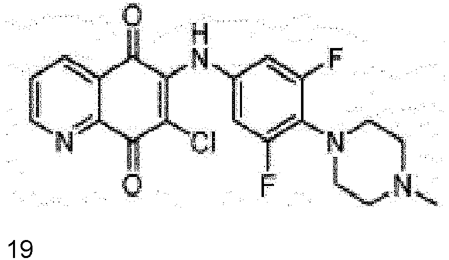 | 7-chloro-6-((3,5-difluoro-4-(4-méthylpipérazin-1-yl)phényl)amino)quinoléine-5,8-dione |
| 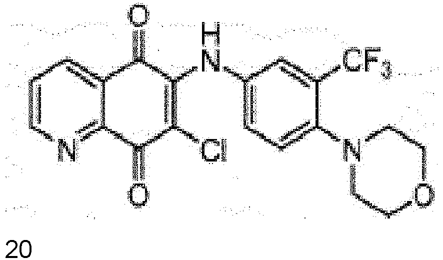 | 7-chloro-6-((4-morpholino-3-(trifluorométhyl)phényl)amino)quinoléine-5,8-dione |
| 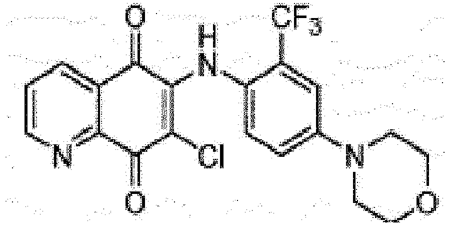 | 7-chloro-6-((4-morpholino-2-(trifluorométhyl)phényl)amino)quinoléine-5,8-dione |
| 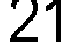 | |
| 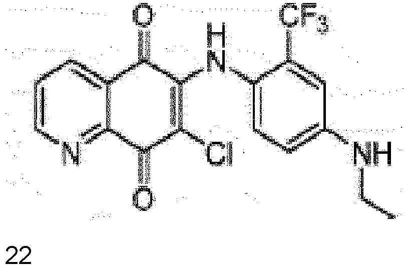 | 7-chloro-6-((4-éthylamino)-2-(trifluorométhyl)phényl)amino)quinoléine-5,8-dione |
| 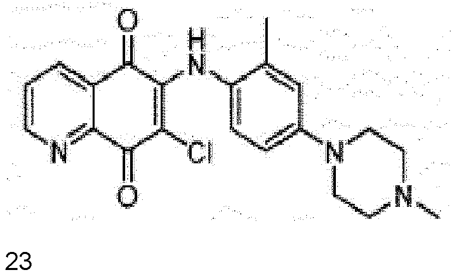 | 7-chloro-6-((2-méthyl-4-(4-méthylpipérazin-1-yl)phényl)amino)quinoléine-5,8-dione |
| 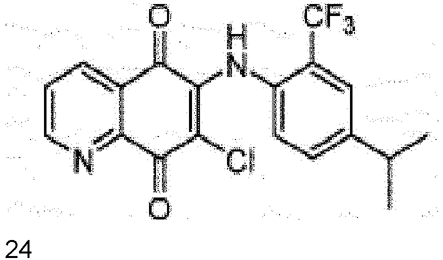 | 7-chloro-6-((4-isopropyl-2-(trifluorométhyl)phényl)amino)quinoléine-5,8-dione |
| 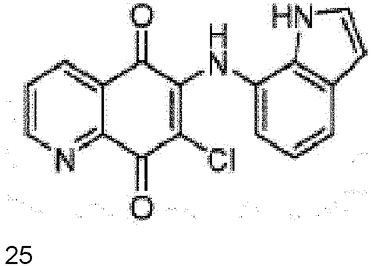 | 6-((1H-indol-7-yl)amino)-7-chloroquinoléine-5,8-dione |
| 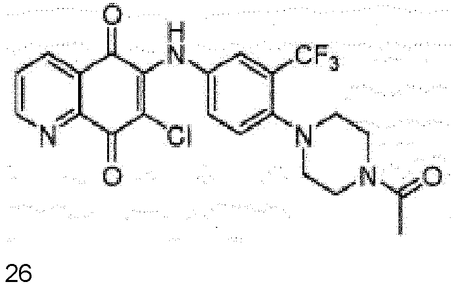 | 6-((4-(4-acétylpipérazin-1-yl)-3-(trifluorométhyl)phényl)amino)-7-chloroquinoléine-5,8-dione |
| 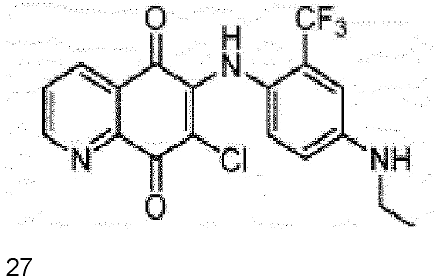 | 7-chloro-6-((4-(éthylamino)-2-(trifluorométhyl)phényl)amino)quinoléine-5,8-dione |
| 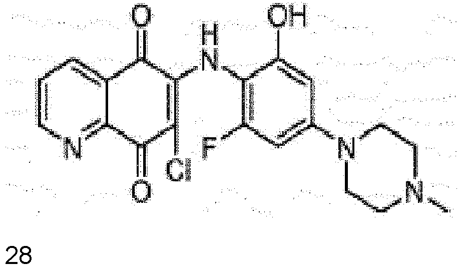 | 7-chloro-6-((2-fluoro-6-hydroxy4-(4-méthylpipérazin-1-yl)phényl)amino)quinoléine-5,8-dione |
| 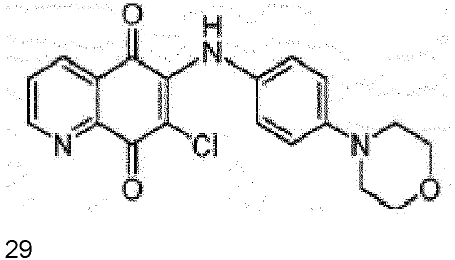 | 7-chloro-6-((4-morpholinophényl)amino)quinoléine-5,8-dione |
| 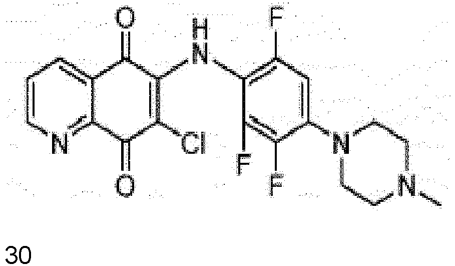 | 7-chloro-6-((2,3,6-trifluoro-4-(4-méthylpipérazin-1-yl)phényl)amino)quinoléine-5,8-dione |
| 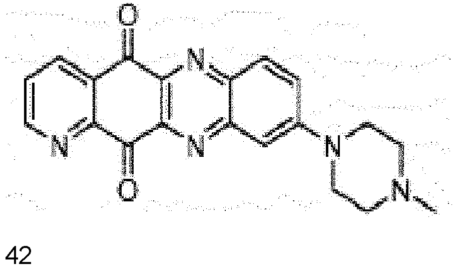 | 9-(4-méthylpipérazin-1-yl)pyrido[2,3-b]phénazine-5,12-dione |
| 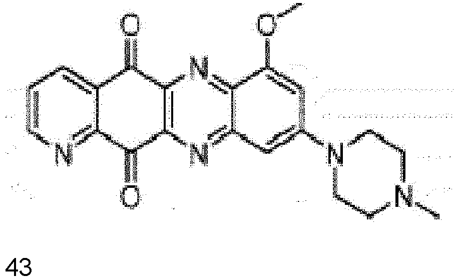 | 7-méthoxy-9-(4-méthylpipérazin-1-yl)pyrido[2,3-b]phénazine-5,12-dione |
| 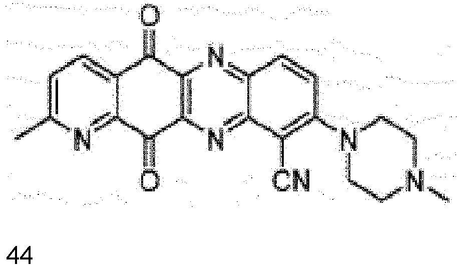 | 2-méthyl-9-(4-méthylpipérazin-1-yl)-5,12-dioxo-5,12-dihydropyrido[2,3-b]phénazine-10-carbonitrile |
| 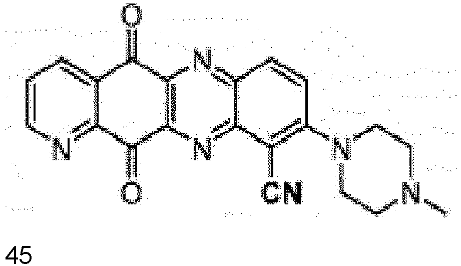 | 9-(4-méthylpipérazin-1-yl)-5,12-dioxo-5,12-dihydropyrido[2,3-b]phénazine-10-carbonitrile |
| 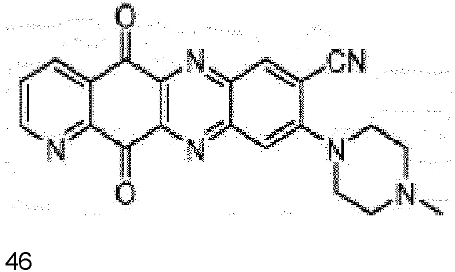 | 9-(4-méthylpipérazin-1-yl)-5,12-dioxo-5,12-dihydropyrido[2,3-b]phénazine-8-carbonitrile |
| 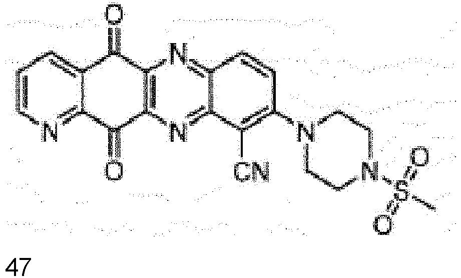 | 9-(4-(méthylsulfonyl)pipérazin-1-yl)-5,12-dioxo-5,12-dihydropyrido[2,3-b]phénazine-10-carbonitrile |
| 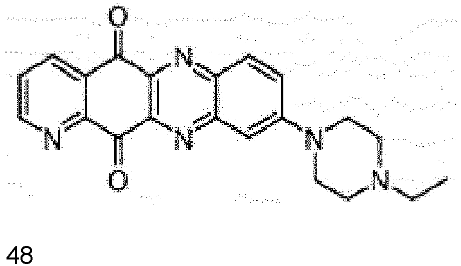 | 9-(4-éthylpipérazin-1-yl)pyrido[2,3-b]phénazine-5,12-dione |
| 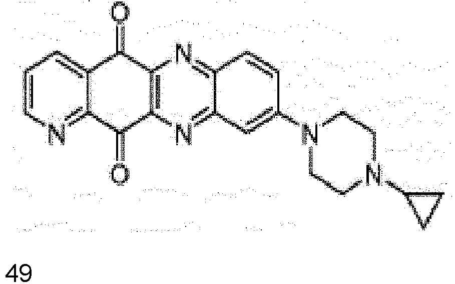 | 9-(4-cyclopropylpipérazin-1-yl)pyrido[2,3-b]phénazine-5,12-dione |
| 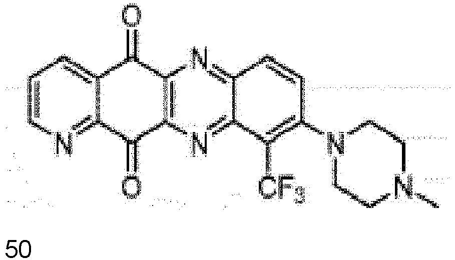 | 9-(4-méthylpipérazin-1-yl)-10-(trifluorométhyl)pyrido[2,3-b]phénazine-5,12-dione |
| 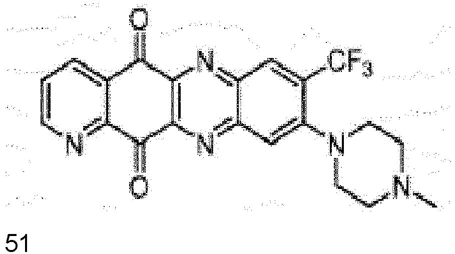 | 9-(4-méthylpipérazin-1-yl)-8-(trifluorométhyl)pyrido[2,3-b]phénazine-5,12-dione |
| 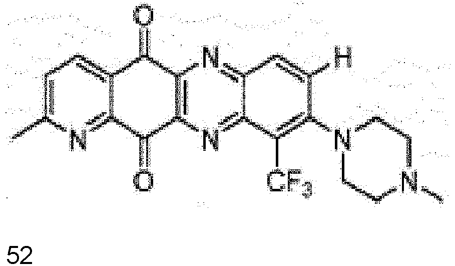 | 2-méthyl-9-(4-méthylpipérazin-1-yl)-10-(trifluorométhyl)pyrido[2,3-b]phénazine-5,12-dione |
| 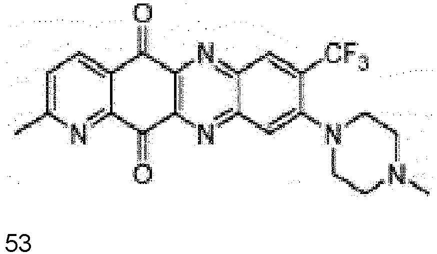 | 2-méthyl-9-(4-méthylpipérazin-1-yl)-8-(trifluorométhyl)pyrido[2,3-b]phénazine-5,12-dione |
| 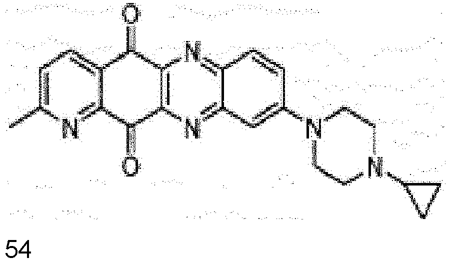 | 9-(4-cyclopropylpipérazin-1-yl)-2-méthylpyrido[2,3-b]phénazine-5,12-dione |
| 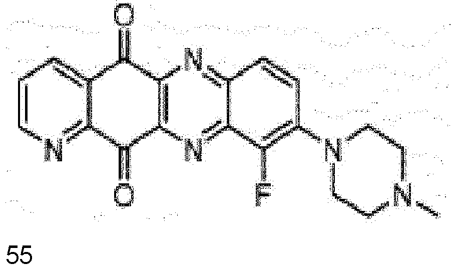 | 10- fluoro-9-(4-méthylpipérazin-1 - yl)pyrido[2,3-b]phénazine-5,12-dione |
| 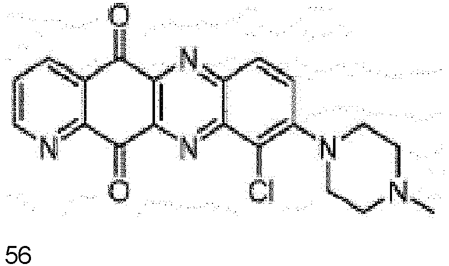 | 10-chloro-9-(4-méthylpipérazin-1-yl)pyrido[2,3-b]phénazine-5,12-dione |
| 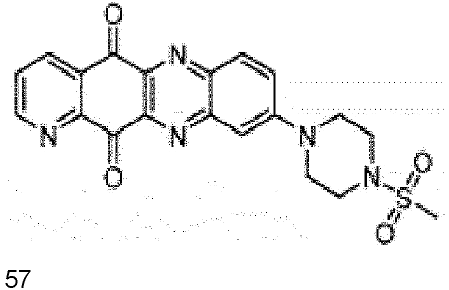 | 9-(4-(méthylsulfonyl)pipérazin-1-yl)pyrido[2,3-b]phénazine-5,12-dione |
| 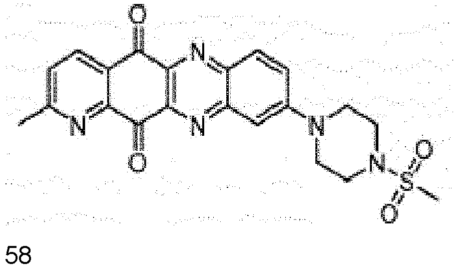 | 2-méthyl-9-(4-(méthylsulfonyl)pipérazin-1-yl)pyrido[2,3-b]phénazine-5,12-dione |
| 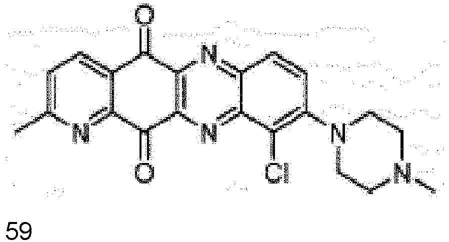 | 10-chloro-2-méthyl-9-(4-méthylpipérazin-1-yl)pyrido[2,3-b]phénazine-5,12-dione |
| 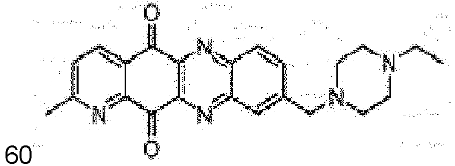 | 9-((4-éthylpipérazin-1-yl)méthyl)-2-méthylpyrido[2,3-b]phénazine-5,12-dione |
| 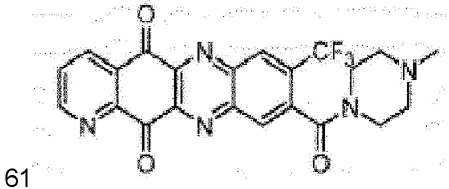 | 9-(4-méthylpipérazine-1-carbonyl)-8-(trifluorométhyl)pyrido[2,3-b]phénazine-5,12-dione |
| 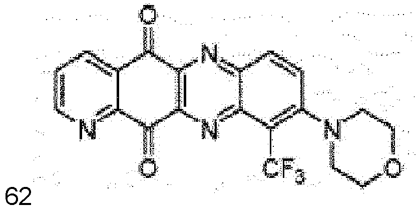 | 9-morpholino-10-(trifluorométhyl)pyrido[2,3-b]phénazine-5,12-dione |
| 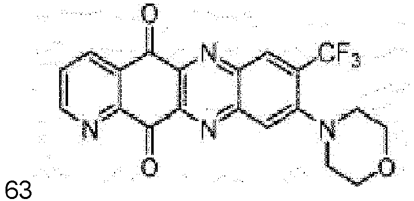 | 9-morpholino-8-(trifluorométhyl)pyrido[2,3-b]phénazine-5,12-dione |
| 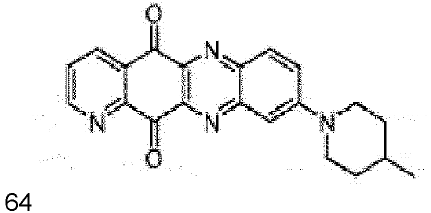 | 9-(4-méthylpipéridin-1-yl)pyrido[2,3-b]phénazine-5,12-dione |
| 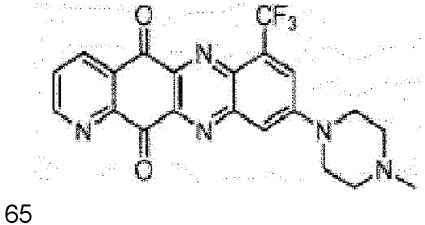 | 9-(4-méthylpipérazin-1-yl)-7-(trifluorométhyl)pyrido[2,3-b]phénazine-5,12-dione |
| 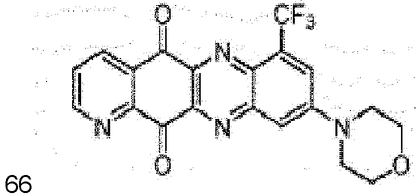 | 9-morpholino-7-(trifluorométhyl)pyrido[2,3-b]phénazine-5,12-dione |
| 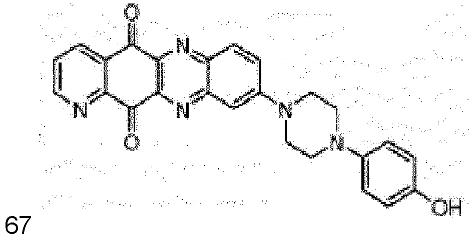 | 9-(4-(4-hydroxyphényl)pipérazin-1-yl)pyrido[2,3-b]phénazine-5,12-dione |
| 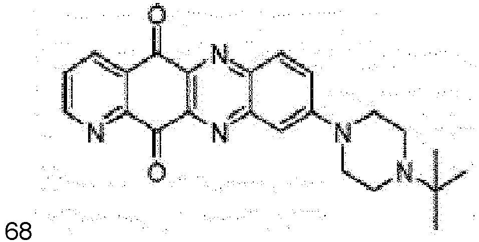 | 9-(4-(tert-butyl)pipérazin-1-yl)pyrido[2,3-b]phénazine-5,12-dione |
| 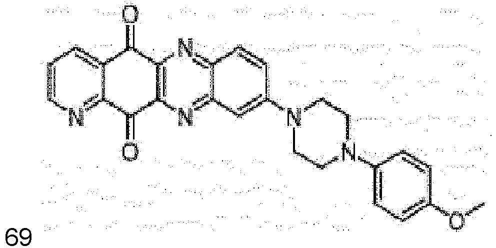 | 9-(4-(4-méthoxyphényl)pipérazin-1-yl)pyrido[2,3-b]phénazine-5,12-dione |
| 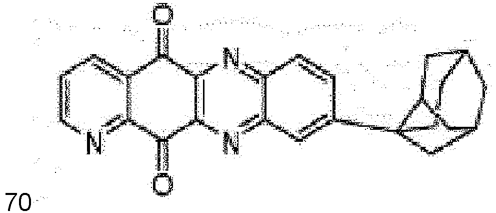 | 9-((3aS,5S,7aS)-octahydro-7aH-2,5-méthanoindén-7a-yl)pyrido[2,3-b]phénazine-5,12-dione |
| 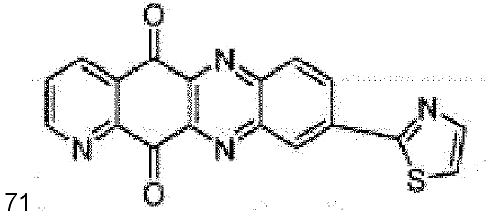 | 9-(thiazol-2-yl)pyrido[2,3-b]phénazine-5,12-dione |
| 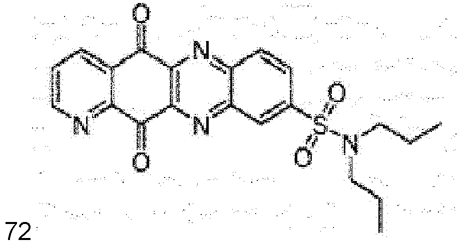 | 5,12-dioxo-N,N-dipropyl-5,12-dihydropyrido[2,3-b]phénazine-9-sulfonamide |
| 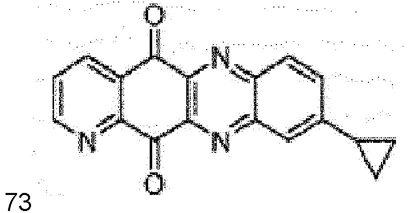 | 9-cyclopropylpyrido[2,3-b]phénazine-5,12-dione |
| 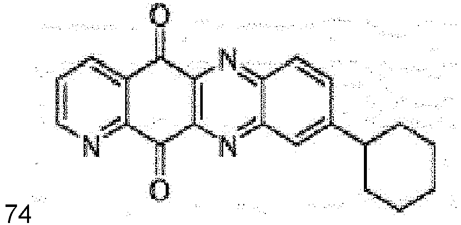 | 9-cyclohexylpyrido[2,3-b]phénazine-5,12-dione |
| 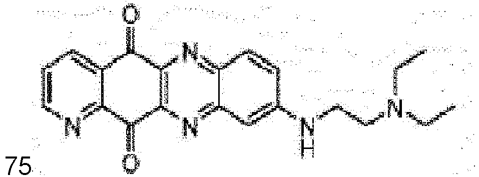 | 9-((2-(diéthylamino)éthyl)amino)pyrido[2,3-b]phénazine-5,12-dione |
| 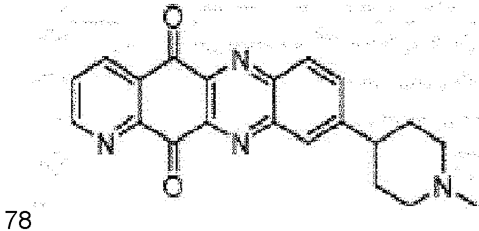 | 9-(1-méthylpipéridin-4-yl)pyrido[2,3-b]phénazine-5,12-dione |
| 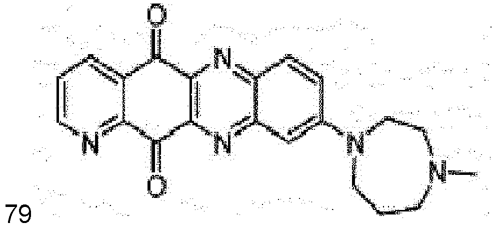 | 9-(4-méthyl-1,4-diazépan-1-yl)pyrido[2,3-b]phénazine-5,12-dione |
| 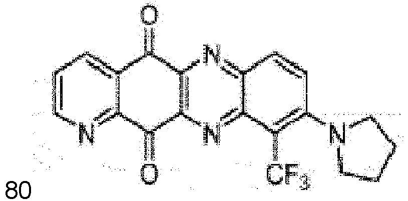 | 9-(pyrrolidin-1-yl)-10-(trifluorométhyl)pyrido[2,3-b]phénazine-5,12-dione |
| 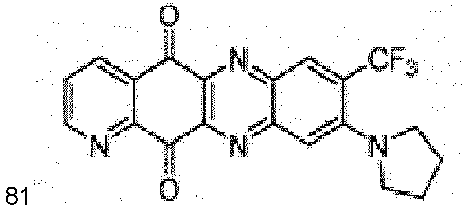 | 9-(pyrrolidin-1-yl)-8-(trifluorométhyl)pyrido[2,3-b]phénazine-5,12-dione |
| 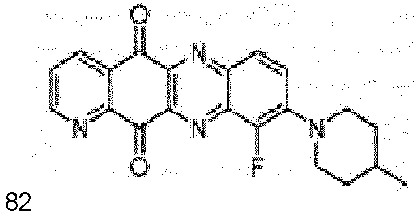 | 10-fluoro-9-(4-méthylpipéridin-1-yl)pyrido[2,3-b]phénazine-5,12-dione |
| 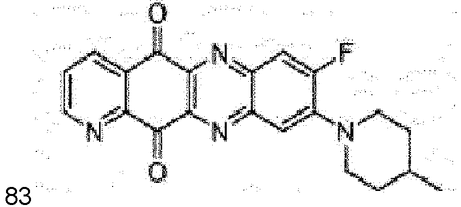 | 8-fluoro-9-(4-méthylpipéridin-1-yl)pyrido[2,3-b]phénazine-5,12-dione |
| 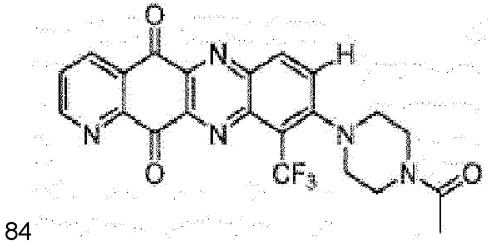 | 9-(4-acétylpipérazin-1-yl)-10-(trifluorométhyl)pyrido[2,3-b]phénazine-5,12-dione |
| 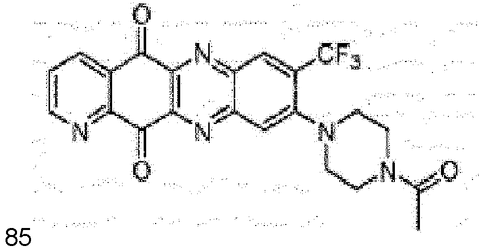 | 9-(4-acétylpipérazin-1-yl)-8-(trifluorométhyl)pyrido[2,3-b]phénazine-5,12-dione |
| 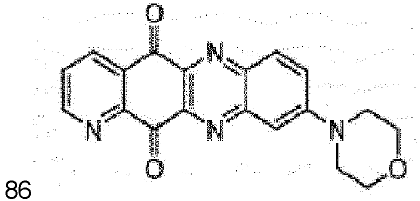 | 9-morpholinopyrido[2,3-b]phénazine-5,12-dione |
| 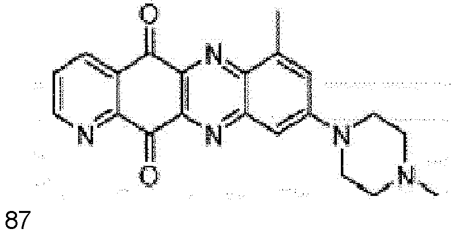 | 7-méthyl-9-(4-méthylpipérazin-1-yl) pyrido[2,3-b]phénazine-5,12-dione |
| 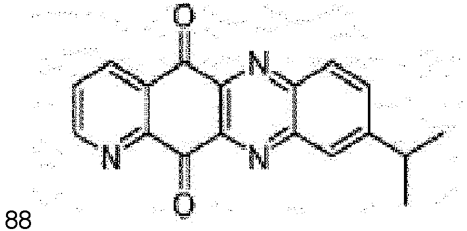 | 9-isopropylpyrido[2,3-b]phénazine-5,12-dione |

4. Composition pharmaceutique comprenant un composé de l'une des revendications 1 à 3 pour une utilisation dans le traitement, l'amélioration ou la prévention d'une maladie hyperproliférative chez un patient, dans laquelle une quantité thérapeutiquement efficace de ladite composition pharmaceutique est administrée audit patient.

5. Composition pharmaceutique pour une utilisation dans le traitement, l'amélioration ou la prévention d'une maladie hyperproliférative de la revendication 4, dans laquelle ladite maladie hyperproliférative est un cancer,
de préférence dans laquelle ledit cancer est choisi parmi le cancer du sein, le cancer de la prostate, le lymphome, le cancer de la peau, le cancer du côlon, le mélanome, le mélanome malin, le cancer de l'ovaire, le cancer du cerveau, le carcinome primaire du cerveau, le cancer de la tête et du cou, le gliome, le glioblastome, le cancer du foie, le cancer de la vessie, le cancer du poumon non à petites cellules, le carcinome de la tête ou du cou, le carcinome du sein, le carcinome de l'ovaire, le carcinome du poumon, le carcinome du poumon à petites cellules, la tumeur de Wilms, le carcinome du col de l'utérus, le carcinome testiculaire, le carcinome de la vessie, le carcinome pancréatique, le carcinome de l'estomac, le carcinome du côlon, le carcinome de la prostate, le carcinome génito-urinaire, le carcinome de la thyroïde, le carcinome de l'œsophage, le myélome, le myélome multiple, le carcinome surrénalien, le carcinome à cellules rénales, le carcinome de l'endomètre, le carcinome du cortex surrénal, l'insulinome pancréatique malin, le carcinome carcinoïde malin, le choriocarcinome, le mycosis fongoïde, l'hypercalcémie maligne, l'hyperplasie cervicale, la leucémie, la leucémie lymphoïde aiguë, la leucémie lymphoïde chronique, la leucémie myéloïde aiguë, la leucémie myéloïde chronique, la leucémie granulocytaire chronique, la leucémie granulocytaire aiguë, la leucémie à tricholeucocytes, le neuroblastome, le rhabdomyosarcome, le sarcome de Kaposi, la maladie de Vaquez, la thrombocytose essentielle, la maladie de Hodgkin, le lymphome non hodgkinien, le sarcome des tissus mous, le sarcome ostéogénique, la macroglobulinémie de Waldenström et le rétinoblastome.

6. Composition pharmaceutique pour une utilisation dans le traitement, l'amélioration ou la prévention d'une maladie hyperproliférative de la revendication 4, dans laquelle ledit patient est un patient humain.

7. Composition pharmaceutique pour une utilisation dans le traitement, l'amélioration ou la prévention d'une maladie hyperproliférative de la revendication 4, dans laquelle en outre un ou plusieurs agents anticancéreux sont administrés.

8. Composition pharmaceutique pour une utilisation dans le traitement, l'amélioration ou la prévention d'une maladie hyperproliférative de la revendication 7, dans laquelle ledit agent anticancéreux est un agent chimiothérapeutique.

9. Composition pharmaceutique pour une utilisation dans le traitement, l'amélioration ou la prévention d'une maladie hyperproliférative de la revendication 7, dans laquelle ledit agent anticancéreux est une radiothérapie.

10. Kit comprenant l'un des composés des revendications 1 à 3 et des instructions pour administrer ledit composé à un patient souffrant d'une maladie hyperproliférative.

11. Kit de la revendication 10, dans lequel ladite maladie hyperproliférative est un cancer.

12. Kit de la revendication 11, dans lequel ledit cancer est choisi parmi le cancer du sein, le cancer de la prostate, le lymphome, le cancer de la peau, le cancer du côlon, le mélanome, le mélanome malin, le cancer de l'ovaire, le cancer du cerveau, le carcinome primaire du cerveau, le cancer de la tête et du cou, le gliome, le glioblastome, le cancer du foie, le cancer de la vessie, le cancer du poumon non à petites cellules, le carcinome de la tête ou du cou, le carcinome du sein, le carcinome de l'ovaire, le carcinome du poumon, le carcinome du poumon à petites cellules, la tumeur de Wilms, le carcinome du col de l'utérus, le carcinome testiculaire, le carcinome de la vessie, le carcinome pancréatique, le carcinome de l'estomac, le carcinome du côlon, le carcinome de la prostate, le carcinome génito-urinaire, le carcinome de la thyroïde, le carcinome de l'œsophage, le myélome, le myélome multiple, le carcinome surrénalien, le carcinome à cellules rénales, le carcinome de l'endomètre, le carcinome du cortex surrénal, l'insulinome pancréatique malin, le carcinome carcinoïde malin, le choriocarcinome, le mycosis fongoïde, l'hypercalcémie maligne, l'hyperplasie cervicale, la leucémie, la leucémie lymphoïde aiguë, la leucémie lymphoïde chronique, la leucémie myéloïde aiguë, la leucémie myéloïde chronique, la leucémie granulocytaire chronique, la leucémie granulocytaire aiguë, la leucémie à tricholeucocytes, le neuroblastome, le rhabdomyosarcome, le sarcome de Kaposi, la maladie de Vaquez, la thrombocytose essentielle, la maladie de Hodgkin, le lymphome non hodgkinien, le sarcome des tissus mous, le sarcome ostéogénique, la macroglobulinémie de Waldenström et le rétinoblastome.

13. Kit de la revendication 10 comprenant en outre un ou plusieurs agents anticancéreux.

14. Kit de la revendication 13, dans lequel ledit composé doit être administré conjointement avec un ou plusieurs agents anticancéreux.
